# EUROPEAN PATENT SPECIFICATION

(11) **EP 4 326 326 B1**
(45) Date of publication and mention of the grant of the patent: **18.11.2026**
(21) Application number: 22724027.2
(22) Date of filing: 20.04.2022
(51) Int. Cl.: A61K 41/00, A61P 35/00

(54) **ENHANCEMENT OF TREATMENT WITH IMMUNOTHERAPEUTIC AGENTS**
VERBESSERUNG DER BEHANDLUNG MIT IMMUNOTHERAPEUTISCHEN MITTELN
AMÉLIORATION DU TRAITEMENT AVEC DES AGENTS IMMUNOTHÉRAPEUTIQUES

(30) Priority: 21.04.2021 GB 202105691
(43) Date of publication of application: 28.02.2024
(60) Divisional application: 26209127.5 / 4 840 516
(73) Proprietor: Act Therapeutics Ltd, Newmarket, Suffolk CB8 8LB (GB)
(72) Inventor: SONTUM, Per Christian, 0259 Oslo (NO); KVÅLE, Svein, 1435 Ås (NO); KOTOPOULIS, Spiros, 2040 Kløfta (NO); HEALEY, Andrew John, 1528 Moss (NO)
(74) Representative: Bryn Aarflot AS
(86) International application number: PCT/EP2022/060431
(87) International publication number: WO 2022/223626

(56) References cited:
- EP-A1- 4 008 358
- WO-A1-2015/047103
- WO-A1-2021/045485
- WO-A1-2021/080438
- WO-A2-2020/115491
- PIN-YUAN CHEN ET AL: "Focused ultrasound-induced blood?brain barrier opening to enhance interleukin-12 delivery for brain tumor immunotherapy: a preclinical feasibility study", JOURNAL OF TRANSLATIONAL MEDICINE, BIOMED CENTRAL, vol. 13, no. 1, 17 March 2015 (2015-03-17), pages 93, XP021218132, ISSN: 1479-5876, DOI: 10.1186/S12967-015-0451-Y
- SHIYA ZHENG ET AL: "Targeted microbubbles with ultrasound irradiation and PD-1 inhibitor to increase antitumor activity in B-cell lymphoma", NANOMEDICINE, vol. 13, no. 3, 1 February 2018 (2018-02-01), GB, pages 297 - 311, XP055667366, ISSN: 1743-5889, DOI: 10.2217/nnm-2017-0296
- BULNER SHARSHI ET AL: "Enhancing Checkpoint Inhibitor Therapy with Ultrasound Stimulated Microbubbles", ULTRASOUND IN MEDICINE AND BIOLOGY, vol. 45, no. 2, 15 November 2018 (2018-11-15), pages 500 - 512, XP085573889, ISSN: 0301-5629, DOI: 10.1016/J.ULTRASMEDBIO.2018.10.002
- ÅSLUND ANDREAS K.O. ET AL: "Efficient Enhancement of Blood-Brain Barrier Permeability Using Acoustic Cluster Therapy (ACT)", THERANOSTICS, vol. 7, no. 1, 1 January 2017 (2017-01-01), AU, pages 23 - 30, XP055926530, ISSN: 1838-7640, Retrieved from the Internet <URL:https://www.ncbi.nlm.nih.gov/pmc/articles/PMC5196882/pdf/thnov07p0023.pdf> DOI: 10.7150/thno.16577
- SONTUM PER ET AL: "Acoustic Cluster Therapy (ACT) - A novel concept for ultrasound mediated, targeted drug delivery", INTERNATIONAL JOURNAL OF PHARMACEUTICS, ELSEVIER, NL, vol. 495, no. 2, 25 September 2015 (2015-09-25), pages 1019 - 1027, XP029292204, ISSN: 0378-5173, DOI: 10.1016/J.IJPHARM.2015.09.047
- BUSH NIGEL ET AL: "Theranostic Attributes of Acoustic Cluster Therapy and Its Use for Enhancing the Effectiveness of Liposomal Doxorubicin Treatment of Human Triple Negative Breast Cancer in Mice", FRONTIERS IN PHARMACOLOGY, vol. 11, 20 February 2020 (2020-02-20), XP055926534, DOI: 10.3389/fphar.2020.00075
- BUSH NIGEL ET AL: "Therapeutic Dose Response of Acoustic Cluster Therapy in Combination With Irinotecan for the Treatment of Human Colon Cancer in Mice", FRONTIERS IN PHARMACOLOGY, vol. 10, 19 November 2019 (2019-11-19), XP055944809, DOI: 10.3389/fphar.2019.01299
- BUSH NIGEL ET AL: "Acoustic Cluster Therapy displays theranostic capability in enhancing the effectiveness of liposomal doxorubicin treatment of human triple negative breast cancer in mice", 2019 IEEE INTERNATIONAL ULTRASONICS SYMPOSIUM (IUS), IEEE, 6 October 2019 (2019-10-06), pages 224 - 226, XP033671023, DOI: 10.1109/ULTSYM.2019.8925626
- BUSH NIGEL ET AL: "Ultrasound, optical and photoacoustic imaging of Acoustic Cluster Therapy enhanced delivery to human tumors in mice", 2019 IEEE INTERNATIONAL ULTRASONICS SYMPOSIUM (IUS), IEEE, 6 October 2019 (2019-10-06), pages 1556 - 1559, XP033670937, DOI: 10.1109/ULTSYM.2019.8925577
- NG SERINA ET AL: "Abstract A099: Acoustic Cluster Therapy enhances the efficacy of chemotherapeutic regimens in patient-derived xenograft mouse models for pancreatic ductal adenocarcinoma", 1 December 2019 (2019-12-01), XP055944853, Retrieved from the Internet <URL:https://aacrjournals.org/mct/article/18/12_Supplement/A099/240208/Abstract-A099-Acoustic-Cluster-Therapy-enhances> [retrieved on 20220721]
- VAN WAMEL ANNEMIEKE ET AL: "Acoustic Cluster Therapy (ACT) enhances the therapeutic efficacy of paclitaxel and Abraxane for treatment of human prostate adenocarcinoma in mice", JOURNAL OF CONTROLLED RELEASE, ELSEVIER, AMSTERDAM, NL, vol. 236, 11 June 2016 (2016-06-11), pages 15 - 21, XP029661427, ISSN: 0168-3659, DOI: 10.1016/J.JCONREL.2016.06.018
- KOTOPOULIS SPIROS ET AL: "Sonoporation with Acoustic Cluster Therapy (ACT ) induces transient tumour volume reduction in a subcutaneous xenograft model of pancreatic ductal adenocarcinoma", JOURNAL OF CONTROLLED RELEASE, ELSEVIER, AMSTERDAM, NL, vol. 245, 18 November 2016 (2016-11-18), pages 70 - 80, XP029877442, ISSN: 0168-3659, DOI: 10.1016/J.JCONREL.2016.11.019
- WAMEL ANNEMIEKE VAN ET AL: "Acoustic Cluster Therapy (ACT) - pre-clinical proof of principle for local drug delivery and enhanced uptake", JOURNAL OF CONTROLLED RELEASE, ELSEVIER, AMSTERDAM, NL, vol. 224, 13 January 2016 (2016-01-13), pages 158 - 164, XP029411490, ISSN: 0168-3659, DOI: 10.1016/J.JCONREL.2016.01.023
- SONTUM P ET AL: "Acoustic Cluster Therapy - A Novel Approach for Ultrasound Mediated Targeted Drug Delivery: Technology Basics and Proof of Concept", 1 January 2016 (2016-01-01), XP055944873, Retrieved from the Internet <URL:https://www.researchgate.net/publication/290955455_Acoustic_Cluster_Therapy_-_A_Novel_Approach_for_Ultrasound_Mediated_Targeted_Drug_Delivery_Technology_Basics_and_Proof_of_Concept> [retrieved on 20220721]
- VAN WAMEL ANNEMIEKE ET AL: "Acoustic Cluster Therapy (ACT): a novel concept for targeted drug delivery - In vivo Characteristics and Proof of Principle", 1 January 2015 (2015-01-01), XP055944876, Retrieved from the Internet <URL:https://www.researchgate.net/publication/277583076_Acoustic_Cluster_Therapy_ACT_a_novel_concept_for_targeted_drug_delivery_-_In_vivo_Characteristics_and_Proof_of_Principle> [retrieved on 20220721]

## Description

### Field of the invention

The present invention relates to ultrasound mediated, targeted delivery of immunotherapeutic agents to pathological sites, and particularly for medicinal treatment using such therapeutic agents. Thus, the invention provides a cluster composition and a pharmaceutical composition, for use in delivery and preparation for administration of immunotherapeutic agents for treatment of pathological conditions such as cancer and autoimmune/inflammatory diseases.

### Background of the invention

Immunotherapy is the treatment of a disease by activating or suppressing the immune system. Use of immunotherapeutic agents (ITAs) is rapidly evolving and new classes, agents, and new uses of current agents are continuously being developed. Different classes of immunotherapeutic agents include: monoclonal antibodies (mAbs), fusion proteins, soluble cytokine receptors, recombinant cytokines, small-molecule mimetics, cell therapies, cancer vaccines and oncolytic viruses. Of these, mAbs is by far the most important class with a wide range of approved agents for treatment of a variety of pathological conditions including cancers and autoimmune diseases.

Monoclonal antibody therapy is a form of immunotherapy that uses mAbs to bind mono-specifically to certain cells or proteins. The objective is that this treatment will stimulate the patient's immune system to attack those cells, or alternatively, that mAbs may be used to bind to molecules involved in T-cell regulation to remove inhibitory pathways that block T-cell responses. This is known as immune checkpoint therapy. Currently more than 80 different mAbs are approved by the FDA for treatment of a range of diseases, particularly within oncology and autoimmune diseases. Therapeutic use of mAbs is a fast-growing segment: in 2019, 7 out of 10 bestselling drugs in the US were mAbs, led by Humira (Abbvie, autoimmune diseases), Opdivo (BMS, cancers) and Keytruda (Merck, cancers).

Immuno-oncology (IO) is the artificial stimulation of the immune system to treat cancer, improving on the immune system's natural ability to fight the disease [Carter and Thurson, Immune-oncology agents for cancer therapy, The Pharmaceutical Journal, May 7 2020]. Normal antibodies of the immune system bind to external pathogens, whilst modified immunotherapy antibodies bind to tumour antigens, marking and identifying the cancer cells for the immune system to inhibit or kill. IO-agents explore a variety of mechanisms of action (MoAs), different to regular chemotherapeutics, e.g. checkpoint inhibitors potentiate the body's own cytotoxic T-cells to recognize and attack tumour cells. Monoclonal antibodies represent the most important class of IO-agents in current clinical practise. However, the IO segment also includes oncolytic viruses (e.g. talimogene laherparepvec), cytokines (e.g. interferons and interleukins) and a range of cancer vaccines.

Immunotherapies are also extensively used to dampen autoimmunity and also to treat allergies or to reduce the rejection of transplanted organs. For example, inhibitors of tumour necrosis factor alpha, TNF-α, are routinely used to reduce inflammation in diseases such as rheumatoid arthritis, inflammatory bowel disease and psoriasis.

A prerequisite for a successful medicinal therapy is that the drug reaches interstitial tissue, outside the vascular compartment. Either for direct action on the pathology (e.g. as with chemotherapeutics) or for interactions with immune cells. However, for most therapeutic agents, only a very small fraction of the administered drug reaches the targeted pathological disease area (e.g. tumour) in the body - most all is taken up by healthy tissue, degraded or excreted before reaching its target. For example, for many chemotherapeutic regimens, less than 0.01% of the administered dose accumulate in the targeted cancerous tissue [Kurdziel et al.: "Human Dosimetry and Preliminary Tumour Distribution of 18F-Fluoropaclitaxel in Healthy Volunteers and Newly Diagnosed Breast Cancer Patients Using PET/CT", J Nucl Med. 2011 Sep; 52(9): 1339-1345]. After systemic administration, extravasation of drug from the vascular compartment to interstitial space can occur via three basic processes: passive diffusion, convective transport, and transcytosis through vascular epithelial cells. For most small molecule drugs, passive diffusion is by far the most important route for extravasation and distribution. However, due to the physiochemical properties and large size of most ITAs, passive diffusion does not play a significant role in the extravasation process. Common to most ITAs is that they rest on large molecular structures or nano-sized constructs, e.g. mAbs have a molecular weight of approximately 150.000 Daltons and a diameter of 10-15 nm whereas oncolytic viruses are large constructs which typically measure 50-150 nm in diameter. This compared to a molecular weight of most chemotherapy agents of less than 1000 Daltons, displaying sub-nm dimensions. These sizes effectively hinder the extravasation of the drugs from the vascular compartment and hence reduces the potential efficacy of the treatment significantly [Ryman and Meibohm, CPT Pharmacometrics Syst Pharmacol. 2017 Sep; 6(9): 576-588]. The vascular wall hence represents a significant barrier for the effective use of ITAs and a way to increase drug extravasation could potentially lead to a market increase the therapeutic effect of these agents.

Furthermore, within immune-oncology, it's not the ITA itself which delivers the therapeutic effect, but rather inflammatory cytokines (e.g. IL-1, IL-12, IL-18, TNFα and INFγ) and activated immune cells (e.g. activated T-cells, e.g. CD3, CD4 and CD8 positive cells) where the ITA blocks receptors and enable the immune cells to initiate the required cytotoxic processes towards the pathological cells in question. So, for the therapy to work, the activated immune cells must be able to infiltrate the pathology after being activated, making the tumour immunologically "hot" instead of immunologically "cold". Here the vascular barrier again represents a significant obstruction - in many cases the immune cell infiltration is limited and a way to enhance such could potentially lead to a significant increase in the therapeutic effect of these agents.

Despite improvements in toxicity profiles compared to for example chemotherapeutics, immunotherapies are still hampered by unwanted systemic effects and dose limiting toxicities. Owing to their mechanism of action, ITAs are associated with a unique but variable spectrum of toxicities. The most serious concern is potential supra-physiologic stimulation of the immune system leading to a potentially life-threatening uncontrolled and rapid production of pro-inflammatory cytokines but, ITAs may also display a range of dermatological, endocrine, hepatic ang gastrointestinal toxicities. Increasing the dose to overcome the limited extravasation is hence not an option. However, if the extravasation efficacy could be improved in some manner, lower doses of drugs could be explored whilst still maintaining the therapeutic efficacy and, at the same time, reducing cost and systemic toxicity.

Many immunotherapy regimes are only efficacious in a small proportion of the patient population treated; some respond well to therapy, and some may not respond at all. There are several possible reasons for these differences in clinical responses, including the presence of different gene mutations and varying degrees of activity of specific signalling pathways in individual patients. However, it is also possible that drug extravasation and/or activated cell infiltration vary from patient to patient and that the variability in response is partly due to insufficient concentration of drug in the interstitial tissue of a given subject, or insufficient infiltration of activated cells to the pathology.

There are significant cost implications associated with immunotherapy-based therapies. For example, the one-year global cost of treating non-small cell lung carcinoma with selected mAbs has been estimated at over US$80 billion. The estimated cost per patient per year for a variety of ITAs is over £100,000, which places significant pressure on healthcare systems. Costs for implementing these newer targeted therapies have escalated dramatically, and the duration of treatment has also lengthened because many diseases are increasingly being treated as chronic conditions. In the UK, the National Institute for Health and Care Excellence (NICE) is the organisation responsible for determining whether new treatments are cost effective for the NHS. The cost of a new therapy is evaluated for its clinical effectiveness using a standardised measurement known as a quality-adjusted life year (QALY). In order to be deemed cost-effective for the NHS, a therapy should cost no more than £20,000-30,000 per QALY gained, or £50,000 for end-of-life therapies. New ITAs are increasingly exceeding these thresholds, resulting in rejection by NICE and reduced access for patients. Identifying a way to increase extravasation from the vascular compartment and enhance the distribution of drug in the pathological tissue could open for a reduction in dose, whilst maintaining therapeutic efficacy, with an ensuing, significant lowering of the cost of treatment.

Therapeutic options specifically focused on targeted drug delivery in treating localized pathologies such as solid tumours are currently under investigation, including nanoparticles, molecular targeting, and ultrasound- and microbubble mediated therapy, i.e. sonoporation. Over the past two decades, there has been growing interest in drug delivery using ultrasound. For a recent review see Castle et al [Castle et al, Am J Physiol Heart Circ Physiol February 1, 2013 304:H350-H357]. Many approaches are based on the use of microbubbles similar to those used as ultrasound contrast agents for medical imaging applications, for release of incorporated or attached drugs and/or for enhanced uptake of systemically (co-) administered drugs. Sonoporation is a methodology where gas microbubbles are injected into the vasculature and stimulated by ultrasound (US) to invoke biomechanical effects that increase the permeability of the vascular barrier and extravasation of drug at a specific location (e.g. within a solid tumour). Microbubbles are stabilized gas bubbles (2-3 µm in diameter) that are injected intravascularly and are typically stable for up to 1-2 minutes in vivo with no known side-effects. Upon the application of ultrasound, these microbubbles oscillate and interact with nearby endothelial/vascular wall cells forming fenestrations via a variety of biomechanical effects. This interaction may permit increased extravasation of drug from the vascular compartment, intracellular drug uptake and also allowing therapeutic agents to penetrate deeper into the tissue than into the vascular wall alone. Whilst this technology show promise, the true potential of sonoporation is limited due to the use of commercially available microbubbles designed and optimised for ultrasound imaging (contrast microbubbles), not therapeutic enhancement. As a result, substantial research is focusing on developing "next-generation" microbubbles optimised for ultrasound mediated, targeted and enhanced therapy. A primary limitation is the size of microbubbles. Being small, the level of biomechanical effects they can exert inside the vascular compartment is limited. Furthermore, physical contact with the endothelial wall is limited and, as the biomechanical effects induced typically decline exponentially with distance from the vessel wall, effectiveness in inducing fenestrations is restricted. As a consequence of these limitations, sonoporation with regular contrast microbubbles requires the use of relative high energy US fields (high mechanical index, MI). Although microbubble mediated delivery mechanisms have been clearly demonstrated in vivo, there are related bio-effects that raise safety issues for the approach. To all likelihood, microbubble cavitation mechanisms are involved and in particular micro-haemorrhage and irreversible vascular damage have been observed. These processes may also lead to vascular shut down, i.e. blood vessels collapsing (transiently or permanently), effectively stopping blood perfusion and, hence, uptake of drugs.

WO2015/047103 is directed to a concept for ultrasound mediated, targeted delivery is proposed, wherein a microbubble-microdroplet cluster composition is administered alongside a therapeutic agent and where ultrasound insonation of a targeted pathology may lead to an increase in the therapeutic effect versus the therapeutic agent alone. This concept, termed as Acoustic Cluster Therapy (ACT Sonoporation or ACT), has later been investigated in a series of pre-clinical proof of principal and proof of concept studies, treating a variety of cancerous conditions with various chemotherapeutics. For example, van Wamel et al., Acoustic Cluster Therapy enhances the therapeutic efficacy of paclitaxel and Abraxane® for treatment of human prostate adenocarcinoma in mice, J Control Release, 236 (2016) 15-21], demonstrates the potential of ACT in combination with standard of care, small molecule chemotherapy regimens. agents.

Based on the above, there is a clear need for new and alternative compositions and methods for treatment with immunotherapy agents, enabling an increase in uptake to interstitial tissues and/or an increase of activated immune cell infiltration to the targeted pathology. Such would have the potential to improve on therapeutic outcome and could also be explored to reduce the systemic dose, limiting toxicities, and reducing cost of treatment significantly.

WO2021/080438 is directed to ultrasound mediated delivery of therapeutic agents to the pancreas, and particularly for treatment of pancreatic cancer such as pancreatic ductal adenocarcinoma (PDAC). More particularly, WO2021/080438 is directed to a cluster composition and a pharmaceutical composition, for use in delivery of therapeutic agents and for treatment of pancreatic cancers, including PDAC.

### Brief summary of the invention

It is an objective of the present invention to provide compositions and methods for use in medicinal treatment with immunotherapy agents (ITAs) and in a method of immunotherapy, and particularly for treatment of cancers and autoimmune diseases. The invention relates to ultrasound mediated, targeted delivery of ITAs to pathological sites, and to medicinal treatment using such therapeutic agents.

The inventors have discovered that Acoustic Cluster Therapy (ACT^{®}) can be used in delivery of ITAs and in enhancement of activated immune cell infiltration. ACT, presented in WO2015/047103, is a concept for ultrasound mediated, targeted delivery, wherein a microbubble-microdroplet cluster composition is administered with a therapeutic agent and wherein ultrasound insonation of a targeted pathology may lead to an increase in the therapeutic effect versus using just the therapeutic agent alone. Whereas WO2015/047103 provides a broad description of the characteristics of the applied US fields during the ACT procedure, the inventors have now surprisingly found that a specific use wherein the ultrasound insonation frequencies and pressures of the method are carefully selected, provide an increased permeability of the vascular barrier, increased extravasation of the co-administered therapeutic agent and/or inflammatory cytokines and/or infiltration of activated immune cells, and avoidance of adverse effects, fully exploiting the potential of ACT.

Based on performed and planned studies, the applicant has now surprisingly found that a specific use of the ACT technology wherein the ultrasound insonation frequencies and the mechanical index of the method are carefully selected, is useful in treatment with ITAs, and that it particularly provides an improved therapeutic effect of co-administered ITA(s), compared to treating a subject with the ITA(s) alone.

A cluster composition, and a procedure for use of this in a method that enhances the therapeutic effect of ITAs, has now been identified. This uses ACT technology to generate large phase shift bubbles *in vivo* from an administered composition comprising microbubble-microdroplet clusters. In combination with local ultrasound insonation, this facilitates enhanced extravasation and uptake of separate pre-, and/or co- and/or post administered therapeutic agent(s) and/or inflammatory cytokines and enhanced infiltration of activated immune cells to a targeted pathology, providing a significant increase in the therapeutic effect over the use of the therapeutic agent alone.

Furthermore, the inventors have combined the ACT technology with clinically relevant agents and identified that with the compositions and methods of the invention, even large ITAs can be delivered to targeted, pathological sites and improve the extravasation of the ITAs from the vascular compartment to the targeted tissue interstitium.

Accordingly, the present invention provides a microbubble-microdroplet cluster composition, for use in methods that enhance delivery of ITAs to a targeted pathological tissue. Likewise, the invention provides a method that enhances delivery of ITAs to a targeted tissue interstitium comprising the administration of a microbubble-microdroplet cluster composition to a subject. The present disclosure demonstrates that a two-component microbubble-microdroplet cluster composition, wherein microbubbles as a first component are physically attached to microdroplets, as a second component, in clusters, can be used in a method which facilitates enhanced uptake of separate pre-, and/or co- and/or post administered ITA(s). The method provides a considerable increase in potential for therapeutic effect over the use of the therapeutic agent alone.

In one aspect, the invention provides a pharmaceutical composition comprising a microbubble-microdroplet cluster composition and at least one immunotherapeutic agent (ITA), for use in a method of treatment of a pathological condition of a mammalian subject, wherein the pathological condition is not pancreatic cancer, and wherein the method comprises the steps of:
(i) administering the at least one immunotherapeutic agent (ITA) to the subject;
(ii) administering the microbubble-microdroplet cluster composition to the subject; wherein the at least one ITA is pre-, and/or co- and/or post administered separate to the cluster composition;
(iii) activating a phase shift of a diffusible component of the microdroplet of the cluster composition from step (ii) by ultrasound insonation of a region of interest within said subject at a first frequency of 1 to 10 MHz and with a first mechanical index of 0.1 to 0.4;
(iv) insonating further with ultrasound at a second frequency of 0.4 to 0.6 MHz and with a second mechanical index of 0.1 to 0.3.

Further disclosed herein is but not encompassed by the wording of the claims is a method of treatment of a pathological condition of a mammalian subject with at least one immunotherapeutic agent (ITA), wherein the method comprises the steps of:
(i) administering at least one immunotherapeutic agent (ITA) to the subject;
(ii) administering a microbubble-microdroplet cluster composition to the subject; wherein the at least one ITA is pre-, and/or co- and/or post administered separate to the cluster composition;
(iii) activating a phase shift of a diffusible component of the microdroplet of the cluster composition from step (i) by ultrasound insonation of a region of interest within said subject at a first frequency of 1 to 10 MHz and with a first mechanical index of 0.1 to 0.4;
(iv) insonating further with ultrasound at second frequency of 0.4 to 0.6 MHz and with a second mechanical index of 0.1 to 0.3.

The further insonation of step (iv) facilitates extravasation of the ITA(s) administered in step (i) and infiltration of activated immune cells to the targeted pathology.

### Brief description of the drawings

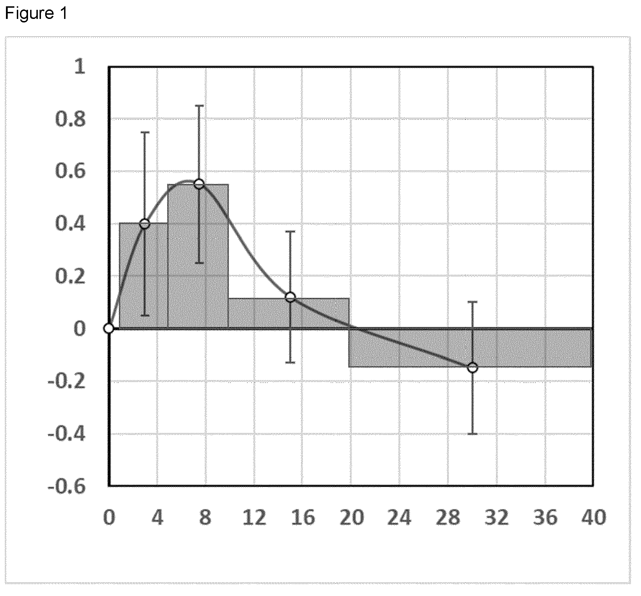

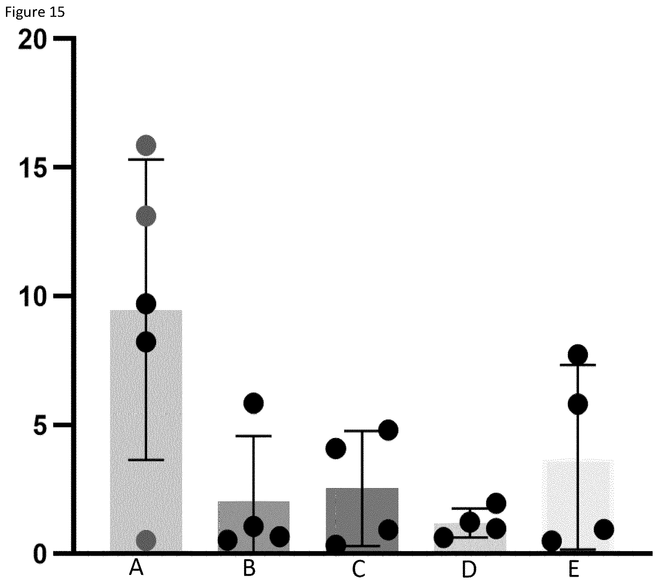

Figure 1 provides a visualization of cluster size versus in-vivo product efficacy, wherein the Y-axis shows the calculated correlation coefficient for Grey Scale enhancement from US imaging (i.e. amount of bubbles deposited after activation) and the X-axis shows cluster diameter in µm.

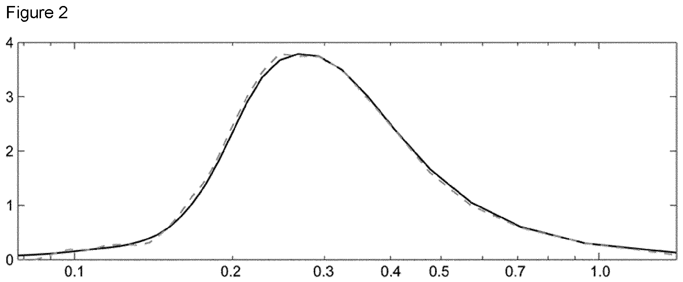

Figure 2 provides the attenuation spectrum of a population of large bubbles after ultrasound activation of a cluster composition. Y-axis shows attenuation in dB/cm. X-axis shows frequency in MHz.

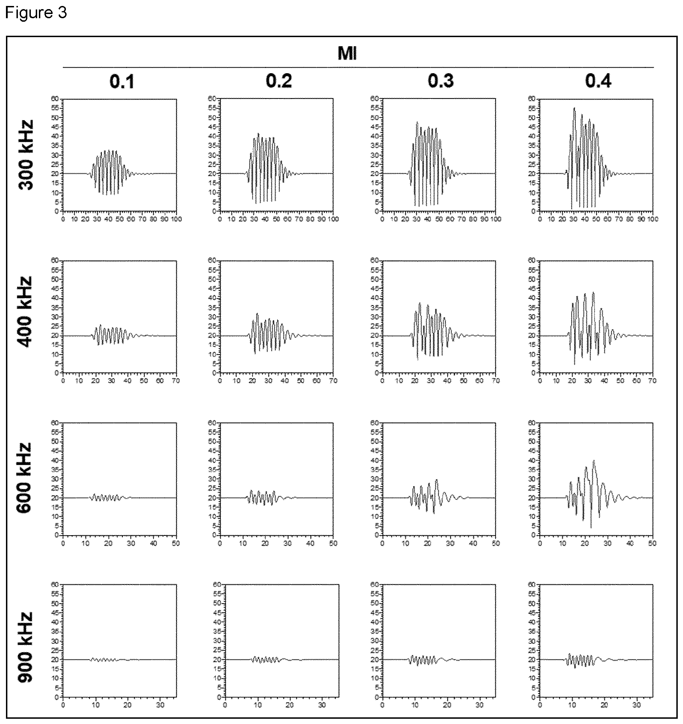

Figure 3 provides results from modelling of activated bubble response to the low frequency Enhancement insonation field for various frequencies and mechanical indices (MIs) of the incident US field. Bubble radius at rest is 20 µm and the incident field consisted of 8 cycles with a frequency and MI as stated in each panel. For each panel the Y-axis shows the radius of the activated bubble in µm. X-axis shows time in µ-seconds.

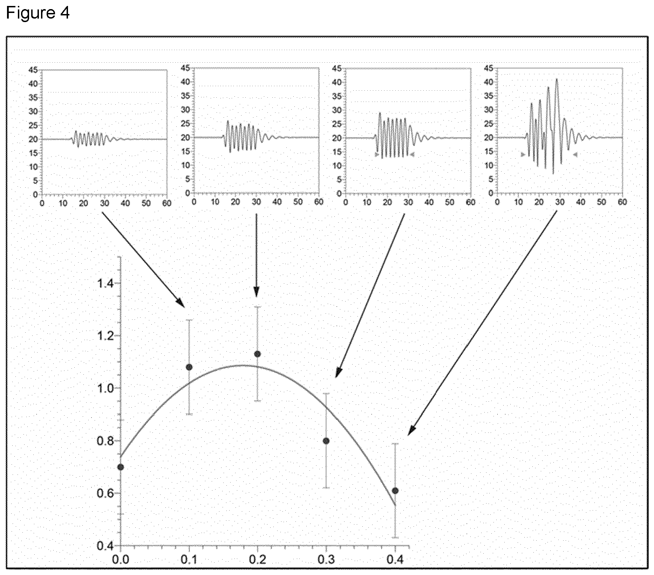

Figure 4 provides results for tumour specific uptake of a fluorescent dye (Evans Blue) upon ACT treatment with an Enhancement step insonation field at 500 kHz, with mechanical indices (MIs) at 0, 0.1, 0.2, 0.3 and 0.4 (lower panel). Y-axis shows tumour specific uptake in mg Evans Blue/mg tumour tissue. X-axis shows mechanical index. The four upper panels show results from modelling of activated bubble response to the incident US field at the different MIs investigated. Y-axis shows the radius of the activated bubble in µm. X-axis shows time in µ-seconds.

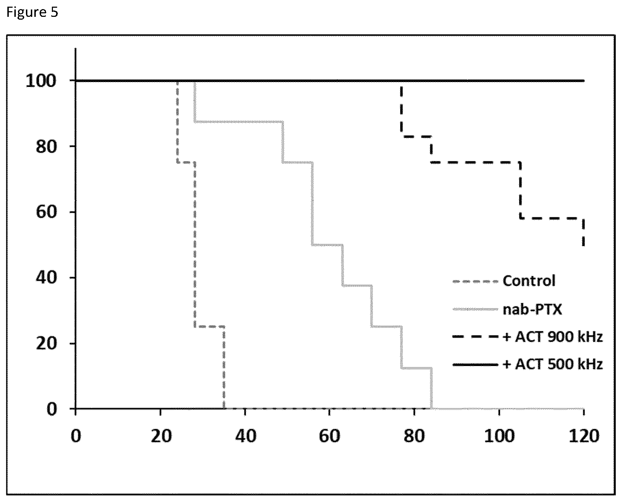

Figure 5 provides results for the therapeutic efficacy of nab-paclitaxel (nab-PTX) ± ACT for treatment of prostate cancer in mice. Y-axis shows overall survival in % of all treated animals. X-axis shows time in days after study start. Groups: saline control (dotted grey line), nab-PTX alone (solid grey line), nab-PTX + ACT with Enhancement field 500 kHz (MI 0.2) (solid black line) and nab-PTX + ACT with Enhancement field 900 kHz (MI 0.2) (dotted black line).

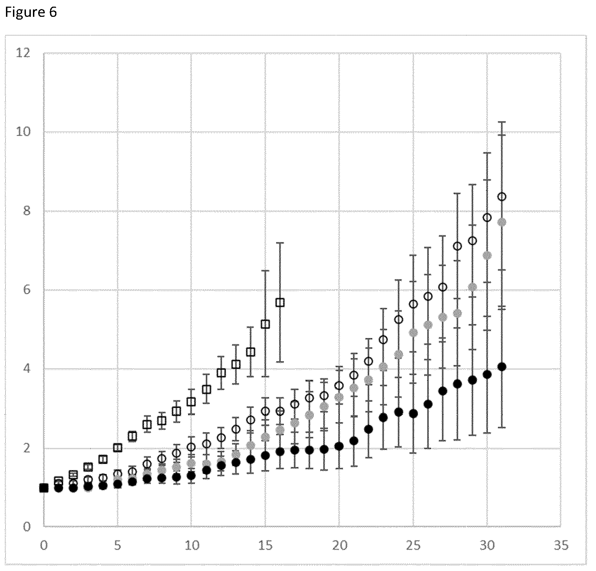

Figure 6 provides results for the therapeutic efficacy of nab-paclitaxel ± ACT for treatment of breast cancer in mice. Y-axis show normalized tumour diameter (mm). X-axis shows time after study start (days). Groups: saline control, (black, open squares), nab-PTX alone (black, open circles), nab-PTX + ACT with Enhancement field MI 0.1 (500 kHz) (grey, closed circles) and, nab-PTX + ACT with Enhancement field MI 0.2 (500 kHz) (black, closed circles).

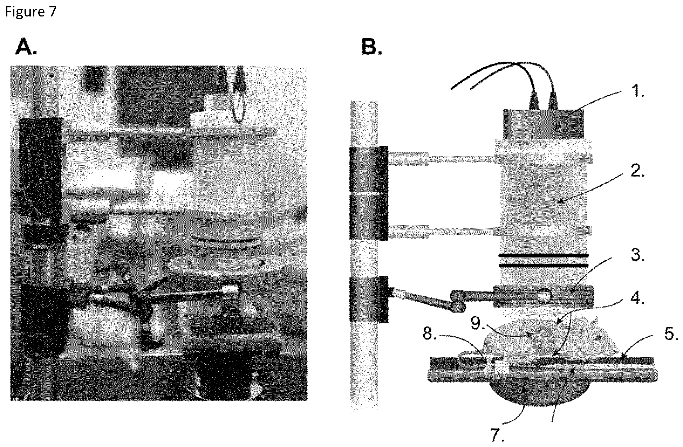

Figure 7 provides A) a setup photograph and B) a set up sketch, of the apparatus used in the study of Example 3, for application of the ACT Sonoporation procedure comprising ultrasound activation and enhancement. In Figure 7B, the numbers denote the following: 1 is a dual frequency ultrasound transducer (2.7 MHz and 500 kHz output), 2 is an ultrasound waveguide, 3 is a water bath, 4 is ultrasound gel, 5 is an ultrasound absorber pad, 6 is an injection syringe with cluster composition, 7 is a VeVo imaging table, 8 is a catheter and 9 is a tumour.

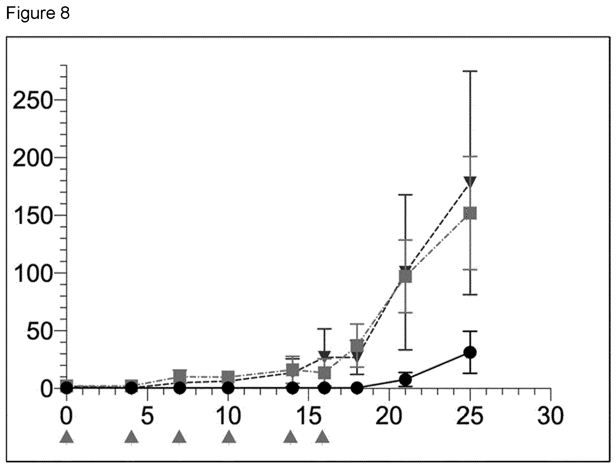

Figure 8 provides results from the study of Example 3: Therapeutic effect of reovirus in combination with ACT for treatment of hepatocellular carcinoma. Y-axis shows tumour volume as a function of time for treatment of Hepatocellular Carcinoma (HCC) in mice with an oncolytic reovirus (filled triangles), saline control (filled squares) and oncolytic reovirus in combination with ACT (filled circles). X-axis shows time from start of treatment. Grey triangles below the X-axis indicate treatment days.

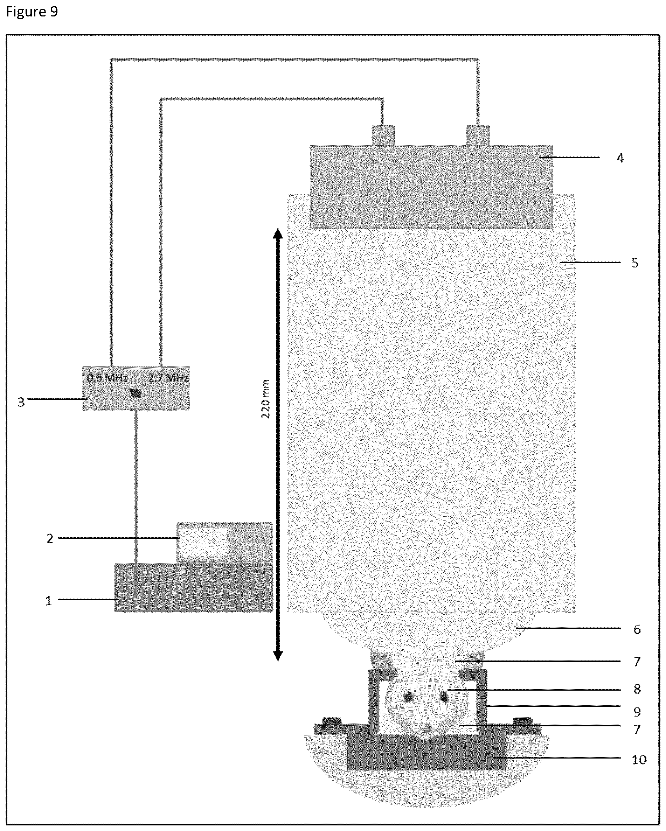

Figure 9 provides a set up sketch of the apparatus used in the study of Example 4 for application of the ACT Sonoporation procedure comprising ultrasound activation and enhancement. The numbers denote the following: 1 - Amplifier, 2 - Signal generator, 3 - Switch box between 0.5 and 2.7 MHz, 4 - Dual frequency transducer, 5 - Water filled cone, 6 - Water filled bag, 7 - Ultrasound gel, 8 - Mouse in prone position, 9 - Ear bar, 10 - Acoustic absorber pad.

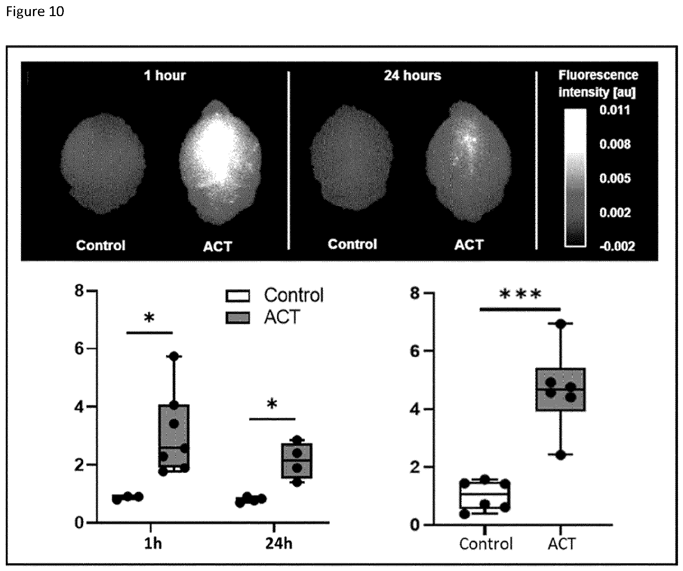

Figure 10 provides a box and whiskers plot of the results from the study of Example 4: ACT induced delivery of nanoparticles across the Blood Brain Barrier. Upper panel: representative pictures from near infrared fluorescence (NIRF) imaging of uptake of CCPM nanoparticles to brain tissue. Control and ACT treated brains at 1 and 24 hours after ACT treatment. Lower left panel: Y-axis shows uptake as measured by NIRF as percent of injected dose per grams of brain tissue for control and ACT groups, at 1 and 24 hours after ACT treatment. Black, filled circles represent individual observations. Line and asterisk indicate statistical significance (***p<0.001) between groups derived from a t-test. Lower right panel: Y-axis shows uptake as measured by confocal microscopy as percent of brain area containing nanoparticles, for control and ACT groups, 1 hour after ACT treatment. Black, filled circles represent individual observations. Line and asterisk indicate statistical significance (*p<0.05) between groups derived from a Mann-Whitney Rank sum test.

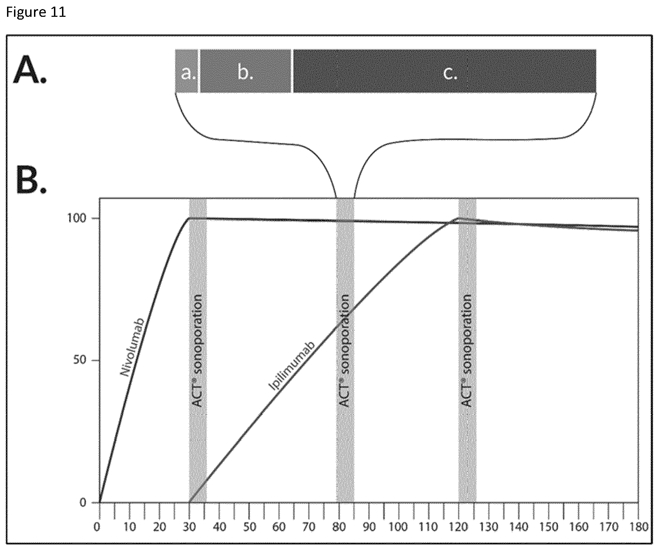

Figure 11 provides a graph of possible ACT treatments performed during treatment with the combination regimen comprising: 30min infusion of nivolumab followed by a 90min infusion of ipilimumab. The ACT procedure is applied three times during administration, as indicated by grey ACT^{®} sonoporation bars. Panel A: ACT procedure comprising a.: injection of the cluster composition, b.: activation of clusters with e.g. 60 second of regular medical imaging ultrasound insonation, and c.: enhancement step with e.g. 5 minutes of 400 to 600 kHz ultrasound insonation at an MI between 0.1 to 0.3.
   Panel B: y-axis showing plasma concentration of the administered therapeutic agents in percent of peak and x-axis showing time in minutes. In this example three ACT procedures are performed at approximately 30 minutes, 80 minutes and 120 minutes in order to cover both drugs and provide treatment of the entire region of interest.

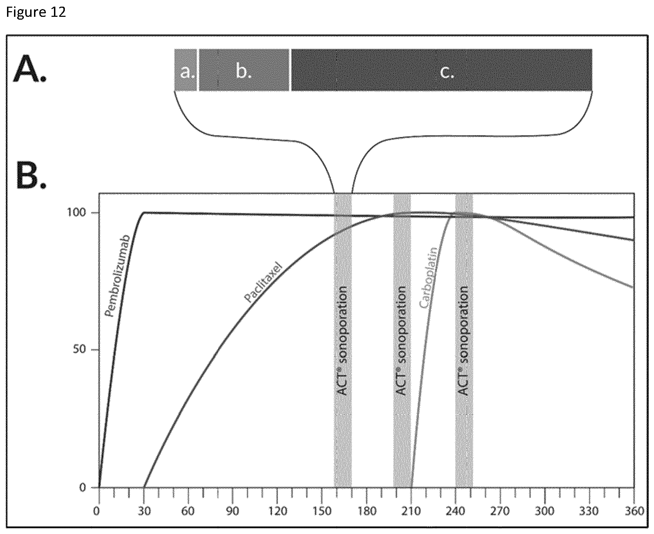

Figure 12 provides a graph of possible ACT treatments performed during treatment with the combination regimen comprising the Standard of Care combination immunotherapy plus chemotherapy regimen for treatment of metastatic squamous non-small cell lung cancer; pembrolizumab followed by paclitaxel and carboplatin.
   Panel A: ACT procedure as detailed in Figure 11.
   Panel B: y-axis showing plasma concentration of the administered therapeutic agents in percent of peak and x-axis showing time in minutes. In this example three ACT procedures are performed at approximately 160 minutes, 200 minutes and 240 minutes in order to cover all drugs and provide treatment of the entire region of interest.
Figure 13 provides a volcano plot from the study of Example 6 showing the impact of ACT on the immunogenic status of the tumours as the expression, of immune genes only, when ACT is combined with the isotype antibody (IgG) relative to treatment with IgG alone.
Figure 14 provides two graphs showing genetic effect of ACT, from the study of Example 6, as indicated by pathways that were either downregulated (A) or upregulated (B) when ACT was combined with the isotype antibody (IgG) relative to treatment with IgG alone. x-axis showing: -log10(pvalue).
   Panel A: Downregulated pathways in IgG+ACT.
   Panel B: Upregulated pathways in IgG+ACT.
   The letters denote the following: GO:0001666 - Response to hypoxia, WP4206 - Hereditary leiomyomatosis and renal cell carcinoma pathway, GO:0001525 - Angiogenesis, GO:0051235 - Maintenance of location, GO:0061061 - Muscle structure development, GO:0006936 - Muscle contraction, GO:0097435 - Supramolecular fibre organisation, GO:0043462 - Regulation of ATPase activity, GO:0030199 - Collagen fibril organisation, GO:0030239 - Myofibril assembly, GO:0044057 - Regulation of system process
Figure 15 provides a plot of preliminary immunohistochemical findings from an automated analysis of whole stained tumour sections, ref. Example 6. The proportion of cells that stained positive for CD8 T cells is shown by treatment group for individual animals (black points), and group means (bars) and standard deviations (error bars). x-axis showing, A: ACT+PD1/CTL4, B: PD1/CTL4, C: ACT+ISO, D: ISO, E: Saline

### Detailed description of the invention

### Definitions:

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art.

As used herein, Acoustic Cluster Therapy (ACT), which is further defined below, comprises the administration of a cluster composition (cf. definition below) in conjunction with at least one therapeutic agent, and subsequent application of ultrasound to a targeted region of interest within a subject (e.g. tumour, interstitial tissue or lymph node). The term "ACT treatment", or "ACT procedure", is used to describe the administration and insonation of the clusters, hence steps (ii), (iii) and (iv) of the method.

As used herein, "subject" means any human, or non-human animal individual selected for treatment or therapy, and encompasses, and may be limited to, a patient, particularly a subject with cancer or an autoimmune disease.

The phrase "therapeutically effective amount" as used herein means the amount of therapeutic agent which is effective for producing the desired therapeutic effect in a subject at a reasonable benefit/risk ratio applicable to any treatment.

The term 'microbubble' or 'regular, contrast microbubble' is used in this text to describe microbubbles with a diameter in the range from 0.2 to 10 microns, typically with a mean diameter between 2 to 3 µm. 'Regular, contrast microbubbles' include commercially available agents such as Sonazoid^{®} (GE Healthcare), Optison^{®} (GE Healthcare), Sonovue^{®} (Bracco Spa.), Definity^{®} (Lantheus Medical Imagin), Micromarker^{®} (VisualSonics Inc.) and Polyson L^{®} (Miltenyi Biotec GmbH).

The term HEPS/PFB microbubble is used in this text to describe the microbubbles formed by reconstituting a 1st component (as provided in Example 1) with 2 mL of water.

The terms 'phase shift bubbles', 'large, phase shift bubbles, 'large, activated bubbles' and 'activated bubbles' in this text is used to describe the large (> 10 µm) bubbles that form after ultrasound (US) induced activation of the cluster composition.

The term 'microdroplet' is used in this text to describe emulsion microdroplets with a diameter in the range from 0.2 to 10 microns.

'Insonation' or 'US insonation' are terms used to describe exposure to, or treatment with, ultrasound.

The term "regular medical imaging ultrasound" is used to describe ultrasound from of the shelf US scanners and probes intended for medical imaging. I.e. at a frequency between 1 to 10 MHz and an MI of < 1.9, preferably < 0.7 and more preferably < 0.4.

The term 'deposit tracer' is used in this text in relation to the activated phase shift bubbles, in the sense that the temporary mechanical trapping of the large bubbles in the microcirculation implies that the regional deposition of phase shift bubbles in the tissue will reflect the amount of blood that flowed through the microcirculation of the tissue at the time of activated bubble deposition. Thus, the number of trapped 'deposited' phase shift bubbles will be linearly dependent on the tissue perfusion at the time of deposition.

The term 'phase shift (process)' is used in this text to describe the phase transition from the liquid to gaseous states of matter. Specifically, the transition (process) of the change of state from liquid to gas of the oil component of the microdroplets of the cluster composition upon US insonation.

In this text the terms "therapy delivery / therapeutic agent(s)" and "drug delivery / drug(s)" are both understood to include the delivery of drug molecules, nanoparticles and nanoparticle delivery systems, and liposomal delivery systems, including at least one therapeutically active agent.

The term '1 st component' (or first component, or C1) is used in this text to describe the dispersed gas (microbubble) component. The term '2nd component' (or second component, or C2) is used in this text to describe the dispersed oil phase (microdroplet) component comprising a diffusible component.

The term 'cluster composition' is used in this text to describe a composition resulting from a combination, such as mixing, of the 1st (microbubble) component and the 2nd (microdroplet) component. Hence, the cluster composition, with characteristics as further described herein, refers to the formulated composition ready for administration to a subject, and for use in the Acoustic Cluster Therapy.

The term "diffusible component" is used in this text to describe a chemical component of the oil phase of the 2nd component that is capable of diffusion in *vivo* into the microbubbles in the 1st component, transiently increasing its size.

The term "pharmaceutical composition" used in this text has its conventional meaning, and in particular is in a form suitable for mammalian administration. The composition preferably comprises two separate compositions; The cluster composition (a), and the therapeutic agent (b), which are both suitable for mammalian administration such as via parenteral injection, intraperitoneal injection or intramuscular injection, either by the same or different administration routes. By the phrase "in a form suitable for mammalian administration" is meant a composition that is sterile, pyrogen-free, lacks compounds which produce excessive toxic or adverse effects, and is formulated at a biocompatible pH (approximately pH 4.0 to 10.5). Such a composition is formulated so that precipitation does not occur on contact with biological fluids (e.g. blood), contain only biologically compatible excipients, and is preferably isotonic.

The term 'Sonometry (system)' in this text refers to an in-vitro measurement system to size and count activated phase shift bubbles dynamically using an acoustic technique.

The term 'Reactivity' is used in this text to describe the ability of the microbubbles in the 1st component and the microdroplets in the 2nd component to form microbubble-microdroplet clusters upon mixing. Coulter counting is suitable for quantification of microbubble and microdroplet concentration and size distribution in C1 and C2, and for characterization of particles in the cluster composition (drug product, DP). Reactivity (R) of the cluster composition defined as; $R = \left({C}_{C 1}+{C}_{C 2}-{C}_{DP}\right) ⋅ 100 / \left({C}_{C 1}+{C}_{C 2}\right)$

Where C_{C1}, C_{C2} and C_{DP} are the number concentration observed in C1, C2 and DP, respectively. The Reactivity is hence a measure of how many of the individual microbubbles and microdroplets in C1 and C2 that are contained in cluster form in the DP. The Reactivity is also correlated to how large these clusters are (i.e. how many individual microbubbles and microdroplets comprises a single cluster). From Coulter analysis of C1, C2 and DP, the Reactivity can easily be calculated.

The terms "microbubble-microdroplet cluster" or "cluster" in this text refers to groups of microbubbles and microdroplets permanently held together by electrostatic attractive forces, in a single particle, agglomerated entity. The term 'clustering' in this text refers to the process where microbubbles in the 1^{st} component and microdroplets of the 2^{nd} component form clusters.

Within medical ultrasound, acoustic power is normally described by "the Mechanical Index" (MI). This parameter is defined as the peak negative pressure in the ultrasound field (PNP) divided be the square root of the centre frequency of the ultrasound field in MHz ( *F_{c}* ) [American Institute of Ultrasound in Medicine. Acoustic Output Measurement Standard for Diagnostic Ultrasound Equipment. 1st ed. 2nd ed. Laurel, MD: American Institute of Ultrasound in Medicine; 1998, 2003]. $MI = \frac{PNP}{\sqrt{{F}_{c}}} .$

Regulatory requirements during medical US imaging are to use a MI less than 1.9. During US imaging with microbubble contrast agents, an MI below 0.7 is recommended to avoid detrimental bio-effects such as micro-haemorrhage and irreversible vascular damage and using an MI below 0.4 is considered "best practise". It should be understood that when referring to MI in the text, this reflects the in-situ MI, i.e. the MI applied to the targeted region of interest.

The term 'activation' or "activation step" in the context of the ACT procedure in this text, refers to the induction of a phase shift of microbubble-microdroplet clusters by ultrasound (US) insonation, i.e. the generation of large, activated bubbles.

The term frequency is defined as number of (ultrasound) cycles per second (Hz). When used herein the term designate the centre frequency of the applied sound field.

The term "enhancement" or "enhancement step", in the context of the ACT procedure in this text, refers to the induction of volume oscillations of the large, activated bubbles and ensuing biomechanical effects, by US insonation.

The term "resonance frequency" or "microbubble resonance frequency", when used in this text, is meant to describe the acoustic resonance frequency of a single bubble in an infinite matrix domain (neglecting the effects of surface tension and viscous attenuation). The resonant frequency is given by: $f = \frac{1}{2 πa} {\left(\frac{3 {γp}_{A}}{ρ}\right)}^{1 / 2}$ where a is the radius of the bubble, γ is the polytropic coefficient, p_{A} is the ambient pressure, and ρ is the density of the matrix.

The term "immunotherapeutic agent" relates to a treatment modality that is intended to treat a disease or condition by inducing, enhancing or suppressing an immune response. It also relates to manipulation of immune response such that inappropriate immune responses are modulated into more appropriate ones in the context of certain diseases.

The term "molecular target" is to be understood as a molecule or a group of molecules in human cells that is intrinsically associated with a particular disease process such as etiology, progression, and/or drug resistance. To be referred to as a target, there must be evidence that by addressing the target with a small molecule, biologic product, or other intervention, a desired therapeutic effect is produced resulting in the alteration of the disease process. In this text, molecular targets are named according to the Human Cell Differentiation Molecules (HCDM) Council nomenclature, as agreed during the ongoing series of Human Leucocyte Differentiation Antigens (HLDA) Workshops, officially approved by the International Union of Immunological Societies (IUIS) and sanctioned by the World Health Organization (WHO). Mostly, molecular targets are named in the text by their Cluster of Differentiation (CD) number, as published by HCDM. However, other scientifically accepted terms detailing the nature of the target are also used, such as CTLA-4, designating Cytotoxic T lymphocyte antigen 4 and PD-1, designating Programmed Cell Death protein 1 etc.

The invention provides a cluster composition for use in a method of treatment with immunotherapeutic agents (ITAs), and particularly for treatment of cancers and autoimmune diseases. The invention uses ACT technology to generate large phase shift bubbles in vivo from an administered pharmaceutical composition comprising microbubble-microdroplet clusters, and which facilitates delivery and uptake of separate pre-, and/or co- and/or post administered therapeutic agent(s). The therapeutic effect of the therapeutic agent is considerably increased compared to administration of the agent alone, due to biomechanical mechanisms in the microvasculature, as further explained below. The present disclosure demonstrates that a specific use of the ACT technology comprising two steps of ultrasound insonation at different frequencies and mechanical indices enables an increased delivery of separately administered ITAs and also enhancement of activated immune cell Infiltration. The activated phase shift bubbles are approximately 1000 times larger in volume (10 times in diameter) than typical, regular contrast microbubbles.

The invention provides a cluster composition, for use in a method of delivery of at least one ITA to a targeted tissue interstitium, and for enhancement of activated immune cell infiltration, wherein the method includes phase shift technology to generate large phase shift bubbles in vivo from an administered cluster composition, and which facilitates delivery and uptake of separately administered ITA(s). The composition for use and the method of the invention potentiate the therapeutic effect of the separately co-administered therapeutic agent, providing an improved therapeutic outcome, compared to treatment without the use of the compositions of the invention.

Hence, in a first aspect, the invention provides a pharmaceutical composition comprising a microbubble-microdroplet cluster composition and at least one immunotherapeutic agent (ITA), for use in a method of treatment of a pathological condition of a mammalian subject, wherein the method comprises the steps of:
(i) administering at least one immunotherapeutic agent (ITA) to the subject;
(ii) administering the microbubble-microdroplet cluster composition to the subject; wherein the at least one ITA is pre-, and/or co- and/or post administered separate to the cluster composition;
(iii) activating a phase shift of a diffusible component of the microdroplet of the cluster composition from step (ii) by ultrasound insonation of a region of interest within said subject at a first frequency of 1 to 10 MHz and with a first mechanical index of 0.1 to 0.4;
(iv) insonating further with ultrasound at a second frequency of 0.4 to 0.6 MHz and with a second mechanical index of 0.1 to 0.3.

The method steps, and particularly step (iv), facilitates extravasation of the ITA(s) administered in step (i) and infiltration of activated immune cells to the targeted pathology.

In one embodiment, the microbubble-microdroplet cluster composition and the at least one ITA may be regarded as a pharmaceutical composition, preferably comprising the two as separate compositions. Hence, the invention provides a pharmaceutical composition comprising a microbubble-microdroplet cluster composition for use in a method of treatment of a pathological condition of a mammalian subject with at least one ITA, wherein the at least one ITA is pre-, and/or co- and/or post administered separate to the cluster composition.

In addition to the required steps (i) to (iv), the method may comprise a step (iib) of optionally imaging the clusters using ultrasound imaging to identify the region of interest for treatment within said subject. Such step should be performed after step (ii) and before step (iii).

The Acoustic Cluster Therapy (ACT ^{®}) technology used in the invention is an ultrasound-mediated targeted drug delivery platform that utilizes microbubble-microdroplet clusters, activated by the application of ultrasound, to create localized openings or fenestrations in the vasculature of the targeted tissue, leading to a transient increase in vascular permeability and thereby allowing drugs and activated immune cells to better penetrate the pathological interstitium. The compositions and methods of the invention hence open fenestrations in the endothelial barrier and may improve extravasation and intratumoural distribution of activated immune cells (e.g. T-cells) and improve intratumoural uptake and distribution of antibodies and stimulate release of tumour antigens. The van Wamel paper demonstrated the potential of ACT to enhance therapeutic regimens with regular, small molecule chemotherapeutic agents. However, it does not point towards the possibility of combining ACT with larger drug constructs such as ITAs, where this delivery concept has now been found particularly useful. Furthermore, it does not note the possibility of applying ACT for treatment of autoimmune disease. And finally, ACT is consistently described in the state of the art as a concept to enhance local delivery of a drug molecule to targeted tissue. The inventors have found that, as ACT opens fenestrations in the endothelial barrier, in addition to improve intratumoural uptake and distribution of antibodies and stimulation of release of tumour antigens, the technique may also be applied to improve extravasation and intratumoural distribution of activated immune cells (e.g T-cells). In essence, the ACT concept resolves most of the limiting attributes related to the use of regular contrast microbubbles for sonoporation;
1) it generates large, activated bubbles *in-vivo* that are approximately three orders of magnitude greater than regular contrast microbubbles, exerting much larger biomechanical effects,
2) these bubbles are in close contact with the endothelial and are hence much more effective, and
3) they are functional in a stable cavitation mode, using low MI US fields, avoiding the safety concerns and vascular damage related to inertial cavitation.

The current invention is partly based on findings from several pre-clinical studies. In Example 3, the applicant has investigated the ability of ACT in enhancing the therapeutic effects of ITAs in the form of oncolytic virus, for treatment of hepatocellular carcinoma. As can be observed from the results displayed in Table 3 and visualized in Figure 8, treatment with reovirus alone showed no significant inhibition of tumour growth at the investigated dose when compared to the saline control group. However, when combining the same dose of virus with ACT treatment a marked and significant tumour inhibition was observed, with a > 95% reduction in tumour volume at Day 25 vs. virus alone. This study demonstrates the ability of the ACT concept to enhance the therapeutic efficacy of a large construct ITA for treatment of a localized pathological condition.

In Example 4, the applicant has investigated the ability of ACT in delivery of cytotoxic nanoparticles across the blood-brain barrier (BBB). The BBB is a highly selective semipermeable border of endothelial cells that prevents solutes in the circulating blood from non-selectively crossing into the extracellular fluid of the central nervous system where neurons reside. The blood-brain barrier is formed by endothelial cells of the capillary wall, astrocyte end-feet ensheathing the capillary, and pericytes embedded in the capillary basement membrane. This system allows the passage of some selected, small molecules by passive diffusion, as well as the selective and active transport of various nutrients, ions, organic anions, glucose, water and amino acids that are crucial to neural function. The blood-brain barrier restricts the passage of pathogens, the diffusion of solutes in the blood, and large or hydrophilic molecules into the cerebrospinal fluid, while allowing the diffusion of small hydrophobic molecules and small polar molecules. The barrier also restricts the passage of peripheral immune factors, like signalling molecules, antibodies, and immune cells, into the CNS, thus insulating the brain from damage due to peripheral immune events.

The BBB hence represents the tightest vascular barrier in the body. Unimpaired, it completely closed to therapeutic agents larger than approximately 4-500 Daltons. Example 4 demonstrates the ability of ACT to enable uptake of large constructs like nanoparticles into the brain tissue and indirectly demonstrates the utility of the ACT concept for localized delivery of large ITAs such as mAbs across any vascular barrier in the body. Compared to dosing of nanoparticles alone, a 280-290% increase in uptake to brain tissue was observed when combining nanoparticles with ACT.

In Example 2 the applicant has investigated attributes of the US fields applied during the second insonation step (the Enhancement step) and their effect on the functionality of the applied procedure. Surprisingly, and contrary to the teachings of WO2015/047103, where the preferred frequency range is disclosed to between 0.2 to 1 MHz and an MI of < 0.4 is suggested, the applicant has found that the functionality of the concept is quite sensitive to these parameters. Based on these studies, the applicant has found that a preferred frequency range is between 0.4 to 0.6 MHz and that the MI applied should be kept to more than 0.1, but less than 0.3. With lower frequencies and higher MI during the Enhancement step, step (iv), the applicant has surprisingly found that the activated bubble oscillations induced are too strong, leading to a significant loss of efficacy and vascular damage. On the other hand, as demonstrated from the comparative example investigating 0.5 vs. 0.9 MHz (cf. Figure 3), with higher frequencies and lower MIs, the bubble oscillations induced are too small, leading to a lack of sufficient biomechanical effects and hence a significant loss in therapeutic efficacy.

The applicant has found that the method of the invention, using the ACT concept, is an effective way to overcome biological barriers for improved uptake of therapeutic agents and activated immune cells. This has been found to be particularly beneficial for treatment with immunotherapeutic agents because of the generally low rate of drug extravasation this class of agents displays. The method of the invention, and particularly the enhancement step (iv) facilitates extravasation of the ITA(s) administered in step (i) and infiltration of activated immune cells to the targeted pathology.

Hence, the administered microdroplet-microbubble clusters are triggered by a localized insonation method, comprising ultrasound insonation at different frequencies. When the clusters are insonated with ultrasound (activated) the oscillating microbubbles initiate an instant vaporisation (phase-shift) of the attached microdroplet. The enlarged resulting bubbles have been shown to form in capillary sized vessels in vivo and are further excited by low frequency ultrasound to induce biomechanical effects that facilitate extravasation and increase drug and/or cell penetration in the insonated tissue. It has now been identified that the method of treatment should comprise two steps of insonation, comprising one step of activation and one step of enhancement. Different ultrasound frequencies are used in these insonation steps. During the activation step (step (iii) of the method), the microbubbles of the clusters oscillate and transfer energy to the microdroplets inducing droplet vaporization, forming large ACT bubbles designed to transiently lodge in the microvasculature. Hence, the clusters are activated to produce large bubbles by application of external ultrasound energy, after administration, such as from a clinical ultrasound imaging system, under imaging control, wherein the further insonation at the lower frequency induces biomechanical effects, extravasation and increased drug penetration.

The steps of the methods of the invention are further described below: Administration, step i) and ii): The cluster composition is administered to said mammalian subject parenterally, preferably intravenously, and the therapeutic agent is pre-, and/or co- and/or post administered separate to the cluster composition as a separate composition, as further described under "Administration routes" herein.

Optional imaging, step iib): The clusters are not activated at low MI (below the cluster activation threshold of approx. 0.1) allowing standard medical ultrasound contrast agent imaging to be performed, for example to identify tumour micro vascular pathology without activation of the clusters. Hence, in one embodiment the method includes a step of imaging, using low MI contrast agent imaging modes (MI < 0.1) to image the microbubble component, i.e. the dispersed gas, without activation of the clusters, to identify the pathological location for treatment. Hence, as the clusters are not activated at low MI (below the activation threshold) standard medical ultrasound contrast agent imaging may be performed, prior to the activation step, for example to identify tumour microvascular pathology.

Activation, step iii): The acoustic resonance frequency of the microbubble component of the clusters is within the diagnostic frequency range (1-10 MHz). When the cluster composition has been administered to the subject, activation of the clusters is readily obtained by insonation of a region of interest with standard diagnostic ultrasound imaging pulses used for example in conventional medical ultrasound abdominal and cardiac applications, at low to mid-range mechanical indices, i.e. an MI below 0.4, but above 0.1.

Activating the phase shift is performed by ultrasound insonation of a region of interest within said subject at a first frequency of 1 to 10 MHz, such as more particularly at 2-3 MHz. Activation of the clusters to phase shift to produce larger (10 µm or more in diameter) activated bubbles can be achieved with a clinical imaging system to within millimetre spatial resolution by employing imaging pulses. Upon activation, the oil in the microdroplet vaporises and the resulting large, activated bubble, transiently deposits in the microvasculature.

In one embodiment, the activation, i.e. the US insonation at the first frequency, starts immediately after each administration of the cluster composition, such as within 20 seconds, and lasts for e.g. 60-120 seconds. In one embodiment, when imaging is performed (step iib), this is performed before and during injection of the cluster composition, and then the activation clock is started when inflow of contrast is seen.

The activation under medical ultrasound imaging control using the imaging pulses allows spatially targeted activation of the clusters in the tissue region being interrogated by the ultrasound field. After activation, the large phase shift bubbles produced are temporarily trapped in the microvasculature of the targeted region of interest due to their size. The resulting large phase shift bubbles are approximately 1000 times the volume of the emulsion microdroplet vaporised (a 20 µm bubble diameter from a 2 µm diameter oil microdroplet). The scattering cross sections of these large phase shift bubbles are orders of magnitude greater than the scattering cross sections of the micron sized microbubbles comprised in the clusters before activation. As a result, the large phase shift bubbles produce copious backscatter signal and are readily imaged in fundamental imaging mode with diagnostic imaging systems. The resonance frequencies of the large phase shift bubbles are also an order of magnitude lower (cf. Example 2) than the resonance frequencies of the microbubbles comprised in the clusters before activation.

Enhancement, step iv): This step uses a second frequency, which is lower than used during the activation step and induces controlled volume oscillations of the ACT-bubbles, thereby exerting biomechanical forces on the capillary wall and enhancing drug delivery locally. This further application of low frequency ultrasound after activation and deposition, facilitates enhancement of delivery mechanisms by effectively overcoming biological barriers to increase the efficiency of drug delivery to spatially targeted tissue. These mechanisms may include the process of sonoporation i.e. a process where insonation, and ensuing volume oscillation, of microbubbles in the vascular compartment increases the permeability of the vascular barrier. In other words, the ACT procedure increases the permeability of the endothelial wall and hence enhances the extravasation, distribution and cellular uptake of the therapeutic drug or activated immune cells. Other mechanisms, such as generation of cellular signalling for enhanced therapeutic effect, mechanical breaking down of interstitial structures that enhances drug penetration etc. may also be induced.

It will be appreciated that for the composition for use and method of the invention, this further insonation of the large, activated bubbles with the application of low frequency ultrasound further enhances the uptake of the therapeutic agent(s) or cells. Hence, it has been found that the application of low frequency ultrasound, close to the resonance frequencies of the large, activated bubbles, can be used to produce mechanical bio-effect mechanisms to enhance the permeability of the vasculature and/or sonoporation and/or distribution in the interstitium and /or endocytosis and hence enhance the therapeutic outcome.

Application of acoustic fields commensurate with the resonance frequencies of the larger phase shift bubbles produces relatively large radial oscillations at MI's within the medical diagnostic range. Thus, low frequency ultrasound, in the range 0.2 to 1 MHz, most preferably 0.4 to 0.6 MHz can be applied to produce the bio-effect mechanisms that enhance the uptake of the administered drug, and hence facilitates extravasation. Surprisingly, as demonstrated in Example 2, a larger therapeutic benefit has been found when the activated bubbles are insonated to induce enhanced uptake by applying ultrasound e.g. in the range of 0.4 to 0.6 MHz, e.g. 500 Hz as used in the Examples, using MIs of 0.1 to 0.3, e.g. 0.2. It has been found that after activation in-vivo, the volume weighted mean diameter of the activated bubbles is approx. 20 µm. The resonance frequency of free microbubbles of this size is approx. 0.33 MHz. However, it is expected that the resonance frequency of such a bubble is somewhat higher, when trapped in a micro vessel. Hence, a most preferred frequency range for the low frequency enhancement step is 0.4-0.6 MHz. Exploiting the resonance effects of the activated bubbles allows better control of initiation of these bio-effects at lower acoustic intensities and at lower frequencies than possible with other technologies. Coupled with the fact that the large phase shift bubbles are activated and deposited in the tissue microvasculature under imaging control (allowing spatial targeting of the large, activated bubbles in tissue), and their prolonged residence time, allows more efficient and controlled implementation of the drug delivery mechanisms.

Hence, in one embodiment, the method comprises an enhancement step (step iv) of insonating at a second frequency in the range of 0.4 to 0.6 MHz. The **MI** for this enhancement step is preferably below 0.3 but above 0.1, preferably above 0.15. If the MI applied during the enhancement step is lower than 0.1 it is expected that the biomechanical effects generated will be insufficient and, hence, reduce the therapeutic benefit significantly. If the MI applied during the enhancement step is above 0.3, it is expected that the biomechanical effects generated will be too strong and induce unwanted effects such as vascular damage or shut down, reducing the therapeutic benefit significantly.

The insonation with low frequency ultrasound follows the activation step and should typically last for 3 to 10 minutes, such as for about 5 minutes. There is preferably an immediate start of step (iv) after step (iii).

In the method of the invention, when activating a phase shift of a diffusible component of the second component of the cluster composition by ultrasound insonation of a region of interest within the subject, the microbubbles of the clusters are enlarged by the diffusible component to give enlarged bubbles which are localised at the region of interest due to transient trapping in the microcirculation at the region of interest. The further ultrasound insonation at low frequency (step iv) facilitates extravasation of the therapeutic agent(s) administered, and infiltration of activated immune cells to the targeted pathology. The method hence facilitates enhanced extravasation and uptake of the separate pre-, and/or co- and/or post administered therapeutic agent(s) and/or enhanced infiltration of activated immune cells to a targeted pathology. Hence, an increased permeability of the vascular barrier, increased extravasation of the co-administered therapeutic agent and/or of inflammatory cytokines and/or infiltration of activated immune cells is provided by the invention,

Hence, the invention further provides a microbubble-microdroplet cluster composition for use in a method of extravasation and uptake of at least one separate pre-, and/or co- and/or post administered ITA(s) and/or of inflammatory cytokines and/or an enhanced infiltration of activated immune cells to a targeted pathology of a mammalian subject, wherein the method comprises the steps of:
(i) administering at least one immunotherapeutic agent (ITA) to the subject;
(ii) administering the microbubble-microdroplet cluster composition to the subject; wherein the at least one ITA is pre-, and/or co- and/or post administered separate to the cluster composition;
(iii) activating a phase shift of a diffusible component of the microdroplet of the cluster composition from step (i) by ultrasound insonation of a region of interest within said subject at a first frequency of 1 to 10 MHz and with a first mechanical index of 0.1 to 0.4;
(iv) insonating further with ultrasound at a second frequency of 0.4 to 0.600 MHz and with a second mechanical index of 0.1 to 0.3.

Further disclosed herein but not encompassed by the wording of the claims is a method of extravasation and uptake of at least one separate pre-, and/or co- and/or post administered ITA(s) and/or enhanced infiltration of activated immune cells to a targeted pathology of a mammalian subject, comprising the above steps. The steps and embodiments of the above method of improving or enhancing extravasation and uptake of at least one ITA, or infiltration of activated immune cells to a targeted pathology are as disclosed for the first aspect directed to treatment.

In some embodiments of these methods, it is not the ITA itself which delivers the therapeutic effect, but rather inflammatory cytokines (e.g. IL-1, IL-12, IL-18, TNFα or INFγ) and/or activated immune cells (e.g. activated T-cells, e.g. CD3, CD4 or CD8 positive cells) where the ITA blocks receptors and enable the immune cells to initiate the required cytotoxic processes towards the pathological cells in question.

### The cluster composition:

In the method of the invention, a premixed cluster composition is administered to the subject, in addition to the separate administration of one or several immunotherapeutic agents. The administered clusters can be activated by ultrasound. The composition of clusters is a premixture of microbubbles (first component) and microdroplets (second component) resulting in small microbubble-microdroplets clusters held together by electrostatic forces. The microdroplets typically comprise an oil component that has a boiling temperature of < 50 °C, and low blood solubility. The cluster composition, i.e. the combination of the first and second components, comprises clusters of gas microbubbles and oil microdroplets, i.e. is a suspension or dispersion of individual microbubbles and microdroplets in the form of stable microbubble-microdroplet clusters. Analytical methodologies for quantitative detection and characterisation of said clusters are described in Example 1. In this text, the term "clusters" refers to groups of microbubbles and microdroplets permanently held together by electrostatic attractive forces, in a single particle, agglomerated entity. The content and size of the clusters in the cluster composition is essentially stable over some time (e.g. > 3h) after combining the first and second components in vitro, i.e. they do not spontaneously disintegrate, form larger aggregates or activate (phase shifts) spontaneously, and are essentially stable over some time after dilution, even during continued agitation. It is hence possible to detect and characterize the clusters in the cluster composition with various analytical techniques that require dilution and/or agitation. Furthermore, the stability of the cluster composition allows for performing the necessary clinical procedures (e.g. reconstitution, withdrawal of dose and administration). The first and second components, and the cluster composition, are prepared according to Good Manufacturing Practice (GMP).

In one embodiment, the cluster composition comprises a suspension of clusters in an aqueous biocompatible medium, where said clusters have a mean diameter in the range 1 to 10 µm, and a circularity < 0.9 and comprises:
(i) a first component which comprises a gas microbubble and first stabilizer to stabilize said microbubble; and
(ii) a second component which comprises a microdroplet comprising an oil phase and second stabilizer to stabilize said microdroplet, where the oil comprises a diffusible component capable of diffusing into said gas microbubble so as to at least transiently increase the size thereof;
where the microbubbles and microdroplets of said first and second components have opposite surface charges and form said clusters via attractive electrostatic interactions.

After combining the first and second components (in vitro), e.g. by reconstituting the microbubble component, such as a lyophilized microbubble component, with a microdroplet component in the form of an emulsion, the prepared cluster composition according to the invention displays an in-use stability which is suitable for its intended use and display stable characteristics for a suitable time window for administration, such as more than 1h or preferably more than 3h from combining the components. The cluster composition is to be administered to the subject within this time window.

Each cluster in the cluster composition comprises at least one microbubble and one microdroplet, typically 2-20 individual microbubbles/microdroplets, and a cluster typically has a mean diameter in the range of 1 to 10 µm and can hence flow freely in the vascular compartment. They are further characterized and separated from individual microbubbles and microdroplets by a circularity parameter. The circularity of a two-dimensional form (e.g. a projection of a microbubble, microdroplet or microbubble-microdroplet cluster) is the ratio of the perimeter of a circle with the same area as the form, divided by the actual perimeter of the form. Accordingly, a perfect circle (i.e. a two-dimensional projection of a spherical microbubble or microdroplet) has a theoretical circularity value of 1, and any other geometrical form (e.g. projection of a cluster) has a circularity of less than 1. Said clusters of the invention have a circularity < 0.9. The definition of circularity parameter is further provided in WO2015/047103.

According to the invention, compositions comprising clusters with a mean size in the range of 1-10 µm, and particularly 3-10 µm, and defined by a circularity of < 0.9 are considered particularly useful, as demonstrated in the examples. In one embodiment the mean cluster diameter is in the range of 3-10 µm, and preferably 4-9 µm, more preferably 5-7 µm. Clusters in this size range are free-flowing in the vasculature before activation, they are readily activated by US insonation and they produce activated bubbles that are large enough to deposit and lodge temporarily in the microvasculature, such as e.g. in cancerous or inflamed tissue. The microbubbles in the clusters permit efficient energy transfer of ultrasound energy in the diagnostic frequency range (1-10 MHz), i.e. upon activation, and allow vaporisation (phase shift) of the emulsion microdroplets at low MI (preferably under 0.4, but more than 0.1) and diffusion of the vaporized liquid into the microbubbles and/or fusion between the vapour bubble and the microbubble. The activated bubble then expands further from the inwards diffusion of matrix gases (e.g. blood gases) to reach a volume weighted, median diameter of more than 10 µm, but less than 40 µm.

The formation of these clusters, i.e. by preparing a cluster composition from the first component and the second component prior to administration, is a prerequisite for an efficient phase shift event and their number and size characteristics are strongly related to the efficacy of the composition, i.e. its ability to form large, activated (i.e. phased shifted) bubbles *in-vivo*, and has been found to be a pre-requisite for its intended functionality *in-vivo.* The number and size characteristics can be controlled through various formulation parameters such as, but not limited to; the strength of the attractive forces between the microbubbles in the first component and the microdroplets in the second component (e.g. the difference in surface charge between the microbubbles and microdroplets): the size distribution of microbubbles and microdroplets: the ratio between microbubbles and microdroplets: and the composition of the aqueous matrix (e.g. pH, buffer concentration, ionic composition and strength). When the cluster composition has been prepared and is to be administered, the mean circular equivalent diameter of the clusters formed should preferably be larger than 3 µm, more preferably between 5 to 7 µm, but smaller than 10 µm.

The concentration of clusters in the combined preparation (cluster composition) should be more than 3 million/mL, preferably be more than 10 million/mL, more preferably more than 20 million/mL. As shown in Example 1, a cluster composition for use according to the invention had a cluster concentration of 40 - 44 million/mL with a mean diameter of 5.8-6.2 µm measured 0-3 hours after mixing of the first and second components.

Figure 11 (left panel) of applicant's WO2015047103 shows the correlation between the in-vivo enhancement in ultrasound signal (measured as increase in Grey Scale units) upon injection and activation of cluster compositions with variable concentration of clusters between 5 to 10 µm. The level of GS enhancement observed is a direct measure of the amount of large, activated bubbles generated and retained in the targeted tissue, and represents a direct measure of potential for increased extravasation and therapeutic benefit (i.e. product efficacy). In one embodiment, the composition for administration should comprise at least 3 million/mL of clusters with a diameter between 5-10 um. Such a minimum would, according to Figure 11 of applicant's WO2015047103, assure an enhancement of > 150 GS units, and a certain, minimum level of product efficacy and therapeutic benefit. In another embodiment, the concentration of clusters in size range 1-10 µm, should be at least 10 million/mL, such as at least around 25 million/ml.

Hence, drug delivery to a targeted extravascular tissue and treatment with immunotherapeutic agents according to the invention is achieved by the use of a two component, microbubble-microdroplet formulation system (i.e. the cluster composition) where microbubbles in a first component, via electrostatic attraction, are physically attached to micron sized emulsion microdroplets in a second component prior to administration. The composition for use in a method of treatment, according to the invention, provides improved uptake of the at least one ITA, resulting in a beneficial treatment, including e.g. a reduction in tumour volume or total remission. The invention hence further provides a two-component formulation system for preparation of a composition of microbubble-microdroplet clusters dispersed in an aqueous biocompatible medium, the formulation system comprising the first and second components as disclosed above, for use in the methods of the invention.

It should be appreciated that, whereas the direct mechanism of action, i.e. the produced mechanical and/or thermal bio-effect increases delivery and enhancing distribution of the therapeutic agent, the nature of these bio-mechanical effects is a direct result of the chemical attributes of the cluster composition, i.e. a result of the chemical composition and properties of the clusters. For example, the longevity of a gas bubble in an aqueous matrix is inversely proportional to the solubility and diffusion coefficient of the gas in the matrix, and proportional to the density of the gas. Hence, a bubble made from a heavy gas with low solubility and diffusivity will grow bigger and last longer than a bubble made from a light gas with high solubility and diffusivity. As an example, a 5 µm microbubble of perfluorobutane will last 500 times longer in water than a 5 µm microbubble of air. The chemical composition of the microdroplet component will hence govern the longevity of the activated bubbles in-vivo and, hence, the level of bio-mechanical force that can be induced and the therapeutic effect level that can be achieved with the ACT procedure. From this, perfluorated oils will be particularly useful for use in microdroplets of the second component, as gases from such are very low in water solubility and diffusivity, and high in density.

If comparing the compositions and methods of the invention with methods wherein free-flowing, regular contrast microbubbles are used, the large phase shift microbubbles generated *in vivo* from the administered clusters of the current invention are entrapped in a segment of the vessels and the activated bubble surface is in close contact with the endothelium. In addition, the volume of an activated bubble is typically 1000 times that of a regular microbubble. At equal Mechanical Index (MI), insonated at a frequency close to resonance for both bubble types (approximately 0.5 MHz for phase shift microbubbles and approximately 3 MHz for regular contrast agent microbubbles) it has been shown that the absolute volume displacement (i.e. biomechanical force exerted) during oscillations are three orders of magnitude larger with the phase shift bubbles than with a regular contrast microbubble. Hence, insonation of phase shift bubbles will produce completely different levels of bio-mechanical effects, with significantly larger effect size and penetration depth than during insonation of regular contrast microbubbles, as demonstrated in [Ng et al., Abstract A099: Acoustic Cluster Therapy enhances the efficacy of chemotherapeutic regimens in patient-derived xenograft mouse models for pancreatic ductal adenocarcinoma, AACR Conference on Molecular Targets and Cancer Therapeutics, Boston, October 2019]. The bio-effects observed with free-flowing, regular contrast microbubbles are likely dependent upon cavitation mechanisms, with ensuing safety concerns such as micro-haemorrhage and irreversible vascular damage. The larger phase shift bubbles from the clusters however, can be oscillated in a softer manner (lower MI, e.g. < 0.3), avoiding cavitation mechanisms, but still induce sufficient mechanical force to enhance the uptake of drug from the vasculature and into the target tissue. The trapping of the large phase shift bubbles will also act as a deposit tracer. This further allows quantification of the number of activated clusters and perfusion of the tissue and allows contrast agent imaging of the tissue vasculature to identify the spatial extent of the tissue to be treated.

The chemical composition of the administered clusters, and the processes taking place during activation of the clusters, are crucial for the effect of the clusters. For instance, chemistry of the encapsulated oil droplet influences the amount of activated bubbles that deposits upon US insonation as well as their longevity in-vivo. Physicochemical attributes of the oil, such as vapour pressure, boiling point and water solubility all correlate with the amount of activated bubbles that deposit and the time they remain deposited. For a C4-C6 homologue, perfluorated hydrocarbon chain, the amount of activated bubbles and their longevity increase with the length of the chain, as the water solubility and vapour pressure decrease and the boiling point increases. Further it should be noted that the activated large bubbles of the clusters act mechanically on the cells of the vasculature, potentially generating biochemical signals, leading to an increased uptake of the therapeutic agent.

The cluster composition, and the first and second components of this, are engineered to cluster and phase shift in a controlled manner. The size of the activated bubbles (*in vivo*) can be engineered by varying different formulation parameters of the 1^{st} and 2^{nd} components and the size characteristics of the clusters as prepared (see Example 1).

When exposed to ultrasound, e.g. standard medical imaging frequency and intensity, at the targeted tissue, the microbubble of the administered cluster composition transfers acoustic energy to the attached oil microdroplets and may act as an evaporation seed, or merge with the microbubble so that the oil undergoes a liquid-to-gas phase shift (vaporisation). The resulting bubble undergoes an initial rapid expansion due to vaporisation of the oil, followed by a slower expansion due to inward diffusion of blood gases, and temporarily blocks the microcirculation (metarteriole and capillary network) for approximately 1 minute or more, preferably 2-3 minutes or more, most preferably 3-6 minutes or more. In the method of the invention, or for the composition for use, a therapeutic agent is further administered to the subject, such as being co-administered or pre-administered or post-administered with the cluster composition. The clusters are activated to produce large bubbles by application of external ultrasound energy, and these are trapped in the microvasculature of the targeted tissue (e.g. tumour or site of inflammation). Further application of low frequency ultrasound after trapping facilitates extravasation of the therapeutic agent or activated immune cells to the targeted tissue. Hence, the major limitation of existing technology in limited extravasation and uptake of immunotherapeutic agents and infiltration of activated immune cells can be overcome by the technology of the current invention, as it has been found that the accessibility to the targeted tissue for the therapeutic agent or activated cells is considerably increased. The large, activated bubbles are temporarily embedded in the microvasculature of the insonated tissue and facilitate drug or cell uptake to the target tissue by further application of low power, low frequency ultrasound. The activated phase shift bubbles are approximately 10 times larger in diameter than typical microbubbles, resulting in:
- transient deposition/trapping of activated bubbles in the microvasculature of the targeted (i.e. insonated) tissue;
- close contact between the activated bubbles and the endothelium;
- compared to regular contrast microbubbles; orders of magnitude larger biomechanical effects during post activation US treatment, avoiding inertial cavitation mechanisms.

The above noted attributes lead to a marked enhancement of drug and immune cell extravasation, distribution and uptake.

### The first component of the cluster composition:

The first component comprises a gas microbubble and a first stabilizer to stabilize the microbubble. The first component is hence an injectable aqueous medium comprising dispersed gas and material to stabilize the gas. The microbubbles may be similar to conventional ultrasound contrast agents that are on the market and approved for use for several clinical applications such as Sonazoid^{®}, Optison^{®}, Definity^{®} or Sonovue^{®}, or similar agents used for pre-clinical application such as Micromarker^{®} and Polyson L^{®}. Any biocompatible gas may be present in the gas dispersion, the term "gas" as used herein including any substances (including mixtures) at least partially, e.g. substantially or completely in gaseous (including vapour) form at the normal human body temperature of 37 °C. The gas may thus, for example, comprise air; nitrogen; oxygen; carbon dioxide; hydrogen; an inert gas such as helium, argon, xenon or krypton; a sulphur fluoride such as sulphur hexafluoride, disulphur decafluoride or trifluoromethylsulphur pentafluoride; selenium hexafluoride; an optionally halogenated silane such as methylsilane or dimethylsilane; a low molecular weight hydrocarbon (e.g. containing up to 7 carbon atoms), for example an alkane such as methane, ethane, a propane, a butane or a pentane, a cycloalkane such as cyclopropane, cyclobutane or cyclopentane, an alkene such as ethylene, propene, propadiene or a butene, or an alkyne such as acetylene or propyne; an ether such as dimethyl ether; a ketone; an ester; a halogenated low molecular weight hydrocarbon (e.g. containing up to 7 carbon atoms); or a mixture of any of the foregoing. Preferably, the gas is a halogenated gas, and more preferably a perfluorinated gas. Advantageously at least some of the halogen atoms in halogenated gases are fluorine atoms; thus, biocompatible halogenated hydrocarbon gases may, for example, be selected from bromochlorodifluoromethane, chlorodifluoromethane, dichlorodifluoromethane, bromotrifluoromethane, chlorotrifluoromethane, chloropentafluoroethane, dichlorotetrafluoroethane, chlorotrifluoroethylene, fluoroethylene, ethylfluoride, 1,1-difluoroethane and perfluorocarbons. Representative perfluorocarbons include perfluoroalkanes such as perfluoromethane, perfluoroethane, perfluoropropanes, perfluorobutanes (e.g. perfluoro-n-butane, optionally in admixture with other isomers such as perfluoro-iso-butane), perfluoropentanes, perfluorohexanes or perfluoroheptanes; perfluoroalkenes; perfluoroalkynes; and perfluorocycloalkanes.

The use of perfluorinated gases, for example sulphur hexafluoride and perfluorocarbons such as perfluoropropane, perfluorobutanes, perfluoropentanes and perfluorohexanes, are particularly advantageous in view of the recognised high stability in the bloodstream of microbubbles containing such gases. In one embodiment, the gas of the first component is selected from the group of sulphur fluorides and halogenated low molecular weight hydrocarbons (e.g. containing up to 7 carbon atoms). Other gases with physicochemical characteristics which cause them to form highly stable microbubbles in the bloodstream may likewise be useful. Most preferably, the dispersed gas comprises sulphur hexafluoride, perfluoropropane, perfluorobutane, perfluoropentane, perflurohexane (i.e. a C3-6 perfluorocarbon), nitrogen, air or a mix thereof. Even more preferably, the dispersed gas comprises sulphur hexafluoride, perfluoropropane, or perfluorobutane, or mixture there. And even more preferably, the dispersed gas is perfluorobutane.

The dispersed gas may be in any convenient form, for example using any appropriate gas-containing ultrasound contrast agent formulation as the gas-containing component such as Sonazoid^{®}, Optison^{®}, Sonovue^{®} or Definity^{®} or pre-clinical agents such as Micromarker^{®} or PolySon L^{®}. The first component will also contain material in order to stabilize the microbubble dispersion, in this text termed 'first stabilizer'. Representative examples of such formulations include microbubbles of gas stabilized (e.g. at least partially encapsulated) by a first stabilizer such as a coalescence-resistant surface membrane (for example gelatin), a filmogenic protein (for example an albumin such as human serum albumin), a polymer material (for example a synthetic biodegradable polymer, an elastic interfacial synthetic polymer membrane, a microparticulate biodegradable polyaldehyde, a microparticulate N-dicarboxylic acid derivative of a polyamino acid - polycyclic imide), a non-polymeric and non-polymerisable wall-forming material, or a surfactant (for example a polyoxyethylene-polyoxypropylene block copolymer surfactant such as a Pluronic, a polymer surfactant, or a film-forming surfactant such as a phospholipid). Preferably, the dispersed gas is in the form of phospholipid-, protein- or polymer-stabilized gas microbubbles. Hence, in one embodiment, the first stabilizer is selected from the group of phospholipids, proteins and polymers. A particularly useful first stabilizer is selected from the group of surfactants which include phospholipids comprising molecules with net overall negative charge, such as naturally occurring (e.g. soya bean or egg yolk derived), semisynthetic (e.g. partially or fully hydrogenated) and synthetic phosphatidyl-serines, phosphatidylglycerols, phosphatidylinositols, phosphatidic acids and/or cardiolipins. Alternatively, the phospholipids applied for stabilization may carry an overall neutral charge and be added a negative surfactant such as a fatty acid, e.g. phosphatidylcholine added palmitic acid, or be a mix of differently charged phospholipids, e.g. phosphatidylethanolamines and/or phosphatidylcholine and/or phosphatidic acid. For the first stabilizer, i.e. stabilizing the microbubble of the first component, different examples are demonstrated in WO2015/047103, Example 5, and Tables 9 and 10, wherein various microbubble formulations with different excipients have been tested. The results demonstrate that the ACT concept used in the current invention is applicable to a wide variety of microbubble formulations, also with regards to the composition of the stabilizing membrane.

The microbubble size of the dispersed gas component should preferably be less than 7 µm, more preferably less than 5 µm and most preferably less than 3 µm in order to facilitate unimpeded passage through the pulmonary system, even when in a microbubble-microdroplet cluster.

### The second component of the cluster composition:

The second component comprises a microdroplet comprising an oil phase and a second stabilizer to stabilize said microdroplet, where the oil comprises a diffusible component. This diffusible component is capable of diffusing into the gas microbubble of the first component so as to at least transiently increase the size thereof. For the second component the "diffusible component" is suitably a gas/vapour, volatile liquid, volatile solid or precursor thereof capable of gas generation, e.g. upon administration, the principal requirement being that the component should either have or be capable of generating a sufficient gas or vapour pressure in vivo (e.g. at least 50 torr and preferably greater than 100 torr) so as to be capable of promoting inward diffusion of gas or vapour molecules into the dispersed gas. The 'diffusible component' is preferably formulated as an emulsion (i.e. a stabilized suspension) of microdroplets in an appropriate aqueous medium, since in such systems the vapour pressure in the aqueous phase of the diffusible component will be substantially equal to that of pure component material, even in very dilute emulsions.

The diffusible component in such microdroplets is advantageously a liquid at processing and storage temperature, which may for example be as low as -10 °C if the aqueous phase contains appropriate antifreeze material, while being a gas or exhibiting a substantial vapour pressure at body temperature. Appropriate compounds may, for example, be selected from the various lists of emulsifiable low boiling liquids given in the patent applications WO-A-9416379 or WO2015/047103, the contents of which are incorporated herein by reference. Specific examples of emulsifiable diffusible components include aliphatic ethers such as diethyl ether; polycyclic oils or alcohols such as menthol, camphor or eucalyptol; heterocyclic compounds such as furan or dioxane; aliphatic hydrocarbons, which may be saturated or unsaturated and straight chained or branched; cycloaliphatic hydrocarbons such as cyclobutane, cyclobutene, methylcyclopropane or cyclopentane; and halogenated low molecular weight hydrocarbons, e.g. containing up to 7 carbon atoms. Representative halogenated hydrocarbons include dichloromethane, methyl bromide, 1,2-dichloroethylene, 1,1-dichloroethane, 1-bromoethylene, 1-chloroethylene, ethyl bromide, ethyl chloride, 1-chloropropene, 3-chloropropene, 1-chloropropane, 2-chloropropane and t-butyl chloride. Advantageously at least some of the halogen atoms are fluorine atoms, for example as in dichlorofluoromethane, trichlorofluoromethane, 1,2-dichloro-1,2-difluoroethane, 1,2-dichloro-1,1,2,2-tetrafluoroethane, 1,1,2-trichloro-1,2,2-trifluoroethane, 2-bromo-2-chloro-1,1,1-trifluoroethane, 2-chloro-1,1,2-trifluoroethyl difluoromethyl ether, 1-chloro-2,2,2-trifluoroethyl difluoromethyl ether, partially fluorinated alkanes (e.g. pentafluoropropanes such as 1H, 1H,3H-pentafluoropropane, hexafluorobutanes, nonafluorobutanes such as 2H-nonafluoro-t-butane, and decafluoropentanes such as 2H,3H-decafluoropentane), partially fluorinated alkenes (e.g. heptafluoropentenes such as 1H, 1H, 2H-heptafluoropent-1-ene, and nonafluorohexenes such as 1H, 1H,2H-nonafluorohex-1-ene), fluorinated ethers (e.g. 2,2,3,3,3-pentafluoropropyl methyl ether or 2,2,3,3,3-pentafluoropropyl difluoromethyl ether) and, more preferably, perfluorocarbons. Examples of perfluorocarbons include perfluoroalkanes such as perfluorobutanes, perfluoropentanes, perfluorohexanes (e.g. perfluoro-2-methylpentane), perfluoroheptanes, perfluorooctanes, perfluorononanes and perfluorodecanes; perfluorocycloalkanes such as perfluorocyclobutane, perfluorodimethyl-cyclobutanes, perfluorocyclopentane and perfluoromethyl-cyclopentane; perfluoroalkenes such as perfluorobutenes (e.g. perfluorobut-2-ene or perfluorobuta-1,3-diene), perfluoropentenes (e.g. perfluoropent-1-ene) and perfluorohexenes (e.g. perfluoro-2-methylpent-2-ene or perfluoro-4-methylpent-2-ene); perfluorocycloalkenes such as perfluorocyclopentene or perfluoro-cyclopentadiene; and perfluorinated alcohols such as perfluoro-t-butanol. Hence, the oil (the diffusible component) of the second component may be selected from the group of aliphatic ethers, heterocyclic compounds, aliphatic hydrocarbons, halogenated low molecular weight hydrocarbons and perfluorocarbons. In one embodiment, the oil phase of the second component comprises a perfluorocarbon.

Particularly useful in the current invention are diffusible components with an aqueous solubility below 1·10⁻⁴ M, more preferably below 1·10⁻⁵ M. It should be noted, however, that if a mixture of diffusible components and/or co-solvents are used, a substantial fraction of the mixture may contain compounds with a higher water solubility. Based on the water solubility, examples of suitable oils (diffusible components) are: perfluorodimethylcyclobutane, perfluoromethylcylopentane, 2-(trifluoromethyl)perfluoropentane and perfluorhexane.

It will be appreciated that mixtures of two or more diffusible components may if desired be employed in accordance with the invention; references herein to "the diffusible component" are to be interpreted as including such mixtures.

The second component will also contain material in order to stabilize the microdroplet dispersion, in this text termed 'second stabilizer'. The second stabilizer may be the same as or different from any materials(s) used to stabilize the gas dispersion, e.g. a surfactant, such as a phospholipid, a polymer or a protein. The nature of any such material may significantly affect factors such as the rate of growth of the dispersed gas phase. In general, a wide range of surfactants may be useful as stabilizers, and representative examples of useful surfactants include fatty acids (e.g. straight chain saturated or unsaturated fatty acids, for example containing 10-20 carbon atoms) and carbohydrate and triglyceride esters thereof, phospholipids (e.g. lecithin), fluorine-containing phospholipids, proteins (e.g. albumins such as human serum albumin), polyethylene glycols, and polymer such as a block copolymer surfactants (e.g. polyoxyethylene-polyoxypropylene block copolymers such as Pluronics, extended polymers such as acyloxyacyl polyethylene glycols, for example polyethyleneglycol methyl ether 16-hexadecanoyloxy-hexadecanoate, e.g. wherein the polyethylene glycol moiety has a molecular weight of 2300, 5000 or 10000), and fluorine-containing surfactants (e.g. as marketed under the trade names Zonyl and Fluorad). Particularly useful surfactants include phospholipids, and particularly phospholipids comprising molecules with overall neutral charge, e.g. distearoyl-sn-glycerol-phosphocholine (DSPC). For the second component, a range of different stabilizers may be used to stabilize the microdroplet. Further, a wide range of ionic, preferably cationic, substances may be used in order to facilitate a suitable surface charge.

**It** will be appreciated that, to facilitate attractive electrostatic interactions to achieve clustering between the microbubbles in the first component and the emulsion microdroplets in the second component, these should be of opposite surface charge. Hence, if the microbubbles of the first component are negatively charged, the microdroplets of the second component should be positively charged, or vice versa. In a preferred embodiment, the surface charge of the microbubbles of the first component is negative, and the surface charge of the microdroplets of the second component is positive. In order to facilitate a suitable surface charge for the oil microdroplets a cationic surfactant may be added to the stabilizing structure. A wide range of cationic substances may be used, for example at least somewhat hydrophobic and/or substantially water-insoluble compounds having a basic nitrogen atom, e.g. primary, secondary or tertiary amines and alkaloids. A particularly useful cationic surfactant is stearylamine. In one embodiment, the second stabilizer is a neutral phospholipid added a cationic surfactant, for example such as a DSPC-membrane with stearylamine.

In one embodiment, the first stabilizer and the second stabilizer each independently comprises a phospholipid, a protein, a polymer, a polyethyleneglycol, a fatty acid, a positively charged surfactant, a negatively charged surfactant or mixtures thereof. More particularly, the first stabilizer comprises a phospholipid, a protein, or a polymer optionally added a negatively charged surfactant, and the second stabilizer comprises a phospholipid, protein, or a polymer optionally added a positively charged surfactant.

In one embodiment, the first component comprises a dispersed gas selected from the group of sulphur hexafluoride, perfluoropropane, perfluorobutane, perfluoropentane, perflurohexane, nitrogen and air or a mix thereof, stabilized by a first stabilizer selected from the group of phospholipids, proteins and polymers; the second component comprises a diffusible component selected from the group of perfluorocarbons, e.g. a perfluorocycloalkane, stabilized with a second stabilizer selected from the group of surfactants, e.g. including phospholipids, polymers and proteins. More specifically, either of the stabilizers are selected from phospholipids.

The first and second components of the two-component formulation system are combined shortly before the intended use, for preparation of a composition of microbubble-microdroplet clusters, and for use in an appropriate time window according to the methods of the invention. It will also be appreciated that the mixing of the first and second components can be achieved in various manners depended on the form of the components; e.g. mixing two fluid components, reconstitution of one component in dry powder form with one component in fluid form, mixing two components in dry form prior to reconstitution with fluid (e.g. water for injection or buffer solution). Hence, in one embodiment of the invention, the method comprises a step of preparing the microbubble-microdroplet cluster composition prior to the administration step (step ii). In a preferred embodiment, the microbubble-microdroplet cluster composition is prepared by reconstitution of the first component (microbubbles) in dry powder form with the second component (microdroplets) in fluid form. More particularly a first vial comprising the first component is reconstituted with the second component withdrawn from a second vial, using a sterile, single use syringe and needle. The content of the syringe is to be added through a stopper of the first vial and the resulting cluster composition is homogenised, e.g. by manual mixing.

Also, it will be appreciated that other components may influence the ability of the microbubbles and microdroplets to form clusters upon mixing including, but not limited to; the level of surface charge of the microbubbles/microdroplets, the concentration of the microbubbles/microdroplets in the two components, the size of the microbubbles/microdroplets, the composition and concentration of ions in the liquid matrix, the pH, the composition and concentration of excipients (e.g. buffer or tonicity components) etc. (see WO2015/047103, Example 1). Such characteristics of the components and the composition may also influence the size and stability (both *in-vitro* and *in-vivo*) of the clusters generated and may be important factors influencing biological attributes (e.g. efficacy and safety profile). It is also appreciated that not all of the microbubbles/microdroplets in the cluster composition may be present in clustered form, but that a substantial fraction of the microbubbles and/or microdroplets may be present together in a free (non-clustered) form together with a population of microbubble-microdroplet clusters. In addition, the way the two components are mixed may influence these aspects, including, but not limited to; shear stress applied during homogenization (e.g. soft manual homogenization or strong mechanical homogenization) and time range for homogenization. The cluster composition is to be administered to the subject during a time window wherein the characteristics of the clusters are substantially unchanged, such as within 5 hours, such as within 3 hours, from combining the two components. In-use stability studies of the applicant show that the clusters display stable characteristics for at least 3 hours, please see Example 1.

The microdroplet size of the dispersed diffusible component in emulsions intended for intravenous injection should preferably be less than 7 µm, more preferably less than 5 µm, most preferably less than 4 µm, and greater than 0.5 µm, more preferably greater than 1 µm, most preferably greater than 2 µm in order to facilitate unimpeded passage through the pulmonary system, but still retain a volume that is sufficient for activated bubble retention in the microvasculature.

Growth of the dispersed gas phase *in vivo* may, for example, be accompanied by expansion of any encapsulating material (where this has sufficient flexibility) and/or by abstraction of excess surfactant from the administered material to the growing gas-liquid interfaces. It is also possible, however, that stretching of the encapsulating material and/or interaction of the material with ultrasound may substantially increase its porosity. Whereas such disruption of encapsulating material has hitherto in many cases been found to lead to rapid loss of echogenicity through outward diffusion and dissolution of the gas thereby exposed, we have found that when using compositions in accordance with the present invention, the exposed gas exhibits substantial stability. Whilst not wishing to be bound by theoretical calculations, we believe that the exposed gas, e.g. in the form of liberated microbubbles, may be stabilized, e.g. against collapse of the microbubbles, by a supersaturated environment generated by the diffusible component, which provides an inward pressure gradient to counteract the outward diffusive tendency of the microbubble gas. The exposed gas surface, by virtue of the substantial absence of encapsulating material, may cause the activated bubbles to exhibit exceptionally favourable acoustic properties as evidenced by high backscatter and low energy absorption (e.g. as expressed by high backscatter: attenuation ratios) at typical diagnostic imaging frequencies; this echogenic effect may continue for a significant period, even during continuing ultrasound insonation.

It is envisioned that the ACT concept for delivery of ITAs and/or activated immune cells to targeted tissues, i.e. the composition for use and methods of the invention, is a concept that applies for a broad combination of components (first and second) components, and also for a wide range of immunotherapeutic agents, optionally combined with a chemotherapeutic agent. Hence, any of the ingredients listed for the first component, including gases and first stabilizers, can be combined with the ingredients listed for the second component, including the diffusible component and the second stabilizers.

To summarize, in one embodiment, the two-component formulation system for preparation of the microbubble-microdroplet cluster composition, for use according to the methods of the invention, comprises:
(i) a first component which comprises a gas microbubble and a first stabilizer to stabilize said microbubble, wherein the gas of the gas microbubble is selected from the group of halogenated gases, preferably is a perfluorinated gas, and most preferably is perfluorobutane; and the first stabilizer is selected from the group of phospholipids, proteins and polymers, optionally added a negatively charged surfactant, and more preferably is a phospholipid, and most preferably is hydrogenated egg phosphatidyl serine-sodium (HEPS-Na); and
(ii) a second component which comprises a microdroplet comprising an oil phase and a second stabilizer to stabilize said microdroplet, where the oil comprises a diffusible component capable of diffusing into said gas microbubble so as to at least transiently increase the size thereof, wherein the oil is selected from the group of aliphatic ethers, heterocyclic compounds, aliphatic hydrocarbons, halogenated low molecular weight hydrocarbons and perfluorocarbons, is preferably a perfluorocarbon, and is most preferably perfluoromethyl-cyclopentane (pFMCP); and the second stabilizer is selected from the group of phospholipids, polymers and proteins, optionally added a positively charged surfactant, is more preferably a phospholipid added a positively charged surfactant, and is most preferably 1,2-distearoyl-sn-glycerol-3-phosphocholine (DSPC) added stearylamine (SA);
where the microbubbles and microdroplets of the first and second components have opposite surface charges and form said clusters via attractive electrostatic interactions.

### Therapeutic agents:

The therapeutic agent or agents, also called "the drug" or "the drugs", for use according to the invention is selected from the group of immunotherapeutic agents (ITAs), optionally in combination with a drug from the group of chemotherapeutic agents. This or these are administered as a separate composition to the cluster composition. The therapeutic agent(s) is administered according to standard of care, i.e. according to the respective Summary of Product Characteristics (SmPC).

Classes of ITAs include, but are not limited to, monoclonal antibodies (mAbs), fusion proteins, soluble cytokine receptors, recombinant cytokines, small-molecule mimetics, cell therapies, cancer vaccines and oncolytic viruses. The term "Immune-oncology agents (IO)" tends to be used as a generic term to describe the group of ITAs collectively, hence the classes of ITAs useful in the invention also include (IOs) as further described below.

Within oncology, ITAs are used to potentiate the immune system to attack cancer cells, whereas within treatment of autoimmune diseases their function is to dampen autoimmune responses attacking healthy cells in the body.

Within cancer therapy, immunotherapy antibodies bind to tumour antigens (molecular target), marking and identifying the cancer cells for the immune system to inhibit or kill. Various classes of cancer immunotherapeutic agents target various tumour antigens. In the following, the molecular target is stated in parenthesis behind the drug names.

Immune-oncology is a rapidly developing field and agents targeting previously unexplored antigens are continuously entering pre-clinical and clinical development. An embodiment of the current invention is the use of novel IO agents targeted to all and any antigen identified and named according to the Human Cell Differentiation Molecules (HCDM) Council nomenclature (CD1 through CD371), and as identified and agreed during future Human Leucocyte Differentiation Antigens (HLDA) Workshops.

### Monoclonal Antibodies (mAbs):

Immune Checkpoint Inhibitors (ICIs) are monoclonal antibody drugs that block proteins called checkpoints that are made by some types of immune system cells, such as T cells, and some cancer cells. These checkpoints help keep immune responses from being too strong and sometimes can keep T cells from killing cancer cells. When these checkpoints are blocked, T cells can kill cancer cells better. Examples of checkpoint proteins found on T cells or cancer cells include PD-1 (antibody) / PD-L1/PD-L2 (antigens) and CTLA-4 (antibody) / CD80/CD86 (antigens). ICIs are used to treat a range of cancerous diseases including; melanoma, lung cancer, renal cancer, lung cancer, lymphoma and urothelial cancer. ICIs such as the anti-PD-1/PD-L1 agents, prevent the interaction between PD-L1 on tumour cells and PD-1 on T-cells, allowing the immune system to launch an antitumour response. Within the current invention delivery of ICIs, and particularly of anti-PD1 or anti-PD-L1/L2 agents, represents a preferred embodiment. Preferred agents within ICIs include but are not limited to; ipilimumab (CTLA-4), nivolumab (PD-1), pembrolizumab (PD-1), atezolizumab (PD-L1), avelumab (PD-L1), durvalumab (PD-L1) and cemiplimab (PD-1). In one embodiment, the at least one ITA is an immune checkpoint inhibitor selected from the group of nivolumab (PD-1), pembrolizumab (PD-1), atezolizumab (PD-L1), avelumab (PD-L1), durvalumab (PD-L1) and cemiplimab (PD-1).
mAbs directed against other targets:
   A range of other IO-agents, directed against various internal and external targets (antigens), representing preferred embodiments of the current invention, include but are not limited to; Alemtuzumab (CD52), Rituximab (CD20), Tositumomab (CD20), Obinotuzumab (CD20), Ofatumumab (CD20), Ibritumomab (CD20), Dinutuximab (GD2), Blinatumumab (CD19/CD3), Daratumumab (CD38), Isatuximab (CD38), Elotuzumab (SLAMF7), Cetuximab (EGFR), Panitumumab (EGFR), Necitumumab (EGFR), Catumaxomab (Ep CAM), Trastuzumab (HER2), Pertuzumab (HER2), Olaratumab (PDGF-Rα), Bevasizumab (VEGF), Ramucirumab (VEGF R2), Imiquimod (TLR7), Tocilizumab (IL-R6),
Drug conjugated *mAbs*:
   Another class of IO-agents are represented by drug-antibody conjugates. These represent preferred embodiments of the current invention and include but are not limited to; Moxetumumab pasudotox-tdfk (CD22), Brentuximab vedotin (CD30), Trastuzumab emtancin (HER2), Inotuzumab ozogamicin (CD22), Gemtuzumab ozogamicin (CD33), Tagraxofusp-erzs (CD123), Polatuzumab vedotin-piiq (CD79B), Erfortumab vedotin-ejfv (Nectin 4), Traztuzumab deruxtcan (HER2), Sasituzumab govitecan-hziy (Trop 2).
Cytokine therapy:
   Cytokines are proteins produced by many types of cells present within a tumour which often employ them to allow it to grow and reduce the immune response. These immune-modulating effects allow them to be used as drugs to provoke an immune response.

Interleukin-2 and interferon-α are cytokines, proteins that regulate and coordinate the behaviour of the immune system and represent embodiments of the current invention. They have the ability to enhance anti-tumour activity and thus can be used as passive cancer treatments. Interferon-α is used in the treatment of hairy-cell leukaemia, AIDS-related Kaposi's sarcoma, follicular lymphoma, chronic myeloid leukaemia and malignant melanoma. Interleukin-2 is an approved treatment for malignant melanoma and renal cell carcinoma.

### Cell therapies:

The premise of CAR-T and other cell immunotherapy is to modify immune cells to recognize cancer cells in order to more effectively target and destroy them. E.g. T-cells are harvested from the patient, genetically altered to add a chimeric antigen receptor (CAR) that specifically recognizes cancer cells, then infused back into the patient to attack their tumours. Agents within this category, which represent preferred embodiments of the current invention include but are not limited to; Sipuleucel-T (Provenge), Tisangenlecleucel (Kymriah) and Axicabtagene ciloleucel (Yescarta).

### Oncolytic virus:

An oncolytic virus is a virus that preferentially infects and replicates in cancer cells. As the infected cancer cells are destroyed by oncolysis, they release new infectious virus particles or virions to help destroy the remaining tumour. Oncolytic viruses are thought not only to cause direct destruction of the tumour cells, but also to stimulate host anti-tumour immune responses for long-term immunotherapy. Oncolytic viruses may also be used as vectors for transgene delivery to cancer cells, enabling localised expression of transgene products. Such encoded products include antibody fragments, bispecific antibodies, T-cell engaging ligands, secreted immunomodulators or other IOs.

A number of viruses including adenovirus, reovirus, measles, herpes simplex, Newcastle disease virus and vaccinia have been clinically tested as oncolytic agents and several are in clinical development. T-Vec (Talimogene laherparepvec) is currently the only FDA-approved oncolytic virus (for treatment of melanoma). However, a number of other oncolytic viruses are in Phase II-III development including but not limited to; Ad2/5 dl1520 (Onyx-015), GLV-1h68 (GL-ONC1) and CV706. The use of oncolytic viruses represents a preferred embodiment of the current invention.

### Cancer Vaccines:

A therapeutic cancer vaccine is a vaccine that treats existing cancer. Antigens, found on the surface of cells, are substances the body thinks are harmful. The immune system attacks the antigens and, in most cases, gets rid of them. This leaves the immune system with a "memory" that helps it fight those antigens in the future. Cancer treatment vaccines boost the immune system's ability to find and destroy antigens. Often, cancer cells have certain molecules called cancer-specific antigens on their surface that healthy cells do not have. When a vaccine gives these molecules to a person, the molecules act as antigens. They tell the immune system to find and destroy cancer cells that have these molecules on their surface. Two therapeutic cancer vaccines are currently used in clinical practice; Bacillus Calmette-Guérin (BCG) for treatment of early-stage bladder cancer and Sipuleucel-T for treatment of prostate cancer. Furthermore Oncophage^{®} is approved in Russia for treatment of bladder cancer. A range of other vaccines are currently in clinical development. The use of cancer vaccines represents a preferred embodiment of the current invention.

### Emerging IOs:

Anti-CD47 therapy: Many tumour cells overexpress CD47 to escape immune-surveillance of host immune system. CD47 binds to its receptor signal regulatory protein alpha (SIRPα) and downregulate phagocytosis of tumour cell. Therefore, anti-CD47 therapy aims to restore clearance of tumour cells. Additionally, growing evidence supports the employment of tumour antigen-specific T cell response in response to anti-CD47 therapy. A number of therapeutics is being developed, including anti-CD47 antibodies, engineered decoy receptors, anti-SIRPα antibodies and bispecific agents. Use of anti-CD47 therapeutics represents a preferred embodiment of the current invention.

*Anti-GD2 antibodies:* Carbohydrate antigens on the surface of cells can be used as targets for immunotherapy. GD2 is a ganglioside found on the surface of many types of cancer cell including neuroblastoma, retinoblastoma, melanoma, small cell lung cancer, brain tumours, osteosarcoma, rhabdomyosarcoma, Ewing's sarcoma, liposarcoma, fibrosarcoma, leiomyosarcoma and other soft tissue sarcomas. It is not usually expressed on the surface of normal tissues, making it a good target for immunotherapy. Use of anti-GD2 agents represents a preferred embodiment of the current invention.

*Anti-TIM3:* Recent studies have highlighted that TIM3 has an important role to play in T-cell exhaustion and correlates with the outcome of anti-PD-1 therapy. Targeting TIM3 might be a promising approach for cancer immunotherapy. Use of anti-TIM3 agents represents a preferred embodiment of the current invention.

*Anti-LAG3:* LAG3 is an ICI with multiple biological effects on the function of T-cells. LAG3 is highly expressed in various types of tumour infiltrating lymphocytes and participates in the immune escape mechanism of tumours. For this reason, LAG3 is currently being clinically explored as an indicator of tumour prognosis and as target tumour therapy. Use of anti-LAG3 agents represents a preferred embodiment of the current invention.

Quite often, IO regimes comprise combination therapies in which one or several chemotherapeutic agents are given in conjunction with the IO-agent. A preferred embodiment of the current invention is to apply such combination regimens. A list of preferred chemotherapeutic agents are listed below.

### Alkylating agents:

| | |
|---|---|
| *Nitrogen Mustards:* | Mechlorethamine Hydrochloride |
| *Nitrosoureas:* | Carmustine, Streptozocin, Lomustine |
| *Tetrazines:* | Dacarbazine, Temozolomide |
| *Aziridines:* | Thiotepa, Mitomycin, Aziridinylbenzoquinon |
| *Cisplatins:* | Cisplatin, Carboplatin, Oxaliplatin |

### Antimetabolites

| | |
|---|---|
| *Anti-folates:* | Methotrexate, Pemetrexed |
| *Fluoropyrimidines:* | Fluorouracil, Capecitabine |
| *Deoxynucleotide analogues*: | Cytarabine, Decitabine, Azacitidine, Gemcitabine, Fludarabine, Nelarabine, Pentostatin |
| *Thiopurines:* | Thioguanine, Mercaptopurine |

### Anti-microtubule:

| | |
|---|---|
| *Vinca alkaloids:* | Vinorelbine, Vinicristine, Vindesine, Vinflunine |
| *Taxanes:* | Paclitaxel or nab-Paclitaxel, Cabazitaxel, Docetaxel |
| *Podophyllotoxin:* | Etoposide, Teniposide |

### Topoisomerase inhibitors

| | |
|---|---|
| *Topoisomerase I*: | Irinotecan or liposomal Irinotecan, Topotecan |
| *Topoisomerase II*: | Doxorubicin or liposomal doxorubicin, Mitoxantrone, Teniposide, Nobiocin, Merbarone, Aclarubicin |

### Cytotoxic antibiotics

| | |
|---|---|
| *Anthracyclines*: | Doxorubicine, Daunorubicin, Epirubicin, Idarubicin, Bleomycin, Mitomycin |

In addition to the use of the ITAs according to the invention in oncology, the other main area of use is for treatment of autoimmune diseases, including but not limited to; psoriasis, lupus, rheumatoid arthritis, Crohn's disease, multiple sclerosis and alopecia areata. This class of agents is also frequently used to avoid rejection after organ transplants. The pathological condition treated according to the method of the invention is preferably cancer or autoimmune disease.

Immunotherapy for treatment of autoimmune diseases is a rapidly developing field and agents targeting previously unexplored antigens are continuously entering pre-clinical and clinical development. An embodiment of the current invention is the use of novel ITAs targeted to all and any antigen identified and named according to the Human Cell Differentiation Molecules (HCDM) Council nomenclature (CD1 through CD371), and as identified and agreed during future Human Leucocyte Differentiation Antigens (HLDA) Workshops. Hence, in one embodiment the at least one ITA for use in the method of the invention is selected from ITAs having ability to target any of the antigens named CD1 through to CD371.

Preferred ITAs for treatment of autoimmune diseases under the current invention include, but are not limited to;
Small molecule inhibitors such as prednisone, budesonide, prednisolone, tofacitinib, cyclosporine, tacrolimus, sirolimus, everolimus, azathioprine, leflunomide and mycophenolate.

Monoclonal antibodies and other biologics such as abatacept (CD80 and CD86), adalimumab (TNFα), anakinra (IL-1), certolizumab (TNFα), etanercept (TNFα), golimumab (TNFα), infliximab (TNF), ixekizumab (IL17A), natalizumab (alpha-4 integrin), rituximab (CD20), secukinumab (IL17A), tocilizumab (IL-6), ustekinumab (IL-12 and IL-23), vedolizumab (Integrin α4β7), basiliximab (CD25) and daclizumab (CD 25). These monoclonal antibodies and biologics represent a preferred embodiment of the current invention.

Hence, in one embodiment, the immunotherapeutic agent(s) is selected from the group of IOs, monoclonal antibodies (mAbs), fusion proteins, soluble cytokine receptors, recombinant cytokines, small-molecule mimetics, cell therapies, cancer vaccines and oncolytic viruses.

In another embodiment, one or more ITA is for use in combination therapies in which one or several chemotherapeutic agents are given in conjunction with the one or more ITAs. Hence, the treatment with one or more immunotherapeutic agents is combined with treatment with one or more chemotherapeutic agents., such as for treatment of cancer, wherein the chemotherapeutic agent is selected from the non-limited group of Alkylating agents, Antimetabolites, Anti-microtubules, Topoisomerase inhibitors, Anthracyclines and Cytotoxic antibiotics.

In another embodiment, the immunotherapeutic agent has a molecular weight of more than 15.000 Daltons, preferably more than 30.000 Daltons, more preferably more than 50.000 Daltons and most preferably more than 100.000 Daltons. In some embodiments, the immunotherapeutic agent has a molecular weight of up to 1500 Daltons.

The composition for use, and the method of treatment, of the invention may be useful for autoimmune diseases or cancers, or of sites of inflammation (e.g. joint). In one embodiment, the composition and the method of use/treatment is for treatment of cancers, such as of localized pathological lesions, e.g. solid cancers, or metastatic cancers, such as for example of any of melanomas, sarcomas, prostate cancers, colon cancers, anal cancers, oesophageal cancers, gastric cancers, rectal cancers, small intestine cancers, hepatic cancers, lung cancers, renal cancers, breast cancers, brain cancers, bile duct cancers, head and neck cancers, lymphomas, urothelial cancers, adrenocortical carcinoma, Merkel cell carcinoma, parathyroid cancer, paraganglioma, pheochromocytoma, pituitary tumors, thyroid cancer, bladder cancer, penile cancer, testicular cancer, cervical cancer, endometrial cancer, fallopian tube cancer, gestational trophoblastic tumors, ovarian cancer, peritoneal cancer, vaginal cancer and vulvar cancer.

In one embodiment, the composition and the method of use/treatment is for treatment of autoimmune diseases, such as of any of psoriasis, lupus, rheumatoid arthritis, Crohn's disease, multiple sclerosis and alopecia areata.

Pancreatic cancer, such as pancreatic ductal adenocarcinoma (PDAC), is disclaimed from the pathological conditions to be treated by the method of the invention.

In another embodiment the composition and the method of use/treatment is for treatment after organ transplants.

In one embodiment, either of the following monoclonal antibodies are disclaimed from the use and method of the invention: Bevacizumab, Cetuximab, Ipilimumab, Ofatumumab, Ocrelizumab, Panitumab, Rituximab.

In one embodiment, the therapeutic agent is formulated in a vehicle, such as included in the form of liposomes, conjugates, nanoparticles or microspheres, used as vehicles for the therapeutic agent. Hence, the therapeutic agent may be part of a larger drug construct such as nano-drugs, e.g. in liposomal or particulate formulations, or as monoclonal antibodies. Hence, in one embodiment, the therapeutic agent is formulated in closed lipid spheres, such as including the therapeutic agent in a liposomal formulation, or formulated in polymeric micelles As demonstrated by Examples 3 and 4, the ACT concept may be particularly useful for combination with larger drug molecules or constructs.

Further, combination regimes between one or several chemotherapeutic agents with one or several immunotherapeutic agents are preferred. Further, newer generation of liposomes containing two anticancer drugs with a single liposome, and immunoliposomes that comprise an antibody conjugated to a liposome, are encompassed by the invention.

In a second preferred embodiment, the therapeutic agent is selected from a group of immunotherapeutic agents such as oncolytic viruses which include, but is not limited to, adenovirus, reovirus, measles, herpes simplex, Newcastle disease virus, and vaccinia. The viruses can cause cancer cells to "burst", killing the cancer cells and releasing cancer antigens. These antigens can then stimulate immune responses that can seek out and eliminate any remaining tumour cells nearby and potentially anywhere else in the body.

In yet another embodiment, several therapeutic agents, including at least one ITA, are administered as a combination regimen. Examples of suitable combination regimens include but are not limited to:
1) the combination regimen comprising pembrolizumab followed by paclitaxel and carboplatin and
2) the combination regimen comprising nivolumab followed by ipilimumab.

Hence, in one embodiment of the method, the ITA is selected from group of the monoclonal antibodies anti-PD1, anti-PDL1 or CTLA4, and is used in combination with a chemotherapeutic agent, e.g. such as a chemotherapeutic pembrolizumab in combination with cisplatin plus oxaliplatin or capecitabine.

Disclaimer: in one embodiment, the chemotherapeutic agent is not paclitaxel, i.e. as the free non-albumin-bound form. In one embodiment, the therapeutic agent is not a combination of gemcitabine with nab-paclitaxel.

Disclaimer: the disease to be treated is not pancreatic cancer. Disclaimer: in one embodiment the immunotherapeutic agent is not targeted to CTLA-4, CD-20, VEGF or EGF.

### Administration routes:

The cluster composition is administered to said mammalian subject parenterally, preferably intravenously. The route of administration might also be selected from the intra-arterial, intramuscular, intraperitoneal, intratumoural or subcutaneous administration. For administration to the subject, the therapeutic agent is pre-, and/or co- and/or post administered separate to the cluster composition as a separate composition. The therapeutic agent is administered according to the respective approved Summary of Product Characteristics. Typically, the route is selected from the group comprising, but not limited to, intravenous, intraperitoneal, intratumoural and intramuscular administration. The two compositions, i.e. the cluster composition (a) and the therapeutic agent composition(s) (b) may hence be administered via the same or via different routes of administration.

### Treatment schedules:

It will be appreciated that the composition for use, the method for treatment, and/or the method for delivery of drugs, of the invention, may e.g. be employed as part of a multi-drug treatment regimen. In one embodiment of the invention, this includes the use of more than one therapeutic agent. Such chemotherapeutic combination regimen may comprise e.g. paclitaxel and cisplatin, in addition to at least one ITA, such as pembrolizumab.

The applicant has surprisingly found that the perfusion pattern in the targeted tissue (e.g. a tumour) may vary on a short time scale. US imaging studies have demonstrated that the deposition pattern of activated bubbles vary considerably from injection to injection within the same subject and targeted region of interest, likely due to a temporal variation in perfusion pattern. This variance points towards a therapeutic benefit if the targeted tissue region is treated multiple times in order to enhance extravasation within the entire tissue volume. Furthermore, in many cases, therapeutic regimens comprise the administration of several therapeutic agents at different timepoints in the regimen. This also points towards the benefit of performing the ACT treatment several times in order to provide enhanced extravasation of all agents.

Hence, in one embodiment, several ACT treatments can be performed during the period of administering the therapeutic agents, e.g. as exemplified in Figure 11 and Figure 12. In one embodiment, the method of treatment includes 1 to 5, such as 2 to 4, ACT treatments. The "ACT treatment" or "ACT procedure" includes at least the administration of a cluster composition, the activation of the clusters by regular medical imaging US insonation and the following, low frequency US insonation to induce enhanced uptake, i.e. as described in the method steps ii), iii) and iv). The Figure 11 and Figure 12 provide examples of treatment schedules wherein combinations of nivolumab and ipilimumab, and pembrolizumab followed by paclitaxel and carboplatin, are used. Other ITAs, potentially combined with chemotherapeutic agents, as disclosed herein, may as well be used, with one or more ACT treatment.

Figure 11 provides a graph of possible ACT treatments for use according to the invention performed during treatment with the combination regimen comprising: 30 minutes infusion of nivolumab followed by a 90 minute infusion of ipilimumab. The ACT procedure is applied three times during administration, as indicated by grey ACT^{®} sonoporation bars.

Panel A of Figure 11: each ACT procedure consists of
- a.: injection of the cluster composition,
- b.: activation of clusters with 60 second of regular medical imaging ultrasound insonation, and
- c.: enhancement step with 5 minutes of 400 to 600 kHz ultrasound insonation at an MI between 0.1 to 0.3.

Panel B of Figure 11: y-axis showing plasma concentration of the administered therapeutic agents in percent of peak and x-axis showing time in minutes. In this example three ACT procedures are performed at approximately 30 minutes, 80 minutes and 120 minutes in order to cover both drugs and provide treatment of the entire region of interest.

Figure 12 provides a graph of possible ACT treatments performed during treatment with the combination regimen comprising the Standard of Care combination immunotherapy plus chemotherapy regimen for treatment of metastatic squamous non-small cell lung cancer; pembrolizumab followed by paclitaxel and carboplatin.

Panel A for Figure 12: ACT procedure as detailed in Figure 11. Panel B of Figure 12: y-axis showing plasma concentration of the administered therapeutic agents in percent of peak and x-axis showing time in minutes. In this example three ACT procedures are performed at approximately 160 minutes, 200 minutes and 240 minutes in order to cover all three drugs and provide treatment of the entire region of interest.

The inventors have found that it is beneficial to repeat the ACT procedure multiple times, even when a single therapeutic agent is administered. Using US imaging during activation of ACT, the inventors have observed a notable effect in deposition of ACT bubbles in tumours. A strong variance in the deposition pattern from injection to injection in the same animal was observed; the density of deposited ACT bubbles differed between various segments of the tumour, and this pattern changed between injections. Although not fully elucidated, it is hypothesised that these effects are due to a temporal variation in perfusion for various tumour segments. Based on these observations, in order to reach as much of the tumour volume as possible, in the examples, the inventors have applied the ACT procedure three consecutive times, back-to-back. This also points to the benefit of applying several ACT procedures during clinical use, as noted above for the regimens visualized in Figures 11 and 12.

Hence, in one embodiment, more than one therapeutic agent, such as 1 to 5 therapeutic agents, are administered simultaneously or sequentially over a certain time span, such as over up to 3 hours, wherein at least one, such as 1 to 5, ACT treatments (ACT procedures) are performed during the same period.

In one embodiment, an ACT procedure is provided comprising the following steps: The administration, such as an intravenous administration, of a cluster composition is followed by local US insonation of the tissue region of interest with regular medical imaging US (activation), followed by low frequency US insonation to induce enhanced extravasation of drugs or activated immune cells. These steps are performed 2-5 consecutive times, such as 3 consecutive times. Hence, the steps (ii) to (iv) are repeated one to four times. These steps are performed in conjunction with administration of one or more therapeutic agents, including at least one ITA. The activation, i.e. the initial US insonation, should start prior to or immediately after each administration of the cluster composition, such as within 20 seconds, and lasts for e.g. 30-120 seconds. The insonation with low frequency ultrasound follows the activation step and should typically last for 3 to 10 minutes, such as for about 5 minutes. There is preferably an immediate start of step (iv) after step (iii). A dual frequency transducer may beneficially be used in the treatment, for both the activation step and the enhancement step. By using such, the switch from the activation insonation in step (iii) to the enhancement insonation in step (iv) can be made without any delay. Application of the enhancement field immediately after activation may be important for the resulting therapeutic benefit. In this respect it would be beneficial to apply both the activation and the enhancement insonation using a broad band or dual frequency US transducer. I.e. a transducer capable of delivering sufficient US pressure (i.e. MI) over all frequencies required by the stated preferred ranges. E.g. a transducer capable of delivering MIs of up to 0.4 at both 1 to 10 MHz and at 0.1 to 1 MHz, more preferably 0.4 to 0.6 MHz.

Furthermore, it might be beneficial to apply the activation and enhancement insonation fields simultaneously or intermittently e.g. using a transducer capable of emitting both fields at the same time or emitting both fields in a short temporal series (e.g. 1 second of activation field followed by one second of enhancement field followed by one second activation field etc. etc.).

In another embodiment, the multidrug regimen comprises an anti-PD1, anti-PDL1 or CTLA4 monoclonal antibody and a chemotherapeutic agent, e.g. pembrolizumab + cisplatin plus oxaliplatin or capecitabine. Hence, several therapeutic drugs can be used, and several ACT procedures can be applied during the treatment regimen. In a preferred embodiment, the ACT procedure is performed when the active therapeutic molecule displays maximum or close to maximum concentration in the blood after administration. Hence, the timing of the ACT treatment(s) may vary dependent upon the pharmacokinetics of the therapeutic agent.

In one embodiment, the pharmaceutical composition of the invention is for use in delivery of therapeutic agent(s) to a targeted tissue, particularly to subjects diagnosed with cancers or autoimmune diseases. The composition for use, and using the ACT technology, provides a site-specific delivery of the therapeutic agent(s), to reach an effective local concentration of the therapeutic agent, and further provides an improved uptake of this and activated immune cells at the region of interest.

Hence, the invention also provides a microbubble-microdroplet cluster composition for use in a method of localized delivery of at least one ITA to a subject, wherein the method comprises the steps of:
(i) administering at least one ITA to the subject;
(ii) administering the cluster composition to the subject;
   wherein the at least one therapeutic agent is pre-, and/or co- and/or post administered to the cluster composition;
(iii) activating a phase shift of a diffusible component of the microdroplet of the cluster composition from step (i) by ultrasound insonation of a region of interest within said subject at a first frequency of 1 to 10 MHz and with a first mechanical index of 0.1 to 0.4;
(iv) insonating further with ultrasound at a second frequency of 0.4 to 0.6 MHz and with a second mechanical index of 0.1 to 0.3.

Further disclosed herein but not encompassed by the wording of the claims a method of delivering at least one ITA to a mammalian subject, comprising the steps of:
(i) administering at least one ITA to the subject;
(ii) administering a microbubble-microdroplet cluster composition to the subject; wherein the at least one ITA is pre-, and/or co- and/or post administered to the cluster composition;
(iii) activating a phase shift of a diffusible component of the microdroplet of the cluster composition from step (i) by ultrasound insonation of a region of interest within said subject at a first frequency of 1 to 10 MHz and with a first mechanical index of 0.1 to 0.4;
(iv) insonating further with ultrasound at a second frequency of 0.4 to 0.6 Hz and with a second mechanical index of 0.1 to 0.3.

Also for this composition for use and method for delivery of at least one ITA, the steps of the method may comprise a step (iib) optionally imaging the clusters using ultrasound imaging to identify the region of interest for treatment within said subject.

The use and method facilitate enhanced extravasation and uptake of the separate pre-, and/or co- and/or post administered ITA(s) and/or enhanced infiltration of activated immune cells to a targeted pathology. In the method, the therapeutic agent is pre-, and/or co- and/or post administered to the cluster composition and before steps ii) to iv) or after any of steps ii) to iv).

In some embodiments, only the cluster composition without a therapeutic agent is administered to a subject, for the preparation of a subject for a subsequent administration of a therapeutic agent. In such embodiments, the administration of the cluster composition is such that the administration is not a treatment, but a preparation for a treatment, such as a preparation for a treatment with an ITA.

The methods and composition for use of the invention may be accompanied by diagnostic imaging, e.g. by doing ultrasound imaging including using the microbubbles of the clusters as contrast agents, as disclosed above, but may also be combined with other types of imaging studies, typically to diagnose and/or assess the outcome of the treatment. Such imaging may include abdominal computed tomography (CT), e.g. as an initial test to identify the targeted pathology, or abdominal magnetic resonance imaging (MRI), abdominal ultrasonography (US), and endoscopic ultrasound (EUS).

The invention shall not be limited to the shown embodiments and examples. While various embodiments of the present disclosure are described herein, it will be obvious to those skilled in the art that such embodiments are provided by way of example only. Numerous modifications and changes to, and variations and substitutions of, the embodiments described herein will be apparent to those skilled in the art without departing from the disclosure. It is to be understood that various alternatives to the embodiments described herein can be employed in practicing the disclosure.

It is to be understood that every embodiment disclosed for one aspect equally well apply for the other aspects. Hence, for example the features disclosed for the composition for use in therapy also apply for the method of localised delivery and for the method of increasing the uptake and enhanced infiltration of activated immune cells.

It is to be understood that every embodiment of the disclosure can optionally be combined with any one or more of the other embodiments described herein.

It is to be understood that each component, compound, or parameter disclosed herein is to be interpreted as being disclosed for use alone or in combination with one or more of each and every other component, compound, or parameter disclosed herein. It is further to be understood that each amount/value or range of amounts/values for each component, compound, or parameter disclosed herein is to be interpreted as also being disclosed in combination with each amount/value or range of amounts/values disclosed for any other component(s), compound(s), or parameter(s) disclosed herein, and that any combination of amounts/values or ranges of amounts/values for two or more component(s), compound(s), or parameter(s) disclosed herein are thus also disclosed in combination with each other for the purposes of this description. Any and all features described herein, and combinations of such features, are included within the scope of the present invention provided that the features are not mutually inconsistent.

It is to be understood that each lower limit of each range disclosed herein is to be interpreted as disclosed in combination with each upper limit of each range disclosed herein for the same component, compound, or parameter. Thus, a disclosure of two ranges is to be interpreted as a disclosure of four ranges derived by combining each lower limit of each range with each upper limit of each range. A disclosure of three ranges is to be interpreted as a disclosure of nine ranges derived by combining each lower limit of each range with each upper limit of each range, etc. Furthermore, specific amounts/values of a component, compound, or parameter disclosed in the description or an example is to be interpreted as a disclosure of either a lower or an upper limit of a range and thus can be combined with any other lower or upper limit or a range or specific amount/value for the same component, compound, or parameter disclosed elsewhere in the application to form a range for that component, compound, or parameter.

The embodiments and features described in the context of one aspect, e.g. for the aspect directed to the composition for use in therapy, also apply to the other aspects of the invention directed to the use in delivery or in the methods for treatment or delivery.

The following examples are provided to illustrate the invention in accordance with the principles of the invention but, are not to be construed as limiting in any way.

### Examples

### Example 1. Cluster preparation, analytical tools and basic characteristics

Reference is made to the applicant's application WO2015/047103, and particularly to the Examples 1 and 2 of this, the contents of which are summarised herein by reference, providing descriptions of analytical methodologies for characterisation of the clusters compositions, results from use of the clusters, etc.

In the following the 1st component is designated C1, the 2nd component is designated C2 and the cluster composition, i.e. the composition resulting from a combination of the 1st and 2nd components, is designated DP (drug product). The microbubble-microdroplet clusters formed upon combining C1 and C2, i.e. present in DP, are crucial to the critical quality attributes of the composition, i.e. its functionality for delivery of drugs. Hence, analytical methodology to characterize and control the clusters formed with regards to concentration and size, is an imperative tool to assess the current invention as well as for medicinal Quality Control (QC). We have identified three different analytical tools that can be applied for this purpose; Coulter counting, Flow Particle Image Analysis (FPIA) and Microscopy/Image analysis.

In addition to these techniques, applied for characterization of the clusters in the cluster composition, analytical methodology has been developed to study the activation of the clusters in vitro, i.e. the generation of large, activated bubbles upon ultrasound irradiation. This methodology; "Sonometry" is detailed in E1-6 of WO2015/047103. Primary report responses from the Sonometry analysis are attenuation spectra and number and volume of activated bubbles and their size distribution, both vs. time after activation. Activation responses may also be explored by Microscopy/Image analysis as detailed in E1-5 of WO2015/047103.

### Components and compositions:

The 1st component (C1) in the compositions investigated in the included example consisted of per-fluorobutane (PFB) microbubbles stabilized by a hydrogenated egg phosphatidyl serine-sodium (HEPS-Na) membrane and embedded in lyophilized sucrose. HEPS-Na carries a negatively charged head group with an ensuing negative surface charge of the microbubbles. Each vial of C1 contains approximately 16 µL or 2.10⁹ microbubbles, with a mean diameter of approximately 2.0 µm. The freeze-dried formulation displays long shelf life, more particularly 3 years, stored at ambient room temperature.

The 2nd component (C2) in the compositions investigated in this example consisted of perfluoromethyl-cyclopentane (pFMCP) microdroplets stabilized by a 1,2-Distearoyl-sn-glycerol-3-phosphocholine (DSPC) membrane with 3% mol/mol stearylamine (SA) added to provide a positive surface charge. The microdroplets in the C2 were dispersed in 5 mM TRIS buffer. The standard formulation of C2 investigated in these studies contains approximately 4 µL or 0.8,10⁹ microdroplets per mL, with a mean diameter of approximately 1.8 µm. The 2nd component displays long shelf life, more particularly 18 months or more, stored refrigerated.

In some cases, to elucidate effects on cluster characteristics, a variety of formulation variables such as SA content, microdroplet size, microdroplet concentration, TRIS concentration and pH was varied in a controlled manner. In case such samples have been used, these aspects are detailed in the text.

The cluster composition (DP) was prepared aseptically by reconstituting a vial of C1 with 2 mL of C2 followed by 30 seconds of manual homogenisation. 2 mL was withdrawn from a vial of C2 using a sterile, single use syringe and needle. The content of the syringe was added through the stopper of a vial of C1 and the resulting DP was homogenised preparing the composition for administration. The prepared cluster composition is also called PS101 herein.

As shown in WO2015/047103, the first and second components, i.e. the microbubble formulation and the microdroplet formulation, can be varied. E.g. as shown in tables 9 and 10 of WO2015/047103 both the gas and the stabilising membrane of the first component can be varied, to prepare clusters with suitable properties, expected to be useful in treatment according to the invention.

### Stability of clusters in the cluster composition during analysis:

The clusters in the DP are formed and held together by the electrostatic attraction between the microbubbles and the microdroplets. These forces are finite and the clusters may break up after formation through various routes/influences such as mechanical stress or thermal (Brownian) motion. For precise and accurate characterization, it is important that the clusters remain stable during the time of analysis. This stability has been investigated with all the methodologies described above. To evaluate stability, 3 to 5 analyses were repeated on a single DP sample covering a timespan of > 5 minutes. No significant change in neither concentration nor size has been observed cross these replicates, proving that the microbubbles, microdroplets and clusters are stable for > 5 minutes under the analytical conditions stated, i.e. after dilution in PBS or water and under continuous homogenization (stirring).

### Formulation aspects:

A number of different formulation aspects can be explored for controlling the cluster content and size in the DP and for targeting optimal properties. Parameters that can be used to engineer cluster content and size distribution include, but are not limited to; the difference in surface charge between the microbubbles and the microdroplets e.g. SA%: the microdroplet size of C2: the pH: the concentration of TRIS in C2: and the concentration of microbubbles and microdroplets. In addition, chemical degradation of the components, e.g. during prolonged storage at high temperatures, may influence the ability of C1 and C2 to form clusters during preparation of the DP.

From *in-vitro* characterisation of 30 different compositions, as reported in WO2015/047103, several important correlations that elucidate the nature and characteristics of the system can be extracted. We found that the size of the clusters formed is also strongly connected to the Reactivity of the system. Only small clusters (i.e. 1- 5 um) and medium sized clusters (i.e. 5-10 µm) are formed at relatively low levels of Reactivity (e.g. < 20%). With increasing Reactivity, larger clusters start to form; at R > approx. 20%, 10-20 µm clusters start to form and at R > approx. 50%, 20-40 µm clusters start to form. When larger clusters form, it is at the expense of smaller and medium sized clusters; we found a clear optimum in content vs. Reactivity for cluster concentration 1- 5 µm and 5 -10 µm. We found that formation of larger clusters (i.e. larger than 10 um, or larger than 20 um) is detrimental to the efficacy of the composition and that the clustering potential must be balanced accordingly.

Based on applicant's experiments, and the results shown in Tables 5 and 6 of WO2015/047103, the efficacy (linear enhancement in Grey Scale units (GS)) of the cluster composition is correlated with the cluster mean size and the concentration of clusters (million/ml). Grey Scale enhancement is the increase in brightness (contrast) observed by US imaging after administration and activation of the cluster composition *in-vivo* and is a measure of the amount of activated bubbles in the imaged tissue. The results reported there are from a multivariate, principal component analysis (PCA) of the contribution of clusters in various size classes to the linear enhancement in the ultrasound signal from dog myocardium (Grey Scale units) upon i.v. administration of the cluster composition and activation in the left ventricle, please see Example 2 of WO2015/047103. The PCA was performed on data for 30 samples detailed in Tables 5 and 6 of this. The results demonstrate that small and medium sized clusters (< 10 µm) contribute significantly to the efficacy of the cluster composition whereas larger clusters (> 10 µm) do not. These results and conclusion also apply for the current invention. The results from the PCA analysis are stated in Table 1 below. Figure 1 shows a visualization of cluster size versus product efficacy based on the data in Table 1, demonstrating that clusters having a mean diameter in the range of 3 to 10 µm have an optimal efficacy. Hence, in Figure 1 product efficacy vs. cluster diameter is provided. Y-axis shows correlation coefficient to Grey Scale enhancement from US imaging of dog myocardium after injection and activation of clusters in the left ventricle and reflects the amount of activated bubbles deposited. X-axis shows cluster diameter in µm. Grey boxes represent the different cluster size bins evaluated: 1 to 5 µm, 5 to 10 µm, 10 to 20 µm and 20 to 40 µm. Solid line represents the continuous function of efficacy vs. cluster diameter. Error bars are standard error. Figure 1 is an alternative visualization of Figure 12 (left side) of WO2015/047103. As can be observed, the mean cluster diameter should be in the range of 3-10 µm, and preferably 4-9 µm, more preferably 5-7 µm.

**Table 1: Efficacy of clusters vs. mean diameter as reported in WO2015/047103.**

| **Channel Group** | **Mean Channel Diameter (µm)** | **Efficacy Coefficient** |
|---|---|---|
| 1 to 5 µm | 3 | 0.4 |
| 5 to 10 µm | 7.5 | 0.55 |
| 10 to 20 µm | 15 | 0.12 |
| 20 to 40 µm | 30 | -0.15 |

The cluster concentration and mean diameter of the cluster composition, prepared according to Example 1, was analysed and found to have a cluster concentration of about 40-44 million per mL and with a cluster mean diameter of about 5.8-6.2 µm, for several hours. The results are shown in Table 2 below and are consistent with the results of Table 6 of WO2015/047103. The data of Table 2 shows that the prepared cluster composition has an acceptable stability, and that an optimal size and concentration of clusters can be achieved.

**Table 2: Cluster concentration and mean diameter at various timepoints after preparation of the cluster composition.**

| **Time (hours)** | **Cluster Concentration (millions/mL)** | **Cluster Mean Diameter (µm)** |
|---|---|---|
| 0h | 44 ± 2 | 6.0 ± 0.2 |
| 1h | 43 ± 1 | 5.8 ± 0.2 |
| 2h | 44 ± 5 | 6.2 ± 0.1 |
| 3h | 40 ± 1 | 6.0 ± 0.2 |

Applying the concept of the present invention, i.e. by preparing a cluster composition from C1 and C2 prior to administration, hence forming microbubble-microdroplet clusters, as opposed to co-injection of the two components as taught by WO/9953963, enable a > 10-fold increase in efficacy. The formation of microbubble-microdroplet clusters upon combination of the 1st component and 2nd component, and administering these pre-made clusters, is a pre-requisite for its intended functionality *in-vivo.* The cluster composition is to be administered to the subject during a time window wherein the characteristics of the clusters are substantially unchanged, such as within 3 hours from combining the two components.

### Example 2. Frequency and Mechanical Index for the Enhancement step.

As earlier noted, the application of further US insonation after activation of the large ACT bubbles, i.e. the Enhancement step, leads to an increase in extravasation of drug from the vascular compartment to the targeted tissue interstitium and an increase in the infiltration of activated immune cells.

However, the attributes of this Enhancement field with regards to frequency and MI may strongly influence the efficacy of the procedure. A certain minimum level of biomechanical effects is needed in order for increased permeability to be induced but, if too strong, inertial cavitation mechanisms may be induced with ensuing vascular damage and a reduction in efficacy.

The level of bubble oscillations will be depended on several parameters, most importantly the US frequency and pressure, the latter defined by the mechanical index (MI). In order to study the effect of frequency and MI on the nature of the induced bubble oscillations and on the efficacy of the ACT procedure, 5 studies have been performed:
- The attenuation spectrum of a population of activated bubbles was measured.
- The radial oscillations of a typical activated bubble with a resting diameter of 20 µm, induced during the Enhancement step, have been modelled using the modified Rayleigh-Plesset model [Postema and Schmitz, Ultrasonic bubbles in medicine: influence of the shell, Ultrason Sonochem, 2007. 14(4): p. 438-44] for a series of frequencies and MIs.
- The tissue uptake of a drug mimicking chromophore (Evans Blue) has been explored as a function of MI with an Enhancement step US frequency of 0.5 MHz. For this study, the bubble oscillations were modelled using the modified Rayleigh-Plesset model.
- The therapeutic efficacy, treating prostate cancer in mice with nab-paclitaxel ± ACT, has been explored with an Enhancement MI of 0.2 at both 0.5 and 0.9 MHz.
- The therapeutic efficacy, treating breast cancer in mice with nab-paclitaxel ± ACT, has been explored with an Enhancement frequency of 0.5 MHz at MIs of 0.1 and 0.2.

### Materials and Methods:

The cluster composition investigated was as detailed in Example 1.

The attenuation spectrum of a population of activated bubbles was measured by Sonometry, as detailed in E1-6 of WO2015/047103.

Modelling of bubble oscillations as a function of frequency and MI was performed by solving the partial differential modified Rayleigh-Plesset equation in MATLAB 2020b (MathWorks, Natick, MA, USA). Specifically, a 20 µm diameter bubble was modelled in blood using a negligible shell stiffness and C4F10 gas properties. A linear ultrasound pulse was mimicked using a sinusoidal wave with a 3 cycle start and end gaussian ramp.

To investigate the effect of MI variance of the US Enhancement field, tumour specific uptake of Evans Blue (EB, fluorescent dye) has been investigated in a subcutaneous prostate cancer model (PC3) in mice. Five groups with Enhancement insonation MIs of 0, 0.1, 0.2, 0.3 and 0.4 were investigated (N=3 animals per group). Immediately after i.v. injection of EB, a single dose of cluster composition (2 mL/kg, (i.v.)) was given followed by 45 sec Activation US (2.25 MHz, MI 0.4) and 5 min Enhancement US (0.5 MHz, variable MI), focused to the tumour volume. 30 min after treatment, the tumours were excised and the amount of EB was measured by spectrophotometry at 620 nm.

The therapeutic effect of nab-paclitaxel (Abraxane^{®}, ABR) ± ACT with 2 mL cluster dispersion/kg, for treatment of human prostate cancer in mice, was investigated in a subcutaneous prostate cancer model (PC3). ABR (12 mg/kg, i.v.) was administered weekly for 4 weeks, each time immediately followed by three, back to back, ACT procedures. N=9 to 12 animals per group. In addition, a saline control group was performed (N=4). Activation US consisted of 45s insonation at 2.5 MHz, MI=0.4 and Enhancement US consisted of 5 minutes at 0.5 or 0.9 MHz, both with MIs of 0.2. End point was overall survival. Animals were culled when the tumour reached a maximum volume of 1000 mm³.

The therapeutic effect of nab-paclitaxel (Abraxane^{®}, ABR) ± ACT with 2 mL cluster dispersion/kg, for treatment of human breast cancer in mice, was investigated in a subcutaneous breast cancer model (Ca-MDA-MB231). ABR (12 mg/kg, i.v.) was administered weekly for 4 weeks, each time immediately followed by three, back to back, ACT procedures at MIs of 0.1 or 0.2. In addition, a drug alone group was investigated. N=9 to 12 animals per group. Activation US consisted of 45s insonation at 8 MHz MI=0.33 and Enhancement US consisted of 5 minutes at 0.5 MHz with MIs of 0.1 or 0.2. End point was tumour volume measured with caliper (each day), normalized by tumour volume at first day of treatment. Animals were culled when the tumour reached a maximum volume of 1000 mm³.

### Results:

### Attenuation spectrum of activated bubbles.

The attenuation spectrum of a typical population of activated ACT bubbles was measured and results are visualized in Figure 2. As can be noted, the resonance frequency was determined to approx. 0.3 MHz. Essentially, the attenuation spectrum describes the coupling between the bubble population and the incident US field; at or close to the resonance frequency, the bubbles are at their most effective in attenuating, hence, at their most effective in transforming the incident US energy to volume oscillations and biomechanical effects. Notably then, the spectrum demonstrates that the bubbles will respond very limited to frequencies outside a range from approx. 0.15 MHz to 0.6 MHz. However, this is the situation when the bubbles are dispersed in a practically infinite matrix, which is not the case when they are lodged in a micro vessel. In this case, the contact with the vessel wall will dampen the bubble response and shift the attenuation spectrum upwards, depending on the diameter and elasticity of the vessel. It is difficult to provide a precise modelling of such dampening effect, however, based on experience, a shift in resonance frequency to approx. 0.5 MHz is a reasonable estimate. Hence, an optimal coupling between the ACT bubbles, with ensuing optimal generation of biomechanical effects, is expected to occur between approx. 0.4 to 0.6 MHz. This then represents the preferred frequency range for the enhancement step under the current invention.

### Modelling of bubble oscillations as a function of frequency and MI.

Modelling of bubble oscillations for 300, 400, 600 and 900 kHz at MIs of 0.1, 0.2. 0.3 and 0.4 are visualised in Figure 3. Strong oscillations are evidence for induction of increased biomechanical effects. However, sharp sawtooth responses indicate onset of non-linear and/or inertial cavitation behaviour. As can be noted, at 900 kHz, for all MIs, the radial oscillations are small. In line with the prediction from the attenuation spectrum above, and likely too limited to induce the necessary biomechanical effects necessary to produce a significant therapeutic benefit. On the other hand, for 300 kHz, even for low MIs, the radial oscillations are very strong and non-linear, likely to lead to some level of inertial cavitation and to induce vascular damage. For frequencies between 400 and 600 kHz, however, the radial oscillations seem to meet with the requirement to induce sufficient biomechanical work to induce a therapeutic effect, while at the same time avoiding too much non-linear behaviour and vascular damage.

### Tissue uptake of Evans blue and bubble oscillations as a function of MI.

Results are visualized in Figure 4. As can be noted, the tissue uptake increases from no US (MI=0) to MI=0.1 and further with MI=0.2, but then drops again with MI=0.3 and further with M=0.4. At an MI of 0.2 an almost 60% increase in tumour specific uptake is observed, compared to MI=0 (no ultrasound). At the same time, from the embedded bubble oscillation panels, maximum radial oscillation increases from approx. 3 µm at MI=0.1, to approx. 6 µm at MI=0.2, to approx. 10 µm at MI=0.3 and more than to 20 µm at MI=0.4. Importantly, then onset of a reduction in tissue uptake (from MI=0.2 to MI=0.3) is concurrent with the onset of significant non-linear behaviour, where inertial cavitation starts to occur.

### Therapeutic effect of nab-paclitaxel ± ACT for treatment of prostate cancer - effect of US frequency during the Enhancement step

The results showing overall survival vs. time are visualized in Figure 5. As can be noted, for the ACT (0.5 MHz group), 100% animals survived until end of study, with 80% of the animals in stable, complete remission (i.e. cancer free). While the ACT (0.9 MHz) group also showed therapeutic benefit vs. drug alone, the effect is significantly inferior to ACT (0.5 MHz). With 0.9 MHz, most tumours started to regrow with only 25% in stable, complete remission and 57% survival at end of study. These results demonstrate that a certain, minimum radial oscillation is needed to induce an optimal therapeutic effect.

As a pilot investigation to this study, in order to evaluate if higher MIs could be applied, an MI of 0.40 was also tested at 0.9 MHz. However, at this MI, clear evidence of superficial haemorrhaging was observed.

### Therapeutic effect of nab-paclitaxel ± ACT for treatment of breast cancer - effect of MI during the Enhancement step

The results showing tumour growth rate vs. time are visualized in Figure 6. As can be noted, for the ACT (MI 0.1) group, a marginal but insignificant reduction in tumour growth rate is observed, with tumour growth inhibition vs drug alone at Day 31 of only 8%. For the ACT (MI 0.2) group, however, a strong and significant reduction in tumour growth rate is observed, with tumour growth inhibition vs drug alone at Day 31 of 52%. Again, these results demonstrate that a certain, minimum radial oscillation is needed to induce therapeutic benefit.

### Conclusion:

Based on the results generated in these examples, it has been demonstrated that the functionality of the ACT concept is quite sensitive to variance in frequency and MI applied during the Enhancement step. Based on these studies, it is concluded that a preferred frequency range is between 0.4 to 0.6 MHz in combination with an applied MI between 0.1 to 0.3. With lower frequencies and higher MI applied during the Enhancement step, it has surprisingly been demonstrated that the activated bubble oscillations induced are too strong, leading to a significant loss of efficacy and vascular damage. On the other hand, with higher frequencies and lower MIs, the bubble oscillations induced are too small, leading to a lack of sufficient biomechanical effects and hence a significant loss in therapeutic efficacy.

### Example 3. Therapeutic effects in a subcutaneous mouse model for Hepatocellular Carcinoma (HCC) - Cancer immunotherapy with reovirus ± Acoustic Cluster Therapy.

The current example reports the effect of ACT in combination with an oncolytic virus for treatment of HCC.

Oncolytic viruses are a form of cancer immunotherapy that uses viruses to infect and destroy cancer cells. Viruses are particles that infect or enter our cells and then use the cell's genetic machinery to make copies of themselves and subsequently spread to surrounding uninfected cells. Recently, viruses have been used to target and attack tumours that have already formed. These viruses are known as oncolytic viruses and they represent a promising approach to treating cancer. Cancer cells often have impaired antiviral defenses that make them susceptible to infection. After infection, these oncolytic viruses can cause cancer cells to "burst", killing the cancer cells and releasing cancer antigens. These antigens can then stimulate immune responses that can seek out and eliminate any remaining tumour cells nearby and potentially anywhere else in the body.

Viruses from the reoviridare family (reoviruses) have been extensively explored for treatment of cancer (NCT01280058, NCT02620423, NCT03723915) with clear evidence or therapeutic benefit. However, a factor which may reduce the efficacy of reoviruses is limited uptake by the cancer cells. In this respect, it is envisioned that combining ACT with virus therapies may strongly increase the viral uptake and hence lead to an increase in therapeutic efficacy.

### Materials and Methods:

Acoustic Cluster Therapy (ACT) and compositions for use according to the invention (referred to as ACT) was investigated for therapeutic effect level in combination with an oncolytic reovirus.

The cluster composition investigated was as detailed in Example 1. The cluster composition was administered intravenous at 2 mL/kg and followed by local ultrasound (US) insonation of the tumour with 45 second of US Activation field (2.7 MHz, MI 0.3), followed with 5 minutes US Enhancement field (500 kHz, MI 0.2). The procedure was performed 3 consecutive times immediately after administration of reo-virus on each treatment day.

### HCC tumour xenografts were implanted by subcutaneous injection in balb/C mice. 1x10⁷ cancer cells were injected in the flank and allowed to grow freely for approximately 21 days prior to enrolment into the study. The mice (N=5 per group) were treated with either reovirus alone or in combination with ACT. In addition, a phosphate buffer saline (PBS) control group was investigated. Virus was administered six times at days 0, 5, 7, 10, 14 and 16. A dose of 1x10⁷ Plaque Forming Units (PFU) per day was administered intravenously immediately prior to ACT treatment. The animals were monitored twice a week for body weight and tumour size (volume) via caliper measurement for the duration of the study (25 days).

### Results:

The results for average tumour volume and standard error of the mean (SEM) are stated in Table 3 below and visualized in Figure 8.

**Table 3: Results for average tumour volume and standard error of the mean (SEM) vs. time, of treatment groups as detailed.**

| Time | PBS control | | Reovirus | | Reovirus + ACT | |
|---|---|---|---|---|---|---|
| Days | Tumour volume (mm³) | SEM | Tumour volume (mm³) | SEM | Tumour volume (mm³) | SEM |
| 0 | 1.9 | 0.9 | 2.2 | 0.8 | 0.5 | 0 |
| 5 | 0.6 | 0.1 | 2.2 | 0.8 | 0.5 | 0 |
| 7 | 5.1 | 4.4 | 10.1 | 6.0 | 0.5 | 0 |
| 10 | 6.4 | 3.0 | 9.9 | 3.3 | 0.5 | 0 |
| 14 | 13.7 | 12.2 | 16.3 | 11.8 | 0.5 | 0 |
| 16 | 27 | 24.8 | 13.6 | 12.2 | 0.5 | 0 |
| 17 | 27 | 14.8 | 37.2 | 18.7 | 0.5 | 0 |
| 21 | 100.7 | 67.1 | 97.2 | 31.4 | 7.9 | 6.1 |
| 25 | 178.2 | 96.8 | 152.1 | 49.0 | 31.4 | 18.3 |

Figure 8 provides the therapeutic efficacy of ACT in combination with oncolytic reo-virus for treatment of hepatocellular carcinoma. Y-axis showing tumour volume in mm³. X-axis showing time from start of study in days. Grey triangles below x-axis designate treatment days. Treatment groups are: Saline control (inverted triangles), oncolytic reo-virus saline (filled squares) and oncolytic reo-virus with ACT (filled circles).

As can be observed from the results displayed in Table 3 and visualized in Figure 8, treatment with reovirus alone showed no significant inhibition of tumour growth at the investigated dose when compared to the PBS control group. However, when combining the same dose of virus with ACT treatment a marked and significant tumour inhibition was observed, with a > 95% reduction in tumour volume at Day 25 vs. virus alone. At Day 25, p-value was calculated using a two-tailed ANOVA test at 95% confidence interval (non-parametric Kruskal-Wallis test with Dunns multiple comparison correction) to p=0.037.

### Conclusion:

This study hence confirms a strong synergistic effect when combining an immune therapy treatment, such as a reovirus, with ACT according to the current invention. Similar synergistic effects would be expected for treatment of other diseases (e.g. cancers and autoimmune diseases) with other types of immunotherapeutic agents.

### Example 4. Delivery of nano-drugs across the Blood-Brain Barrier (BBB).

The current example investigates the ability of ACT to deliver large, nanoconstructs across the BBB, applying US fields according to the current invention.

### Materials and Methods:

The ACT cluster composition investigated was as detailed in Example 1.

The nanoparticles investigated were core-crosslinked polymeric micelles (CCPMs) from Cristal Therapeutics (Maastricht, The Netherlands). These CCPMs are 70 nm in diameter, labelled with rhodamine B Cy7 for imaging purposes, and the formulation contained 44 mg/ml polymer and 40 nmol/ml Cy7.

The extravasation of CCPM in healthy mouse brains was measured using near infrared fluorescence (NIRF) imaging and the micro-distribution of the CCPM in brain sections was imaged by confocal laser scanning microscopy (CLSM).

Thirteen female albino BL6 mice, purchased at 6-8 weeks of age (Janvier labs, France), were housed in groups of five in individually ventilated cages under conditions free of specific pathogens. Cages were enriched with housing, nesting material and gnaw sticks, and were kept in a controlled environment (20-23°C, humidity of 50-60%) at a 12-hour night/day cycle. Animals had free access to food and sterile water. All experimental procedures were approved by the Norwegian Food Safety Authority.

Illustration of the ultrasound set-up is shown in Figure 9. A custom-built dual frequency transducer (centre frequency 0.5 MHz and 2.7 MHz) [Andersen et al., A Harmonic Dual-Frequency Transducer for Acoustic Cluster Therapy, Ultrasound Med Biol 2019 Sep; 45(9): 2381-2390] was mounted on top of a custom-made cone filled with degassed water. The transducer has a diameter of 42 mm and the -3dB width of the beam profile of the 0.5 and 2.7 MHz were 16 and 6 mm at a distance of 220 mm from the transducer surface. Signals were generated with a signal generator (33500B, Agilent Technologies, USA) and amplified with a 50 dB RF amplifier (2100L, E&I, USA). The amplifier is connected to the switch box which allows for switch from the Activation to the Enhancement US fields. The bottom of the cone was covered with an optically and acoustically transparent plastic foil (Jula Norge AS, Norway), forming a bag. The animal was positioned in prone position on top of an acoustically absorbing material (Aptflex F28, Precision Acoustics, UK), with ultrasound gel for coupling between the acoustic absorber, the animals head and the acoustically transparent foil.

The ACT procedure used comprised an activation and an enhancement step. The attenuation through the murine skull was measured to be approximately 21 ± 17% and 42 ± 21% for the 0.5 MHz and 2.7 MHz frequencies, respectively. These numbers where used to calculate the in situ acoustic pressures/MIs. The following ultrasound parameters were used for each step:
- Activation: Centre frequency of 2.7 MHz, average in situ acoustic pressure of corresponding to mechanical index (MI) of 0.18, 8 cycles pulse length, pulse repetition frequency of 1 kHz and insonation time of 60 s.
- Enhancement: Centre frequency of 0.5 MHz, average in situ acoustic pressure corresponding to MI of 0.15, 4 cycles pulse length, pulse repetition frequency of 1 kHz and insonation time of 300 s.

One round of ACT consisted of a bolus intravenous injection of 2 mL/kg of cluster composition prior to the 360 s insonation. Each animal received 3 rounds of ACT, resulting in a total of 75 µl ACT formulation and 18 min ultrasound. CCPM was injected i.v. immediately prior to the first ACT procedure.

Animals were anaesthetized using 2% isoflurane in medical air (78%) and oxygen (20%) (Baxter, USA) after which their lateral tail vein was cannulated. Hair was removed with a hair trimmer and depilatory cream (Veet, Canada). During the ACT procedure, animals were anaesthetized using 1.5-2% isoflurane in medical air. Respiration rate was monitored using a pressure sensitive probe (SA instruments, USA) and body temperature was maintained with external heating. Each animal received 3 ACT rounds directly after injection of CCPM. Control animals were handled in the same way as the ACT receiving animals but received 3 times a 50 µl saline injection instead of the cluster composition with 6 minutes interval.

Two timepoints were investigated: 1 and 24 hours after ended ACT treatment. At these timepoints, animals were euthanized by an intraperitoneal injection of pentobarbital (200µl) and kept under anaesthesia until their breathing halted. Thereafter they were transcardially perfused with 30 ml of PBS after which the brain was excised and imaged with the NIRF imager. Groups were; control/1 hour N=3, control/24 hours N=3, ACT/1 hour N=5 and ACT/24 hours N=2.

Excised brains were placed in a NIRF imager (Pearl Impulse Imager, LI-COR Biosciences Ltd., USA) to assess accumulation of CCPM^{®} in the brain. Brains were excited at 785 nm and fluorescence emission was detected at 820 nm. Images were analysed with ImageJ (ImageJ 1.51j, USA). A Region of Interest (ROI) was drawn around the brain and the total fluorescence intensity of the brain was acquired and normalized to the wet weight of the brain. A standard curve was used to convert the total fluorescence intensity to the percentage of the injected dose per gram of brain tissue (% ID/g). Results were plotted per timepoint and treatment group.

For confocal microscopy, excised brains were mounted transversely on a piece of cork with Optimum Cutting Temperature Tissue Tek (Sakura, The Netherlands) before submerging the sample slowly in liquid nitrogen. Of the frozen brains, the first 500 µm from the top was removed after which 5 x 10 µm thick sections and 5 x 25 µm thick sections were cut transversely. This was repeated every 800 µm throughout the brain.

### Results:

To study whether the increased permeability would facilitate extravasation of CCPMs, excised brains were imaged in a NIRF imager. Representative NIRF-images of controls and animals injected with the nanoparticles are shown in Figure 10 (upper panels). As can be noted, clear accumulation could be observed in brains which received ACT opposed to the control brains.

Quantitative analysis of the NIRF-images revealed a statistically significant increase in accumulation (% ID/g) between the ACT and control animals at both timepoints (Figure 10, left lower panel). Vs. control, with ACT the median % ID/g increased from 0.9 % ID/g to 2.6 % ID/g 1 hour and from 0.8 % ID/g to 2.2 % ID/g 24 hours after ACT. Respectively, a 290 and 280 % increase in % ID/g was observed.

To verify the increased accumulation of the CCPM in brain tissue after ACT treatment, and to study the location of CCPM with respect to blood vessels, brain sections were imaged by CLSM. Tilescans of ACT-treated brains showed several 'clouds' of fluorescence which were not observed in brains of control animals. 24 hours post ACT, tilescans of ACT-treated brains showed similar cloud patterns as the 1 hour treatment group. From thresholded tilescans of both control and ACT-treated animals, the number of pixels representing CCPM were extracted and normalized by the size of the ROI used to outline the hemispheres. As can be noted from Figure 10 (right lower panel), A clear and statistically significant 4.7-fold increase can be observed in the 1h post ACT-treated sections opposed to the control brains.

High magnification CLSM images at different locations in both the control brains and the ACT-treated brains were acquired to study the location of the CCPM with respect to the blood vessels. In ACT-treated brains, CCPM had clearly extravasated whereas in control brains CCPM were mainly observed intravascularly or minimally displaced from the blood vessel staining.

### Conclusion:

ACT clearly increased the permeability of the BBB for large nanoparticle constructs like the 70nm CCPM compound investigated, when applying the twostep insonation approach of the current invention. ACT resulted in an improved accumulation, extravasation, and penetration of CCPM into the brain parenchyma. It hence demonstrates the ability of ACT to delivery large drug molecules or constructs such as ITAs across any vascular barrier of the body.

### Example 5 (prospective). A pilot study of Acoustic Cluster Therapy (ACT) with PD-1 antibody in a syngeneic mouse model for melanoma.

Until recently, distant metastatic melanoma was considered refractory to systemic therapy. A better understanding of the interactions between tumours, the immune system and the mechanisms of regulation of T-cells led to the development of immune checkpoint inhibitors. Whereas this class of immunotherapy agents has improved clinical outcome for patients, response rates are still in the 30-40% range, hence, there is a clear medicinal need to improve on this therapy regimen.

By using the ACT approach to increase vasculature permeability, one can potentially increase activated T-cell infiltration and increase the delivery of immunotherapeutic agents to improve T-cell activation. Therefore, this may lead to a major impact on the treatment outcome of immunotherapy in patients with melanoma.

The aim of the study is to evaluate the antitumour activity of ACT in combination with a PD-1 antibody in a syngeneic mouse model for melanoma. The cluster composition and the ACT procedures applied will be as described in Example 1 and 3.

### Materials and Methods:

B16-F1 cells will be implanted subcutaneously into immune-competent C57BL/6J Black 6 mice and disease progression will be monitored by weekly primary tumour calliper measurements. When the tumour volumes reach 30-40 mm3, mice (n = 12 per group) will be randomized into six groups: Control (saline), US + PS101, Anti-mouse PD-1 (CD279), Isotype antibody control, Anti-mouse PD-1 + US + PS101 and Isotype antibody control + US + PS101.

Study duration will be until tumour volumes reach 150 mm3. Four animals in each group will be culled at day 7 for gene expression RNA sequence analysis (cf. 3.b). Anti-mouse PD-1 or isotype antibody will be administered at 200 µg/mouse, i.p., twice weekly, prior PS101 administration for the duration of the study. End points will be primary tumour volumes, weight curves and overall survival.

Four animals from each group will be culled at day 7 and tumours excised for full transcriptome mRNA analysis (NanoString analysis). Based on the results from this analysis, immuno-histochemistry (IHC) analysis for selected immune pathway, such as F4/80, CD3, CD4, CD8 and Foxp3 antibodies, will be performed on tumours excised at end of study. End points will me m RNA analysis at 7 days and IHC analysis at end of study.

**Results (prospective):** The results will show that, compared to anti-PD1 treatment alone, the combination with ACT leads to meeting one or more of the following therapeutic efficacy end-points; a significant increase in tumour growth inhibition, a significant increase in median overall survival, a significant increase in tumour infiltration of immune cells.

This study will hence confirm a strong synergistic effect when combining an immunotherapeutic agent such as an anti-PD1 monoclonal antibody with ACT according to the current invention.

### Example 6. Preliminary investigation of synergisms between Acoustic Cluster Therapy (ACT) and immuno-oncology (IO)

The aim of the study was to test efficacy of immune checkpoint inhibitors in combination with ACT in a subcutaneous melanoma model (B16-F1, ATCC). The pilot exploration had two objectives:
a) to test whether ACT enhances efficacy of immune checkpoint inhibitors in a subcutaneous melanoma model, based on the concept that ACT should enhance checkpoint inhibitor delivery to the tumour as it does for chemotherapeutic deliver, and
b) whether any preliminary evidence could be gained that ACT stimulates an immunogenic reaction in the tumour (i.e., renders an immunologically "cold" tumour or more immunologically "hot").

### Methods:

The study was conducted in two parts. First, an inhibitor finding study was carried out to establish in the proposed model (B16-F1, ATCC in immune-competent C57BL/6J Black 6 mice) the optimum checkpoint inhibitor, whether anti-PD-1, anti-CTLA4, or a combination of anti-PD-1 and anti-CTLA4. Data were analysed to establish the regimen that would be most likely to allow demonstration of improved efficacy when combined with ACT, on the basis that this would be when the selected inhibitor-only regimen showed only a moderate effect. For the sake of brevity, results of this part of the study are not reported below. They allowed a decision to use a combination of anti-PD-1 and anti-CTLA4 as checkpoint inhibitor in the second part of the study, which was conducted with the addition of ACT.

The cluster composition was as described in Example 1. "US + PS101" means use of ultrasound and the cluster composition in ACT treatment as disclosed herein.

Tumour volume, mouse weight and survival were monitored for the following treatment groups (n=12):
i) control (saline),
ii) checkpoint inhibitor,
iii) an iso-type antibody control (e.g., InVivoMab Rat IgG2a),
iv) checkpoint inhibitor (anti-PD-1 and anti-CTLA4) + US + PS101 (ACT), and v) iso-type antibody control (IgG2a ) + US + PS101 (ACT)

Data were analysed to determine whether ACT^{®} enhances the efficacy of checkpoint inhibition versus checkpoint inhibition alone.

To test for any stimulated change in immunogenic status of the tumours, four animals in each group were culled at day 7 for transcriptome mRNA (TempO-Seq^{©}, for 20,000 genes) analysis and immuno-histochemistry (IHC), for analysis of selected immune pathway biomarkers. Tumours excised at the end of the study were fixed and stored for potential retrospective RNA sequence analysis and IHC.

Various problems were experienced with the model, including difficulty cannulating tail veins in the black mice which increased with each subsequent cannulation, extremely soft highly cellular tumours which were difficult to palpate and measure by callipers making it difficult to size-match for the start of treatment, and a rapid growth rate that was highly variable from tumour to tumour. This generated considerable inter-tumour variability which limits ability to interpret the results. The considerable inter-tumoural variation in growth rate and, probably, treatment-onset tumour volume, produced substantial heterogeneity in growth curves. Despite this, although the differences in the mean fold-growths for all mice were not statistically significant for the different treatment groups, trends were observed for cautious interpretation. Treatment with the isotype antibody IgG2a only, appeared to have a similar therapeutic effect relative to saline controls as treatment with the checkpoint inhibitor combination anti-PD-11 and anti-CTLA-4 only. The addition of ACT seemed to slightly improve the average therapeutic effect of both isotype and checkpoint inhibitor. Although further analysis of these results could include calculation of tumour doubling times to see whether any significant differences emerge, examination of the individual tumour growth curves suggests that differences between treatment groups may lie more in the differences in spread of growth rates amongst mice treated than in differences between mean values. A trend towards increasing numbers of outliers of mice showing improved response, or even a suggestion of the generation of a bimodal distribution, with the addition of ACT. With this model, a study with many more mice would be needed to confirm whether this is a genuine observation.

Observations regarding the survival curves were similar to those for the mean fold growths, i.e., that the isotype antibody seemed to have an effect and that nonsignificant trends are suggestive that ACT may extend survival.

The growth curves for the subset of mice selected for culling and tumour genetic and IHC analysis suggest a similar story, with trends to the isotype eliciting a response and ACT improving the response. Here, it appears that further analysis, such as calculating tumour doubling times, may again be worthwhile to determine whether any significance between groups emerges.

### Immuno-histochemistry (IHC) analysis of selected immune pathway biomarkers (genes):

Heat maps and other graphs of normalised gene expression and IHC were prepared for the treatment groups. As the maps and graphs include colours, they are not suitable for inclusion, and the findings are summarized below.
- One analysis provided a heat map showing the strength of expression of immune pathway genes for each treatment group relative to the average expression across all animals. The selected genes were: BCR Signaling, M1 Activation, MHC Class I Antigen Presentation, T Cell Checkpoint Signaling, IFNg genes, CTLA4 Signaling, MHC Class II Antigen Presentation, PP1 Signaling, TLR Signaling. The heat map clearly showed that for the treatment group iv) treated with checkpoint inhibitor (anti-PD-1 and anti-CTLA4) and ACT, all the biomarkers were activated, appearing as red in the heat map. The other treatment groups appeared in tones of yellow or blue for all the genes, showing less or none activation.
- Another analysis provided a heat map showing the strength of expression of genes related to the following specific immune types of cells; T cells, CD8 T cells, Monocytes, Fibroblasts, NK cells, Masaat cells, Lymphatics, B derived Endothelial cells, vessels, for the different treatment groups. The heat map clearly showed that for the treatment group iv) treated with checkpoint inhibitor (anti-PD-1 and anti-CTLA4) and ACT, particularly the T cells, the CD8 T cells and the monocytes were activated appearing as red in the heat map.

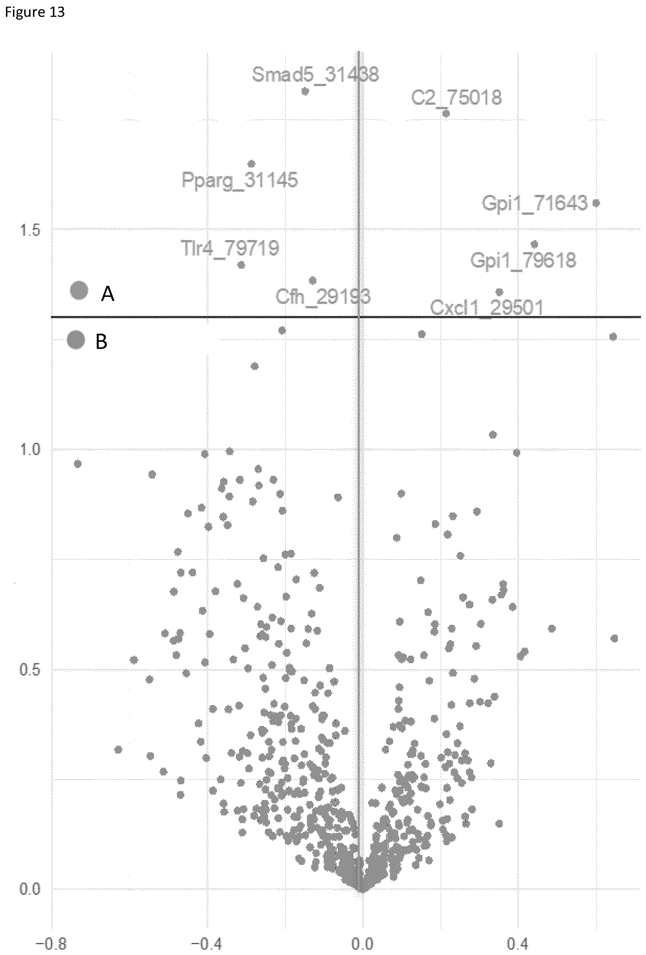

In the absence of a treatment group that was treated with ACT alone, the potential for ACT to elicit a change in immunogenic status of a tumour was assessed as the differential (log2 fold change) gene expression between treatment with the isotype antibody alone and treatment with isotype plus ACT. Figure 13 provides a "Volcano plot" showing the impact of ACT on the immunogenic status of the tumours as the expression, of immune genes only, when ACT is combined with the isotype antibody (IgG) relative to treatment with IgG alone. Median values are shown. Genes that did not show a significant differential expression are not labelled and are plotted as dots only (B), whereas those that were significant are plotted as dots and are labelled. Relatively few immune genes showed a differential expression that reached statistical significance, underlining the need for a study with more animals, a less heterogeneous model and a group treated with ACT alone.

Further analysis of the differential expression, in terms of biochemical pathways, suggested that relative to the isotype antibody alone, ACT in combination with the isotype tended to significantly (-log10(P) > 1.3) downregulate some pathways, including response to hypoxia, and upregulate others. Before considering the importance of these findings a larger study, and one that uses an ACT-alone group, is recommended. Potential then exists for later correlative imaging (e.g., using photoacoustic imaging in vivo, or quantitative morphometric histology) to confirm that gene expression is related to phenotypical characteristics.

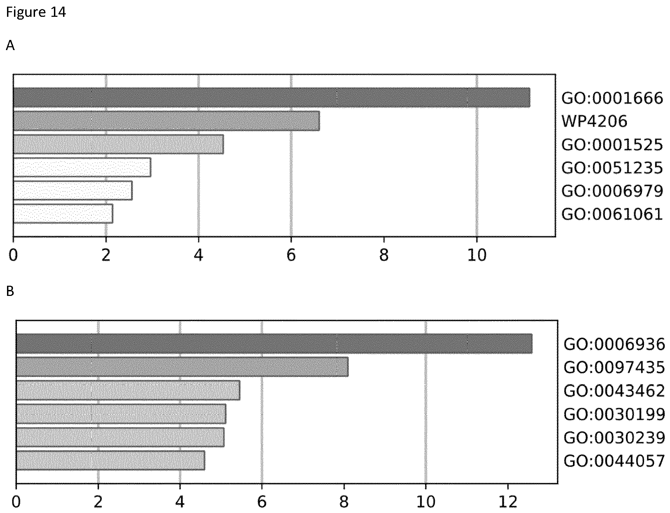

Figure 14 provides the genetic effect of ACT as indicated by pathways that were either downregulated (A) or upregulated (B) when ACT was combined with the isotype antibody (IgG) relative to treatment with IgG alone. x-axis showing: - log10(pvalue).
Panel A: Downregulated pathways in IgG+ACT.
Panel B: Upregulated pathways in IgG+ACT.
The letters denote the following:
   GO:0001666 - Response to hypoxia,
   WP4206 - Hereditary leiomyomatosis and renal cell carcinoma pathway,
   GO:0001525 - Angiogenesis,
   GO:0051235 - Maintenance of location,
   GO:0061061 - Muscle structure development,
   GO:0006936 - Muscle contraction,
   GO:0097435 - Supramolecular fibre organisation,
   GO:0043462 - Regulation of ATPase activity,
   GO:0030199 - Collagen fibril organisation,
   GO:0030239 - Myofibril assembly,
   GO:0044057 - Regulation of system process

Figure 15 provides immunohistochemical findings from an automated analysis of whole stained tumour sections. The proportion of cells that stained positive for CD8 T cells is shown by treatment group for individual animals (black points), and group means (bars) and standard deviations (error bars). x-axis showing treatment groups, wherein A: ACT+PD1/CTL4, B: PD1/CTL4, C: ACT+ISO, D: ISO, E: Saline Although the average normalised strength of expression of immune pathway genes appears to be increased by ACT this is due to an increase in a single tumour, and large within-group genetic differences can be seen between mice, which is replicated for other genes. Such genetic heterogeneity is a feature of cancer and is seen in tumour samples from patients. When analysing tumour samples from patients, previous research has drawn useful conclusions from studying gene expression in the extreme responders and non-responders as defined by RECIST (tumour shrinkage) criteria (Ref.). A similar approach here, or even to compare strength of expression of specific groups of genes with response measured from the growth curves in individual animals, may be helpful in future research but would require more animals than the four used for genetic analysis in this pilot study. The use a tumour model that is easier to work with and produces less heterogeneous growth curves may also allow for greater significance in the differences between groups in terms of mean values.

A similar pattern emerged for tissue inflammation signature (TIS) genes and for those related to immune types of cells. The TIS is being developed to guide potential treatments.

In this pilot study, the IHC analysis has to date been limited to staining for CD8 T cells, although sections remain available for further staining (e.g. for CD4 etc.). CD8 stains for a subpopulation of T cells that are important for mediating adaptive immunity, including cytotoxic (so-called "killer") T cells. Our findings must again carry the caveat of small numbers of animals and corresponding lack of significance, but it is interesting to note that the addition of ACT to treatment with either the checkpoint inhibitor or the isotype appeared to increase the number of tumours that had a high percentage of CD8 positive T cells.

Despite the lack of significance of many of the individual results obtained, when taken together they appear to represent a pattern that ACT may improve the therapeutic effectiveness of checkpoint inhibitors and cause tumours to generate a heightened immunogenic response.

### Example 7 (prospective). Manufacture of cluster compositions with various microbubble and microdroplet components.

In order to show that the invention is applicable for a variety of chemical compositions of the first and second components (C1 and C2), several formulations may be manufactured or sourced commercially and explored for the in-vitro attributes of the resulting cluster composition.

### C1 examples:

The commercially available microbubble US imaging agents Sonovue^{®} (Bracco Spa, Italy) and Micromarker^{®} (VisualSonics Inc., USA) may be sourced and used as C1 components. Sonovue is a sulphur hexafluoride microbubble stabilized with a membrane of distearoylphosphatidylcholine, dipalmitoylphosphatidylglycerol sodium, palmitic acid and PEG4000, and presented in a lyophilized form to be reconstituted with 5 mL of aqueous matrix. Micromarker is a perfluorobutane/nitrogen microbubble stabilized with phospholipids, polyethylenglycol and fatty acid, and presented in a lyophilized form for reconstitution with 0.7 mL of aqueous matrix.

### C2 examples:

Microdroplet (C2) components with diffusible components;
a) perfluorodimethylcyclobutane, b) 2-(trifluoromethyl)perfluoropentane and c) perfluorohexane may be manufactured as follows:
   790 mg distearoylphosphatidylcholine (DSPC) and 8.1 mg stearylamine (SA) is weighed into a 250 ml round bottom flask and 50 ml chloroform is added. The sample is heated under hot tap water until a clear solution is obtained. The chloroform is removed by evaporation to dryness on a rotary evaporator at 350 mm Hg and 40°C, followed by further drying at 50 mm Hg in desiccator overnight.

Thereafter, 160 ml water is added and the flask again placed on a rotary evaporator and the lipids rehydrated by full rotational speed and 80°C water bath temperature for 25 minutes. The resulting lipid dispersion is transferred to a suitable vial and stored in refrigerator until use.

Emulsions are prepared by transferring aliquots of 1 ml of the cold lipid dispersion to 2 ml chromatography vials. Each of 6 vials are added 100 µl of the fluorocarbon oils as detailed above. The chromatography vials are shaken on a CapMix (Espe, GmbH) for 75 seconds. The resulting emulsions are washed three times by centrifugation and removal of infranatant followed by addition of equivalent volume of an aqueous 5 mM TRIS buffer. The vials are immediately cooled on ice, pooled and kept cold until use.

Coulter counter analysis is performed to determine the volume concentration and diameter of the microdroplets, and the emulsions are then be diluted with 5 mM TRIS buffer to a disperse phase concentration 4 µl microdroplets/ml.

Preparation of cluster compositions are performed by reconstituting Sonovue or Micromarker with 5 or 0.7 mL, respectively, of each of the C2 components described above.

### Results (prospective).

Upon mixing of components C1 and C2, all six combinations are expected to comprise more than 10 million clusters per ml, with a mean diameter between 3 to 10 µm.

## Claims

1. A pharmaceutical composition comprising a microbubble-microdroplet cluster composition and at least one immunotherapeutic agent (ITA), for use in a method of treatment of a pathological condition of a mammalian subject, wherein the pathological condition is not pancreatic cancer, and
wherein the method comprises the steps of:
(i) administering the at least one immunotherapeutic agent (ITA) to the subject;
(ii) administering the microbubble-microdroplet cluster composition to the subject;
wherein the at least one ITA is pre-, and/or co- and/or post administered separate to the cluster composition;
(iii) activating a phase shift of a diffusible component of the microdroplet of the cluster composition from step (ii) by ultrasound insonation of a region of interest within said subject at a first frequency of 1 to 10 MHz and with a first mechanical index of 0.1 to 0.4;
(iv) insonating further with ultrasound at a second frequency of 0.4 to 0.6 MHz and with a second mechanical index of 0.1 to 0.3.

2. The pharmaceutical composition of claim 1, for use according to claim 1, wherein the steps (ii) to (iv) are repeated one to four times; and optionally wherein the insonation of step (iii) starts immediately after step (ii) and is immediately followed by the insonation of step (iv); and optionally wherein the insonation of step (iii) lasts for 30-120 seconds, followed by the insonation of step (iv) which lasts for 3-10 minutes.

3. The pharmaceutical composition of claim 1, for use according to any of the claims 1 or 2, wherein 1 to 5 therapeutic agents, including the at least one ITA, are administered simultaneously or sequentially over a certain time span wherein at least one, such as 1 to 5, ACT treatments comprising steps (ii) to (iv) are performed during the same period; and optionally wherein the microbubble-microdroplet cluster composition facilitates enhanced extravasation and uptake of the separate pre-, and/or co- and/or post administered ITA(s) and/or inflammatory cytokines and/or enhanced infiltration of activated immune cells to a targeted pathology.

4. The pharmaceutical composition of claim 1, for use according to any of the claims 1 to 3, wherein a broad band or dual frequency US transducer is used in both the activation insonation of step (iii) and the further insonation of step (iv).

5. The pharmaceutical composition of claim 1, for use according to any of the claims 1 to 4, wherein the clusters have a mean diameter in the range 3-10 µm, and preferably in the range 4-9 µm; and optionally
wherein the cluster concentration of clusters in the size range 1-10 µm is at least 25 million/ml.

6. The pharmaceutical composition of claim 1 or 5, for use according to any one of claims 1 to 4, wherein a gas of the microbubbles of the microbubble-microdroplet clusters comprises sulphur hexafluoride or a C3-6 perfluorocarbon or mixtures thereof.

7. The pharmaceutical composition of any of the claims 1 and 5 or 6, for use according to any one of claims 1 to 4, wherein the microbubble comprises a first stabilizer comprising a phospholipid, a protein, or a polymer optionally added a negatively charged surfactant, and the microdroplet comprises a second stabilizer comprising a phospholipid, protein, or a polymer optionally added a positively charged surfactant.

8. The pharmaceutical composition of any of the claim 1, and 5 to 7, for use according to any of the claims 1 to 4, wherein the therapeutic agent is formulated in a vehicle, such as included in the form of liposomes, micelles, conjugates, nanoparticles, core-crosslinked polymeric micelles (CCPMs) or microspheres; and optionally
wherein the at least one ITA is selected from the group of immune-oncology agents, monoclonal antibodies (mAbs), fusion proteins, soluble cytokine receptors, recombinant cytokines, small-molecule mimetics, cell therapies, cancer vaccines and oncolytic viruses; and optionally, wherein the immunotherapeutic agent is selected from the group of monoclonal antibodies.

9. The pharmaceutical composition of any of the claims 1 and 5 to 8, for use according to any of the claims 1 to 4, or 8, wherein the treatment with at least one ITA is combined with treatment with one or more chemotherapeutic agents.

10. The pharmaceutical composition of any of the claims 1 and 5 to 8, for use according to any of the claims 1 to 4, or 8 or 9, wherein the one or more ITAs are selected from ITAs having ability to target any of the antigens named CD1 to CD371.

11. The pharmaceutical composition of any of the claims 1 and 5 to 8, for use according to any of the claims 1 to 4, or 8 to 10, wherein the ITA is selected from the group of monoclonal antibodies anti-PD1, anti-PDL1 and CTLA4, and is used in combination with a chemotherapeutic agent.

12. The pharmaceutical composition of any of the claims 1 and 5 to 8, for use according to any of the claims 1 to 4, wherein the one or more ITAs are selected from the group of immune checkpoint inhibitors.

13. The pharmaceutical composition of any of the claims 1 and 5 to 8, for use according to any one of the claims 1 to 12, wherein the ITA, or a formulated form of the ITA has a molecular weight of more than 15.000 Daltons.

14. The pharmaceutical composition of any of the claims 1 and 5 to 8, for use according to any of the claims 1 to 13, wherein the use is for treatment of cancer or autoimmune disease; and optionally
wherein the use is for treatment of cancers, such as of localized pathological lesions, solid cancers, such as of melanomas, sarcomas, prostate cancers, colon cancers, anal cancers, oesophageal cancers, gastric cancers, rectal cancers, small intestine cancers, hepatic cancers, lung cancers, renal cancers, breast cancers, brain cancers, bile duct cancers, head and neck cancers or lymphomas, or for treatment of autoimmune diseases, such as of psoriasis, lupus, rheumatoid arthritis, Crohn's disease, multiple sclerosis or alopecia areata, or for avoiding rejection after organ transplants.

15. The pharmaceutical composition of any of claims 1, and 5 to 8, for use according to any one of the claims 1 to 14, wherein the cluster composition is administered in a time window of 3 hours from combining a first component of microbubbles with a second component of microdroplets preparing the microbubble-microdroplet cluster composition.

## Patentansprüche

1. Pharmazeutische Zusammensetzung, umfassend eine Mikrobläschen-Mikrotröpfchen-Clusterzusammensetzung und mindestens ein immunotherapeutisches Mittel (ITA), zur Verwendung in einem Verfahren zur Behandlung eines pathologischen Zustands eines Säugersubjekts, wobei der pathologische Zustand kein Bauchspeicheldrüsenkrebs ist, und wobei das Verfahren die folgenden Schritte umfasst:
(i) Verabreichen des mindestens einen immunotherapeutischen Mittels (ITA) an das Subjekt;
(ii) Verabreichen der Mikrobläschen-Mikrotröpfchen-Clusterzusammensetzung an das Subjekt;
wobei das mindestens eine ITA getrennt von der Clusterzusammensetzung vorher und/oder gleichzeitig und/oder danach verabreicht wird;
(iii) Aktivieren eines Phasenübergangs einer diffundierbaren Komponente des Mikrotröpfchens der Clusterzusammensetzung aus Schritt (ii) durch Ultraschallbeschallen eines Bereichs von Interesse innerhalb des Subjekts mit einer ersten Frequenz von 1 bis 10 MHz und mit einem ersten mechanischen Index von 0,1 bis 0,4;
(iv) weiteres Ultraschallbeschallen mit einer zweiten Frequenz von 0,4 bis 0,6 MHz und mit einem zweiten mechanischen Index von 0,1 bis 0,3.

2. Pharmazeutische Zusammensetzung nach Anspruch 1,
zur Verwendung nach Anspruch 1, wobei die Schritte (ii) bis (iv) ein- bis viermal wiederholt werden; und optional
wobei die Beschallung von Schritt (iii) unmittelbar nach Schritt (ii) beginnt und unmittelbar von der Beschallung von Schritt (iv) gefolgt wird; und optional
wobei die Beschallung von Schritt (iii) 30-120 Sekunden andauert, gefolgt von der Beschallung von Schritt (iv), die 3-10 Minuten andauert.

3. Pharmazeutische Zusammensetzung nach Anspruch 1, zur Verwendung nach einem der Ansprüche 1 oder 2, wobei 1 bis 5 therapeutische Mittel, die das mindestens eine ITA einschließen, gleichzeitig oder nacheinander über eine bestimmte Zeitspanne verabreicht werden, wobei mindestens eine, wie beispielsweise 1 bis 5, ACT-Behandlungen, welche die Schritte (ii) bis (iv) umfassen, während desselben Zeitraums durchgeführt werden; und optional
wobei die Mikrobläschen-Mikrotröpfchen-Clusterzusammensetzung eine verbesserte Extravasation und Aufnahme des/der getrennt vorher und/oder gleichzeitig und/oder danach verabreichten ITA(s) und/oder entzündlicher Zytokine und/oder eine verbesserte Infiltration aktivierter Immunzellen zu einer gezielten Pathologie erleichtert.

4. Pharmazeutische Zusammensetzung nach Anspruch 1, zur Verwendung nach einem der Ansprüche 1 bis 3, wobei ein Breitband- oder Doppelfrequenz-US-Wandler sowohl bei der Aktivierungsbeschallung von Schritt (iii) als auch bei der weiteren Beschallung von Schritt (iv) verwendet wird.

5. Pharmazeutische Zusammensetzung nach Anspruch 1, zur Verwendung nach einem der Ansprüche 1 bis 4, wobei die Cluster einen mittleren Durchmesser im Bereich von 3-10 µm und vorzugsweise im Bereich von 4-9 µm aufweisen; und optional
wobei die Clusterkonzentration von Clustern im Größenbereich von 1-10 µm mindestens 25 Millionen/mI beträgt.

6. Pharmazeutische Zusammensetzung nach Anspruch 1 oder 5, zur Verwendung nach einem der Ansprüche 1 bis 4, wobei ein Gas der Mikrobläschen der Mikrobläschen-Mikrotröpfchen-Cluster Schwefelhexafluorid oder einen C3-6-Perfluorkohlenstoff oder Gemische daraus umfasst.

7. Pharmazeutische Zusammensetzung nach einem der Ansprüche 1 und 5 oder 6, zur Verwendung nach einem der Ansprüche 1 bis 4, wobei das Mikrobläschen einen ersten Stabilisator umfasst, der ein Phospholipid, ein Protein oder ein Polymer umfasst, dem optional ein negativ geladenes Tensid zugesetzt ist, und das Mikrotröpfchen einen zweiten Stabilisator umfasst, der ein Phospholipid, Protein oder ein Polymer umfasst, dem optional ein positiv geladenes Tensid zugesetzt ist.

8. Pharmazeutische Zusammensetzung nach einem der Ansprüche 1 und 5 bis 7, zur Verwendung nach einem der Ansprüche 1 bis 4, wobei das therapeutische Mittel in einem Vehikel formuliert ist, wie beispielsweise eingeschlossen in Form von Liposomen, Mizellen, Konjugaten, Nanopartikeln, kernvernetzten polymeren Mizellen (CCPMs) oder Mikrosphären; und optional
wobei das mindestens eine ITA aus der Gruppe von immunonkologischen Mitteln, monoklonalen Antikörpern (mAbs), Fusionsproteinen, löslichen Zytokinrezeptoren, rekombinanten Zytokinen, niedermolekularen Mimetika, Zelltherapien, Krebsimpfstoffen und onkolytischen Viren ausgewählt ist; und optional wobei das immunotherapeutische Mittel aus der Gruppe von monoklonalen Antikörpern ausgewählt ist.

9. Pharmazeutische Zusammensetzung nach einem der Ansprüche 1 und 5 bis 8, zur Verwendung nach einem der Ansprüche 1 bis 4 oder 8, wobei die Behandlung mit mindestens einem ITA mit einer Behandlung mit einem oder mehreren chemotherapeutischen Mitteln kombiniert wird.

10. Pharmazeutische Zusammensetzung nach einem der Ansprüche 1 und 5 bis 8, zur Verwendung nach einem der Ansprüche 1 bis 4 oder 8 oder 9, wobei das eine oder mehrere ITAs aus ITAs ausgewählt sind, welche die Fähigkeit aufweisen, auf eines der als CD1 bis CD371 bezeichneten Antigene zu zielen.

11. Pharmazeutische Zusammensetzung nach einem der Ansprüche 1 und 5 bis 8, zur Verwendung nach einem der Ansprüche 1 bis 4 oder 8 bis 10, wobei das ITA aus der Gruppe von monoklonalen Antikörpern anti-PD1, anti-PDL1 und CTLA4 ausgewählt ist und in Kombination mit einem chemotherapeutischen Mittel verwendet wird.

12. Pharmazeutische Zusammensetzung nach einem der Ansprüche 1 und 5 bis 8, zur Verwendung nach einem der Ansprüche 1 bis 4, wobei das eine oder mehrere ITAs aus der Gruppe von Immuncheckpoint-Inhibitoren ausgewählt sind.

13. Pharmazeutische Zusammensetzung nach einem der Ansprüche 1 und 5 bis 8, zur Verwendung nach einem der Ansprüche 1 bis 12, wobei das ITA oder eine formulierte Form des ITA ein Molekulargewicht von mehr als 15.000 Dalton aufweist.

14. Pharmazeutische Zusammensetzung nach einem der Ansprüche 1 und 5 bis 8, zur Verwendung nach einem der Ansprüche 1 bis 13, wobei die Verwendung zur Behandlung von Krebs oder Autoimmunerkrankung ist; und optional
wobei die Verwendung zur Behandlung von Krebsarten, wie von lokalisierten pathologischen Läsionen, soliden Krebsarten, wie beispielsweise von Melanomen, Sarkomen, Prostatakrebsarten, Dickdarmkrebsarten, Analkrebsarten, Speiseröhrenkrebsarten, Magenkrebsarten, Rektumkrebsarten, Dünndarmkrebsarten, Leberkrebsarten, Lungenkrebsarten, Nierenkrebsarten, Brustkrebsarten, Gehirnkrebsarten, Gallengangskrebsarten, Kopf- und Halskrebsarten oder Lymphomen, oder zur Behandlung von Autoimmunerkrankungen, wie von Psoriasis, Lupus, rheumatoider Arthritis, Morbus Crohn, multipler Sklerose oder Alopecia areata, oder zur Vermeidung einer Abstoßung nach Organtransplantationen ist.

15. Pharmazeutische Zusammensetzung nach einem der Ansprüche 1 und 5 bis 8, zur Verwendung nach einem der Ansprüche 1 bis 14, wobei die Clusterzusammensetzung in einem Zeitfenster von 3 Stunden ab Kombinieren einer ersten Komponente von Mikrobläschen mit einer zweiten Komponente von Mikrotröpfchen zur Herstellung der Mikrobläschen-Mikrotröpfchen-Clusterzusammensetzung verabreicht wird.

## Revendications

1. Composition pharmaceutique comprenant une composition d'amas de microbulles-microgouttelettes et au moins un agent immunothérapeutique (ITA), pour une utilisation dans un procédé de traitement d'un état pathologique d'un sujet mammifère, dans laquelle l'état pathologique n'est pas un cancer pancréatique, et
dans laquelle le procédé comprend les étapes suivantes :
(i) le fait d'administrer au sujet ledit au moins un agent immunothérapeutique (ITA) ;
(ii) le fait d'administrer au sujet la composition d'amas de microbulles-microgouttelettes ;
dans laquelle ledit au moins un ITA est pré-administré, et/ou coadministré et/ou post-administré séparément de la composition d'amas ;
(iii) le fait d'activer un changement de phase d'un composant diffusible de la microgouttelette de la composition d'amas selon l'étape (ii) par insonation par ultrasons d'une région d'intérêt à l'intérieur dudit sujet à une première fréquence allant de 1 à 10 MHz et avec un premier indice mécanique allant de 0,1 à 0,4 ;
(iv) le fait d'effectuer une insonation supplémentaire avec des ultrasons à une deuxième fréquence allant de 0,4 à 0,6 MHz et avec un deuxième indice mécanique allant de 0,1 à 0,3.

2. Composition pharmaceutique selon la revendication 1, pour une utilisation selon la revendication 1, dans laquelle les étapes (ii) à (iv) sont répétées de une à quatre fois ; et facultativement dans laquelle l'insonation selon l'étape (iii) commence immédiatement après l'étape (ii) et est immédiatement suivie par l'insonation selon l'étape (iv) ; et facultativement
dans laquelle l'insonation selon l'étape (iii) dure pendant 30 à 120 secondes, suivie par l'insonation selon l'étape (iv) qui dure pendant 3 à 10 minutes.

3. Composition pharmaceutique selon la revendication 1, pour une utilisation selon une quelconque des revendications 1 ou 2, dans laquelle de 1 à 5 agents thérapeutiques, incluant ledit au moins un ITA, sont administrés simultanément ou séquentiellement sur une certaine période de temps pendant laquelle au moins un traitement ACT, notamment de 1 à 5 traitements ACT, comprenant les étapes (ii) à (iv) sont mis en œuvre pendant la même période ; et facultativement
dans laquelle la composition d'amas de microbulles-microgouttelettes facilite une extravasation et une captation améliorées du/des ITA préadministré(s), et/ou coadministré(s) et/ou post-administré(s) séparé(s) et/ou des cytokines inflammatoires et/ou une infiltration améliorée de cellules immunitaires activées, vers une pathologie ciblée.

4. Composition pharmaceutique selon la revendication 1, pour une utilisation selon une quelconque des revendications 1 à 3, dans laquelle un transducteur à ultrasons à large bande ou à double fréquence est utilisé à la fois dans l'insonation d'activation selon l'étape (iii) et dans l'insonation supplémentaire selon l'étape (iv).

5. Composition pharmaceutique selon la revendication 1, pour une utilisation selon une quelconque des revendications 1 à 4, dans laquelle les amas présentent un diamètre moyen situés dans la plage allant de 3 à 10 µm, et de préférence dans la plage allant de 4 à 9 µm ; et facultativement
dans laquelle la concentration d'amas dans la plage de taille allant de 1 à 10 µm est d'au moins 25 millions/mL.

6. Composition pharmaceutique selon la revendication 1 ou la revendication 5, pour une utilisation selon une quelconque des revendications 1 à 4, dans laquelle un gaz des microbulles des amas de microbulles-microgouttelettes comprend de l'hexafluorure de soufre ou un perfluorocarbone en C3-6 ou des mélanges de ceux-ci.

7. Composition pharmaceutique selon une quelconque des revendications 1 et 5 ou la revendication 6, pour une utilisation selon une quelconque des revendications 1 à 4, dans laquelle la microbulle comprend un premier stabilisant comprenant un phospholipide, une protéine, ou un polymère facultativement additionné d'un tensioactif chargé négativement, et la microgouttelette comprend un second stabilisant comprenant un phospholipide, une protéine, ou un polymère facultativement additionné d'un tensioactif chargé positivement.

8. Composition pharmaceutique selon une quelconque des revendications 1 et 5 à 7, pour une utilisation selon une quelconque des revendications 1 à 4, dans laquelle l'agent thérapeutique est formulé dans un véhicule, tel qu'inclus sous la forme de liposomes, de micelles, de conjugués, de nanoparticules, de micelles polymériques à cœur réticulé (CCPM) ou de microsphères ; et facultativement
dans laquelle ledit au moins un ITA est sélectionné dans le groupe des agents d'immuno-oncologie, des anticorps monoclonaux (mAbs), des protéines de fusion, des récepteurs de cytokines solubles, des cytokines recombinantes, des mimétiques à petites molécules, des thérapies cellulaires, des vaccins anticancéreux et des virus oncolytiques ; et dans laquelle, facultativement, l'agent immunothérapeutique est sélectionné dans le groupe des anticorps monoclonaux.

9. Composition pharmaceutique selon une quelconque des revendications 1 et 5 à 8, pour une utilisation selon une quelconque des revendications 1 à 4, ou la revendication 8, dans laquelle le traitement avec au moins un ITA est combiné avec un traitement avec un ou plusieurs agents chimiothérapeutiques.

10. Composition pharmaceutique selon une quelconque des revendications 1 et 5 à 8, pour une utilisation selon une quelconque des revendications 1 à 4, ou la revendication 8 ou la revendication 9, dans laquelle lesdits un ou plusieurs ITA sont sélectionnés parmi des ITA présentant une capacité de cibler l'un quelconque des antigènes nommés CD1 à CD371.

11. Composition pharmaceutique selon une quelconque des revendications 1 et 5 à 8, pour une utilisation selon une quelconque des revendications 1 à 4, ou la revendication 8 à la revendication 10, dans laquelle l'ITA est sélectionné dans le groupe des anticorps monoclonaux anti-PD1, anti-PDL1 et CTLA4, et est utilisé en combinaison avec un agent chimiothérapeutique.

12. Composition pharmaceutique selon une quelconque des revendications 1 et 5 à 8, pour une utilisation selon une quelconque des revendications 1 à 4, dans laquelle lesdits un ou plusieurs ITA sont sélectionnés dans le groupe des inhibiteurs de points de contrôle immunitaire.

13. Composition pharmaceutique selon une quelconque des revendications 1 et 5 à 8, pour une utilisation selon une quelconque des revendications 1 à 12, dans laquelle l'ITA, ou une forme formulée de l'ITA présente une masse moléculaire de plus de 15 000 Daltons.

14. Composition pharmaceutique selon une quelconque des revendications 1 et 5 à 8, pour une utilisation selon une quelconque des revendications 1 à 13, dans laquelle l'utilisation est destinée au traitement du cancer ou d'une maladie auto-immune ; et facultativement
dans laquelle l'utilisation est destinée au traitement de cancers, tels que des lésions pathologiques localisées, de cancers solides, tels que des mélanomes, sarcomes, cancers de la prostate, cancers du côlon, cancers anaux, cancers de l'œsophage, cancers gastriques, cancers rectaux, cancers de l'intestin grêle, cancers hépatiques, cancers pulmonaires, cancers rénaux, cancers du sein, cancers du cerveau, cancers des voies biliaires, cancers de la tête et du cou ou lymphomes, ou pour le traitement de maladies auto-immunes, telles que le psoriasis, le lupus, la polyarthrite rhumatoïde, la maladie de Crohn, la sclérose en plaques ou l'alopécie areata, ou pour éviter le rejet après des transplantations d'organes.

15. Composition pharmaceutique selon une quelconque des revendications 1, et 5 à 8, pour une utilisation selon une quelconque des revendications 1 à 14, dans laquelle la composition d'amas est administrée dans une fenêtre temporelle de 3 heures à partir de l'opération de combinaison d'un premier composant de microbulles avec un deuxième composant de microgouttelettes pour préparer la composition d'amas de microbulles-microgouttelettes.
